# EUROPEAN PATENT SPECIFICATION

(11) **EP 1 370 684 B1**
(45) Date of publication and mention of the grant of the patent: **28.05.2008**
(21) Application number: 01948414.6
(22) Date of filing: 15.06.2001
(51) Int. Cl.: C12Q 1/68, C12N 15/11, A61K 31/00, G01N 33/574, G01N 33/68

(54) **POLYNUCLEOTIDES RELATED TO COLON CANCER**
POLYNUKLEOTIDE ZUR BESTIMMUNG VON KOLONKREBS
POLYNUCLEOTIDES LIES AU CANCER DU COLON

(30) Priority: 15.06.2000 US 211835 P
(43) Date of publication of application: 17.12.2003
(60) Divisional application: 08006693.9
(73) Proprietor: Novartis Vaccines and Diagnostics, Inc., Emeryville, CA 94608 (US)
(72) Inventor: KENNEDY, Giulia, C., San Francisco, CA 94116 (US); KANG, Sanmao, Los Angeles, A 90012 (US); REINHARD, Christoph, Alameda, CA 94501 (US); JEFFERSON, Anne, Bennet, Oakland, CA 94610 (US)
(74) Representative: Hallybone, Huw George
(86) International application number: PCT/US2001/019313
(87) International publication number: WO 2001/096523

(56) References cited:
- WO-A-00/22130
- WO-A-99/33963
- US-A- 5 981 279
- US-A- 6 007 991
- DATABASE EBI [Online] EMBL; 7 June 2000 (2000-06-07) HEGDE, P. ET AL.: "Assessment of gene expression patterns in a model of colon tumor metastasis using 19,299 element cDNA microarray" retrieved from EBI, accession no. AW952397 Database accession no. AW952397 XP002229364
- DATABASE EBI [Online] EMBL; 14 March 2000 (2000-03-14) ROSENTHAL ET AL.: "Human prostate tumor cDNA library derived EST fragment #27" retrieved from EBI, accession no. AAZ52884 Database accession no. AAZ52884 XP002214383 & WO 99 55858 A (METAGEN GESELLSCHAFT FÜR GENOMFORSCHUNG) 4 November 1999 (1999-11-04)
- DATABASE EBI [Online] EMBL; 18 January 2000 (2000-01-18) GENSET: "Secreted protein EST coding sequence 108-002-5-0-F4-FL" retrieved from EBI, accession no. AAZ40784 Database accession no. AAZ40784 XP002214384 & WO 99 40189 A (GENSET) 12 August 1999 (1999-08-12)
- RADINSKY R ET AL: "Level and function of epidermal growth factor receptor predict the metastatic potential of human colon carcinoma cells." CLINICAL CANCER RESEARCH: AN OFFICIAL JOURNAL OF THE AMERICAN ASSOCIATION FOR CANCER RESEARCH. UNITED STATES JAN 1995, vol. 1, no. 1, January 1995 (1995-01), pages 19-31, XP002099964 ISSN: 1078-0432
- YEATMAN TIMOTHY J ET AL: "Identification of a differentially-expressed message associated with colon cancer liver metastasis using an improved method of differential display." NUCLEIC ACIDS RESEARCH, vol. 23, no. 19, 1995, pages 4007-4008, XP002214382 ISSN: 0305-1048

## Description

### Field of the Invention

The present invention relates to genes differentially expressed in colon cancer and dysplasia. More specifically, it relates to polynucleotides that are differentially regulated in colon cancer and the encoded gene products.

### Background of the Invention

Colon cancer is the second leading cause of cancer-related deaths in the United States. The American Cancer Society estimates that there will be approximately 94,700 new cases of colon cancer in the United States in 1999, and that colon cancer will be responsible for about 47,900 deaths. The colon has four sections: the ascending colon, the transverse colon, the descending colon and the sigmoid colon, and terminates with the rectum. Adenomatous polyps or adenomas, common benign lesions that progress to carcinomas can develop in any of the four sections of the colon or in the rectum. Over 95% of colon cancers are adenocarcinomas, or cancers of the cells that line the inside of the colon. Colon cancer frequently metastasizes to the liver and the lung.

Unlike lung cancer, in which smoking has been identified as the prime etiologic factor responsible for the disease, the principle mechanisms underlying colon cancer are complex and incompletely understood. Dietary factors are believed to promote carcinogenesis, especially a high fat intake. At the molecular level, a multistep process involving a number of mutations is suspected in the progression of adenomas to colon tumors (Vogelstein et al. (1988) N. Engl. J. Med. 319:525-532). The development and progression of colon cancer is driven by sequential mutations in three gene types: oncogenes, tumor suppressor genes and mismatch repair genes, which control the rate of mutations of other genes, including oncogenes and tumor suppressor genes. These mutations occur as a result of genetic predisposition (germline mutations) or in response to environmental factors (somatic mutations).

Several mutations that are associated with colon cancer have been identified. Germline mutations that have been linked to hereditary, or familial, colon cancer include the tumor suppressor gene adenomatous polyposis coli (APC) (Lengauer et al. (1991) Science 253:665-669) and the mismatch-repair genes MutL and MutS (Modrich (1995) Phil. Trans. R. Soc. Lond. B 347:89-95; Kolodner (1996) Genes Dev. 10:1433-1442). Defective APC has been implicated in familial adenomatous polyposis (FAP) and MutL and MutS in hereditary nonpolyposis colorectal cancer (HNPCC). Somatic mutations identified in association with sporadic colon cancer include the oncogenes K-ras, o-myc, and the tumor suppressor genes p53, APC, neurofibromatosis type 1 GTPase-activating protein (NF 1 GAP), deleted in colon cancer (DCC) and mutated in colon cancer (MCC) (Midgley et al. (1999) Lancet 353:391-399). WO 99/33963 and WO 00/22130 describe differentially-expressed marker genes that are up-regulated in colon cancer. Accession number AAZ 52884 describes isolation of a highly similar sequence from a human prostate tumor cDNA library.

Colon cancer is highly treatable and often curable when detected and treated in the early stages. Conventional diagnostic procedures include invasive procedures, such as digital rectal examination, sigmoidoscopy, colonoscopy and barium enema, and noninvasive procedures, such as fecal occult blood testing and genetic screening. Screening for tumor markers is particularly indicated for the identification of hereditary disease, as well as for the diagnosis of recurrence. For example, screening for careinoembryonic antigen (CEA) is used to diagnose asymptomatic recurrence. Emerging diagnostic methods include laser-induced fluorescence imaging techniques that can detect cancerous cells on the epithelial surface or within the colon wall (see, *e.g.,* von Rueden et al. (1993) J. Surg. Oncol. 53:43-46).

Conventional therapeutic approaches to treat colon cancer include surgical resection, radiation and chemotherapy, including adjuvant therapy. Gene therapeutic approaches include transfer of cytokine or immune antigen genes, transfer of enzyme-prodrug systems (see, *e.g.,* Huber et al (1993) Cancer Res. 53:4619-4626) and replacement of tumor suppressor genes (see, *e.g.,* Venook et al. (1998) Proc. ASCO 17:431a) using viral vectors (Zwacka et al. (1998) Hematol. Oncol. Clin. North Am. 12:595-615).

While several genes associated with colon cancer have been identified, identification of additional genes linked to development (or inhibition of development) of colon cancer can provide additional diagnostic tools and therapeutic targets. Identification of genes differentially expressed in colon cancer is particularly important in the advancement of drug discovery, diagnostic technologies, and the understanding of the progression and nature of colon cancer. The invention provides for identification of such differentially expressed genes.

### Summary of the Invention

This invention relates to polynucleotides that represent genes differentially expressed in colon cancer, e.g., adenomatous polyp, colorectal carcinoma, high metastatic potential colon tumor and metastatic colon cancer. The invention also relates to diagnostics and therapeutics comprising such polynucleotides, their corresponding genes or gene products, including probes, antisense nucleotides, and antibodies.

Accordingly, in one aspect the invention features a method of identifying a cancerous colon cell, where the method involves detecting at least one differentially expressed gene product, where the gene product is encoded by a gene comprising a sequence of SEQ ID NO:1 in a test sample, where the test sample is derived from a test cell suspected of being a cancerous colon cell, and comparing the expression level of the detected differentially expressed gene product with the expression level of the differentially expressed gene product in a control sample, where the control sample is derived from a cancerous colon cell. Detection of the expression level of the differentially expressed gene product in the test sample that is similar to the expression level of the gene product in the control sample indicates that the test cell is a cancerous colon cell. In one embodiment, detection is accomplished by hybridization of the test sample to a reference array, wherein the reference array comprises an identifying sequence of SEQ ID NO:1.

The invention also features a method of identifying a cancerous colon cell, where the method involves detecting at least one differentially expressed gene product, wherein detection is by detecting, hybridization of a polynucleotide comprising a sequence of SEQ ID NOS: 1 in a test sample, where the test sample is derived from a test cell suspected of being a cancerous colon cell, and comparing the hybridization level of the detected differentially expressed gene product with the hybridization level of the differentially expressed gene product in a control sample, where the control sample is derived from a cancerous colon cell. Detection of the hybridization level of the differentially expressed gene product in the test sample that is similar to the hybridization level of the gene product in the control sample indicates that the test cell is a cancerous colon cell. In one embodiment, detection is accomplished by hybridization of the test sample to a reference array, wherein the reference array comprises an identifying sequence of SEQ ID NO:1.

The invention also features an isolated polynucleotide comprising a sequence of SEQ ID NO:1 or degenerate variants thereof. In related aspects, the invention features arrays and recombinant host cells comprising a polynucleotide of the invention In one embodiment the polynucleotide includes the nucleotide sequence of an insert contained in a clone described herein and deposited with the ATCC.

In another aspect the invention features an isolated polypeptide encoded by a differentially expressed gene of the invention, as well as antibodies that specifically bind such polypeptides.

In another aspect, the invention features therapeutic compositions comprising an active agent for modulation of expression of a gene differentially expressed in cancerous colon cells. For example, the active agent of the therapeutic composition may effect a decrease in biological activity of a gene product encoded by a gene that is overexpressed in a cancerous cell relative to a normal cell, or may effect an increase in biological activity of a gene product encoded by a gene underexpressed in a cancerous cell relative to a normal cell.

The invention also features a library of differentially expressed genes, where the library includes the sequence information of the polynucleotide of SEQ ID. NO:1.

The library may be provided as a nucleic acid array or in a computer-readable format, and may include relative amounts of the polynucleotide of SEQ ID NO:1 where the relative amounts are representative of relative amounts of the polynucleotide found in a diseased colon cell.

A primary object of the invention is to provide polynucleotides that correspond to differentially expressed genes, and fragments thereof, that are useful in diagnosis of colon cancer, as well as in rational drug and therapy design.

Various aspects and embodiments of the invention will be readily apparent to the ordinarily skilled artisan upon reading the description provided herein.

### Brief Description of the Drawings

FIG. 1 is a graph showing the message levels of the gene corresponding to SK2. (c9083, SEQ ID NO:3) in the indicated cell lines.
FIG. 2 is a graph showing the effect of SK2 (9083) antisense oligonucleotides upon message levels for the gene corresponding to SK2 (SEQ ID NO:3).
FIGS. 3 and 4 graphs showing the effect of SK2 (9083) antisense oligonucleotides upon proliferation of SW620 cells (Fig. 3) and a non-colon cell line, HT1080 (Fig. 4).
FIG. 5 is a graph showing the effect of antisense oligonucleotides to the gene corresponding to cluster 378805 upon growth of SW620 cells (31-4as: antisense; 31-4rc: reverse control; WT: wildtype control (no oligo)).
FIGS. 6-8are graphs showing the results of proliferation assay with SW620 assays to exaine the effects of expression of K-Ras (control, Fig. 6), the gene corresponding to c3376 (CHIR11-4), and the gene corresponding to 402380 (CHIR33-4).
Fig. 9 si a graph showing the effects of expression of genes corresponding to K-Ras (control) and to 402380 (CHIR33-4) upon colon formation of SW620 cells in soft agar (values normalized to WST 1).

### Detailed Description of the Invention

Before the subject invention is further described, it is to be understood that the invention is not limited to the particular embodiments of the invention described below, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

In this specification and the appended claims, the singular forms "a," "an," and "the" include plural reference unless the context clearly dictates otherwise. Unless defined otherwise, all technical and scientific terms used herein have the same meaning as commonly understood to one of ordinary skill in the art to which this invention belongs..

The invention relates to polynucleotides comprising the disclosed nucleotide sequences, to full length cDNA, mRNA genomic sequences, and genes corresponding to these sequences and degenerate variants thereof, and to polypeptides encoded by the polynucleotides of the invention and polypeptide variants. The following detailed description describes the polynucleotide compositions encompassed by the invention, methods for obtaining cDNA or genomic DNA encoding a full-length gene product, expression of these polynucleotides and genes, identification of structural motifs of the polynucleotides and genes, identification of the function of a gene product encoded by a gene corresponding to a polynucleotide of the invention, use of the provided polynucleotides as probes and in mapping and in tissue profiling, use of the corresponding polypeptides and other gene products to raise antibodies, and use of the polynucleotides and their encoded gene products for therapeutic and diagnostic purposes.

### Definitions

The terms "polynucleotide" and "nucleic acid", used interchangeably herein, refer to a polymeric forms of nucleotides of any length, either ribonucleotides or deoxynucleotides. Thus, these terms further include, but are not limited to, single-, double-, or multi-stranded DNA or RNA, genomic DNA, cDNA, DNA-RNA hybrids, or a polymer comprising purine and pyrimidine bases or other natural, chemically or biochemically modified, non-natural, or derivatized nucleotide bases. These terms further include, but are not limited to, mRNA or cDNA that comprise intronic sequences (see, *e.g*., Niwa et al. (1999) Cell 99(7):691-702). The backbone of the polynucleotide can comprise sugars and phosphate groups (as may typically be found in RNA or DNA), or modified or substituted sugar or phosphate groups. Alternatively, the backbone of the polynucleotide can comprise a polymer of synthetic subunits such as phosphoramidites and thus can be an oligodeoxynucleoside phosphoramidate or a mixed phosphoramidate-phosphodiester oligomer. Peyrottes et al. (1996) Nucl. Acids Res. 24:1841-1848; Chaturvedi et al. (1996) Nucl. Acids Res. 24:2318-2323. A polynuclotide may comprise modified nucleotides, such as methylated nucleotides and nucleotide analogs, uracyl, other sugars, and linking groups such as fluororibose and thioate, and nucleotide branches. The sequence of nucleotides may be interrupted by non-nucleotide components. A polynucleotide may be further modified after polymerization, such as by conjugation with a labeling component. Other types of modifications included in this definition are caps, substitution of one or more of the naturally occurring nucleotides with an analog, and introduction of means for attaching the polynucleotide to proteins, metal ions, labeling components, other polynucleotides, or a solid support.

The terms "polypeptide" and "protein", used interchangebly herein, refer to a polymeric form of amino acids of any length, which can include coded and non-coded amino acids, chemically or biochemically modified or derivatized amino acids, and polypeptides having modified peptide backbones. The term includes fusion proteins, including, but not limited to, fusion proteins with a heterologous amino acid sequence, fusions with heterologous and homologous leader sequences, with or without N-terminal methionine residues; immunologically tagged proteins; and the like.

"Heterologous" means that the materials are derived from different sources (e.g., from different genes, different species, etc.).

The term "differentially expressed gene" is intended to encompass a polynucleotide that represents or corresponds to a gene that is differentially expressed in a cancerous colon cell when compared with a cell of the same cell type that is not cancerous. Such differentially expressed gene may include an open reading frame encoding a gene product (e.g., a polypeptide), as well as introns of such genes and adjacent 5' and 3' non-coding nucleotide sequences involved in the regulation of expression, up to about 20 kb beyond the coding region, but possibly further in either direction. The gene may be introduced into an appropriate vector for extrachromosomal maintenance or for integration into a host genome. In general, a difference in expression level associated with a decrease in expression level of at least about 25%, usually at least about 50% to 75%, more usually at least about 90% or more is indicative of a differentially expressed gene of interest, *i.e*., a gene that is underexpressed or downregulated in the test sample relative to a control sample. Furthermore, a difference in expression level associated with an increase in expression of at least about 25%, usually at least about 50% to 75%, more usually at least about 90% and may be at least about 1 ½-fold, usually at least about 2-fold to about 10-fold, and may be about 100-fold to about 1,000-fold increase relative to a control sample is indicative of a differentially expressed gene of interest, *i.e.,* an overexpressed or up-regulated gene.

"Differentially expressed polynucleotide" as used herein means a nucleic acid molecule (RNA or DNA) comprising a sequence that represents a differentially expressed gene, e.g., the differentially expressed polynucleotide comprises a sequence (e.g., an open reading frame encoding a gene product) that uniquely identifies a differentially expressed gene so that detection of the differentially expressed polynucleotide in a sample is correlated with the presence of a differentially expressed gene or gene product of a differentially expressed gene in a sample. For example, detection of a polynucleotide in a sample that hybridizes (e.g., under stringent conditions) to a differentially expressed polynucleotide is indicative of the presence of the corresponding differentially expressed gene in the sample. "Differentially expressed polynucleotides" is also meant to encompass fragments of the disclosed polynucleotides, e.g., fragments retaining biological activity, as well as nucleic acids that are homologous, substantially similar, or substantially identical (e.g., having about 90% sequence identity) to the disclosed polynucleotides.

"Corresponds to" or "represents" when used in the context of, for example, a polynucleotide or sequence that "corresponds to" or "represents" a gene means that a sequence of the polynucleotide is present in the gene or in the nucleic acid gene product (*e.g*., mRNA). The polynucleotide may be wholly present within an exon of a genomic sequence of the gene, or different portions of the sequence of the polynucleotide may be present in different exons (*e.g*., such that the contiguous polynucleotide sequence is present in an mRNA, either pre- or post-splicing, that is an expression product of the gene). In some embodiments, the polynucleotide may represent or correspond to a gene that is modified in a cancerous cell relative to a normal cell. For example, the gene in the cancerous cell may be modified by insertion of an endogenous retrovirus, a transposable element, or other naturally occurring or non-naturally occurring nucleic acid. In such cases, the polynucleotide may include sequences of both the native gene (*e.g*., the gene without the heterologous sequence) and the inserted, non-native sequence.

"Gene" is generally used herein to encompass a polynucleotide that encodes a gene product, e.g., a nucleic acid sequence defining an open reading frame.

"Gene product" as used herein is meant to encompass all or a portion of a product of expression of a gene corresponding a polynucleotide described herein, including, but not necessarily limited to, an RNA molecule or a polypeptide.

"Diagnosis" as used herein generally includes determination of a subject's susceptibility to a disease or disorder, determination as to whether a subject is presently affected by a disease or disorder, as well as to the prognosis of a subject affected by a disease or disorder. The present invention encompasses diagnosis of subjects in the context of colon cancer (e.g., adenomatous polyp, colorectal carcinoma), as well as any stage of such cancers (e.g., stages I to IV in severity).

"Colon cancer" as used herein is meant to encompass benign or malignant forms of colon and rectal cancer; nonmetastatic, premetastatic and metastasized forms of colon cancer; and any particular type of cancer arising from cells of the colon and rectum (e.g., adenomatous polyp, colorectal carcinoma, and the like).

The terms "individual," "subject," "host," and "patient," used interchangeably herein and refer to any mammalian subject for whom diagnosis, treatment, or therapy is desired, particularly humans. Other subjects may include cattle, dogs, cats, guinea pigs, rabbits, rats, mice, horses, and so on.

The term "sample" or "biological sample" encompasses a variety of sample types obtained from an organism and can be used in a diagnostic or monitoring assay. The term encompasses blood and other liquid samples of biological origin, solid tissue samples, such as a biopsy specimen or tissue cultures or cells derived therefrom and the progeny thereof. The term encompasses samples that have been manipulated in any way after their procurement, such as by treatment with reagents, solubilization, or enrichment for certain components. The term encompasses a clinical sample, and also includes cells in cell culture, cell supernatants, cell lysates, serum, plasma, biological fluids, and tissue samples.

A "host cell", as used herein, refers to a microorganism or a eukaryotic cell or cell line cultured as a unicellular entity which can be, or has been, used as a recipient for a recombinant vector or other transfer polynucleotides, and include the progeny of the original cell which has been transfected. It is understood that the progeny of a single cell may not necessarily be completely identical in morphology or in genomic or total DNA complement as the original parent, due to natural, accidental, or deliberate mutation.

The terms "cancer", "neoplasm", "tumor", and "carcinoma", are used interchangeably herein to refer to cells which exhibit relatively autonomous growth, so that they exhibit an aberrant growth phenotype characterized by a significant loss of control of cell proliferation. In general, cells of interest for detection or treatment in the present application include precancerous (e.g., benign), malignant, premetastatic, metastatic, and non-metastatic cells. Detection of cancerous cell is of particular interest.

"Cancerous phenotype" generally refers to any of a variety of biological phenomena that are characteristic of a cancerous cell, which phenomena can vary with the type of cancer. The cancerous phenotype is generally identified by abnormalities in, for example, cell growth or proliferation (*e.g*., uncontrolled growth or proliferation), regulation of the cell cycle, cell mobility, or cell-cell interaction.

"Therapeutic target" generally refers to a gene or gene product that, upon modulation of its activity (*e.g*., by modulation of expression, biological activity, and the like), can provide for modulation of the cancerous phenotype.

As used throughout "modulation" is meant to refer to an increase or a decrease in the indicated phenomenon (*e.g*., modulation of a biological activity refers to an increase in a biological activity or a decrease in a biological activity).

### Overview of the Invention

In general, the invention is based on the discovery of polynucleotides that represent genes that are differentially expressed in cancerous colon cells. Differential expression of genes in colon cells affected with cancer is determined by, for example, detecting genes expressed in a cancerous colon cell, and comparing the level of gene expression (*e.g*., either qualitatively or quantitatively) to expression of those same genes in a normal colon cell (*i.e.,* a colon cell that is not affected by a colon cancer).

The polynucleotides corresponding to differentially expressed genes described herein were identified using differential displays of samples from normal colon cells, primary colon tumor cells, metastatic colon tumor cells and adenomatous polyp cells. The sequence of specific polynucleotides that represent differentially expressed genes are shown in SEQ ID NOS:1, 3, 5, 7, 9, 11-13, 15,16, 18, 20, 22, 24, 26, 27 and 29.

### Polynucleotide Compositions

The scope of the invention with respect to polynucleotide compositions includes, but is not necessarily limited to, polynucleotides comprising a sequence set forth in SEQ ID NO: 1, polynucleotides obtained from the biological materials described herein or other biological sources (particularly human sources) by hybridization under stringent conditions (particularly conditions of high stringency); genes corresponding to the provided polynucleotides; variants of the provided polynucleotides and their corresponding genes, particularly those variants that retain a biological activity of the encoded gene product (*e.g*., a biological activity ascribed to a gene product corresponding to the provided polynucleotides as a result of the assignment of the gene product to a protein family(ies) and/or identification of a functional domain present in the gene product). Other nucleic acid compositions contemplated by and within the scope of the present invention will be readily apparent to one of ordinary skill in the art when provided with the disclosure here.

"Polynucleotide" and "nucleic acid" as used herein interchangeably with reference to nucleic acids of the composition is not intended to be limiting as to the length or structure of the nucleic acid unless specifically indicted. Further, polynucleotides described herein may consist essentially of exon sequences, e.g., sequences that define an open reading frame and encode all or a portion of a gene product. By "consist essentially of" in the context of a polynucleotide described herein is mean that the polynucleotide is composed of a sequence encoding an open reading frame, which sequence may be flanked by any of a variety of sequences that do not materially affect the basic characteristic(s) of the encoded gene product Suitable flanking sequences include, but are not necessarily limited to, promoter sequence, enhancer sequences, transcriptional start and/or stop sites, construct or vector sequences (*e.g*., sequences that provide for manipulation of the polynucleotide within a linear or circular molecule, including, but not necessarily limited to, sequences for replication and maintenance of the construct or vector, sequences encoding gene products that provide for selection (*e.g*., antibiotic resistance or sensitivity, factors that affect growth in media with or without supplements, and the like)), sequences that provide for production of a fusion protein with the polynucleotide and a heterologous polypeptide (*i.e.,* a polypeptide encoded by a polynucleotide that originates from a source other than the polynucleotide to which it is operably linked), and the like.

The invention features polynucleotides that are expressed in human tissue, specifically human colon tissue. Nucleic acid compositions of the invention of particular interest comprise a sequence set forth in SEQ ID NO:1 or an identifying sequence thereof. An "identifying sequence" is a contiguous sequence of residues at least about 10 at to about 20 nt in length, usually at least about 50 nt to about 100 nt in length, that uniquely identifies a polynucleotide sequence, *e.g*., exhibits less than 90%, usually less than about 80% to about 85.% sequence identity to any contiguous nucleotide sequence of more than about 20 nt. Thus, the subject nucleic acid compositions include full length cDNAs-or mRNAs that encompass an identifying sequence of contiguous nucleotides from SEQ ID NO:1.

The polynucleotides of the invention also include polynucleotides having sequence similarity or sequence identity. Nucleic acids having sequence similarity are detected by hybridization under low stringency conditions, for example, at 50°C and 10XSSC (0.9 M saline/0.09 M sodium citrate) and remain bound when subjected to washing at 55°C in 1XSSC. Sequence identity can be determined by hybridization under stringent conditions, for example, at 50°C or higher and 0.1XSSC (9 mM saline/0.9 mM sodium citrate). Hybridization methods and conditions are well known in the art, see, *e.g*., USPN 5,707,829. Nucleic acids that are substantially identical to the provided polynucleotide sequences, *e.g*. allelic variants, genetically altered versions of the gene, *etc.,* bind to the provided polynucleotide sequences (SEQ ID NO:1) under stringent hybridization conditions. By using probes, particularly labeled probes of DNA sequences, one can isolate homologous or related genes. The source of homologous genes can be any species, *e.g*. primate species, particularly human; rodents, such as rats and mice; canines, felines, bovines, ovines, equines, yeast, nematodes, *etc.*

In general, hybridization is performed using at least 15 contiguous nucleotides (nt) of SEQ ID NO:1. That is, when at least 15 contiguous nt of one of the disclosed SEQ ID NOS. is used as a probe, the probe will preferentially hybridize with a nucleic acid comprising the complementary sequence, allowing the identification and retrieval of the nucleic acids that uniquely hybridize to the selected probe. Probes from more than one SEQ ID NO can hybridize with the same nucleic acid if the cDNA from which they were derived corresponds to the same full-length mRNA. Probes of more than 15 nt can be used, e.g., probes of from about 18 nt to about 100 nt, but 15 nt represents sufficient sequence for unique identification.

The polynucleotides of the invention also include naturally occurring variants of the nucleotide sequences (*e.g*., degenerate variants, allelic variants, *etc*.). Variants of the polynucleotides of the invention are identified by hybridization of putative variants with nucleotide sequences disclosed herein, preferably by hybridization under stringent conditions. For example, by using appropriate wash conditions, variants of the polynucleotides of the invention can be identified where the allelic variant exhibits at most about 25-30% base pair (bp) mismatches relative to the selected polynucleotide probe. In general, allelic variants contain 15-25% bp mismatches, and can contain as little as even 5-15%, or 2-5%, or 1-2% bp mismatches, as well as a single bp mismatch.

The invention also encompasses homologs corresponding to the polynucleotides of SEQ ID NO: 1, where the source of homologous genes can be any mammalian species, e.g., primate species, particularly human; rodents, such as rats; canines, felines, bovines, ovines, equines, yeast, nematodes, *etc.* Between mammalian species, *e.g*., human and mouse, homologs generally have substantial sequence similarity, *e.g*., at least 75% sequence identity, usually at least 90%, more usually at least 95% between nucleotide sequences. Sequence similarity is calculated based on a reference sequence, which may be a subset of a larger sequence, such as a conserved motif, coding region, flanking region, *etc.* A reference sequence will usually be at least about 18 contiguous nt long, more usually at least about 30 nt long, and may extend to the complete sequence that is being compared. Algorithms for sequence analysis are known in the art, such as gapped BLAST, described in Altschul, *et al. Nucleic Acids Res.* (1997) *25*:3389-3402.

In general, variants of the invention have a sequence identity greater than at least about 65%, preferably at least about 75%, more preferably at least about 85%, and can be greater than at least about 90% or more as determined by the Smith-Waterman homology search algorithm as implemented in MPSRCH program (Oxford Molecular). For the purposes of this invention, a preferred method of calculating percent identity is the Smith-Waterman algorithm, using the following. Global DNA sequence identity must be greater than 65% as determined by the Smith-Waterman homology search algorithm as implemented in MPSRCH program (Oxford Molecular) using an affine gap search with the following search parameters: gap open penalty, 12; and gap extension penalty, 1.

The subject nucleic acids can be cDNAs or genomic DNAs, as well as fragments thereof particularly fragments that encode a biologically active gene product and/or are useful in the methods disclosed herein (*e.g*.; in diagnosis, as a unique identifier of a differentially expressed gene of interest, *etc.).* The term "cDNA" as used herein is intended to include all nucleic acids that share the arrangement of sequence elements found in native mature mRNA species, where sequence elements are exons and 3' and 5' non-coding regions. Normally mRNA species have contiguous exons, with the intervening introns, when present, being removed by nuclear RNA splicing, to create a continuous open reading frame encoding a polypeptide of the invention.

A genomic sequence of interest comprises the nucleic acid present between the initiation codon and the stop codon, as defined in the listed sequences, including all of the introns that are normally present in a native chromosome. It can further include the 3' and 5' untranslated regions found in the mature mRNA. It can further include specific transcriptional and translational regulatory sequences, such as promoters, enhancers, *etc*., including about 1 kb, but possibly more, of flanking genomic DNA at either the 5' and 3' end of the transcribed region. The genomic DNA can be isolated as a fragment of 100 kbp or smaller; and substantially free of flanking chromosomal sequence. The genomic DNA flanking the coding region, either 3' and 5', or internal regulatory sequences as sometimes found in introns, contains sequences required for proper tissue, stage-specific, or disease-state specific expression.

The nucleic acid compositions of the invention can encode all or a part of the subject polypeptides. Double or single stranded fragments can be obtained from the DNA sequence by chemically synthesizing oligonucleotides in accordance with conventional methods, by restriction enzyme digestion; by PCR amplification, *etc*. isolated polynucleotides and polynucleotide fragments of the invention comprise at least about 10, about 15, about 20, about 35, about 50, about 100, about 150 to about 200, about 250 to about 300, or about 350 contiguous nt selected from the polynucleotide sequencer as shown in SEQ ID NO:1. For the most part, fragments will be of at least 15 nt, usually at least 18 nt or 25 nt, and up to at least about 50 contiguous nt in length or more. In a preferred embodiment, the polynucleotide molecules comprise a contiguous sequence of at least 12 nt selected from the group consisting of the polynucleotide shown in SEQ ID NO:1.

Probes specific to the polynucleotides of the invention can be generated using the polynucleotide sequences disclosed in SEQ ID NO:1. The probes are preferably at least about a 12,15,16,18,20,22,24, or 25 nt fragment of a corresponding contiguous sequence of SEQ ID NO:1 and can be less than 2,1,0.5,0.1, or 0.05 kb in length. The probes can be synthesized chemically or can be generated from longer polynucleotides using restriction enzymes. The probes can be labeled, for example, with a radioactive, biotinylated, or fluorescent tag. Preferably, probes are designed based upon an identifying sequence of a polynucleotide of one of SEQ ID NO:1.

More preferably, probes are designed based on a contiguous sequence of one of the subject polynucleotides that remain unmasked following application of a masking program for masking low complexity (*e.g*., XBLAST) to the sequence., *i.e.,* one would select an unmasked region, as indicated by the polynucleotides outside the poly-n stretches of the masked sequence produced by the masking program.

The polynucleotides of the subject invention are isolated and obtained in substantial purity, generally as other than an intact chromosome. Usually, the polynucleotides, either as DNA or RNA, will be obtained substantially free of other naturally-occurring nucleic acid sequences, generally being at least about 50%, usually at least about 90% pure and are typically "recombinant", *e.g.*, flanked by one or more nucleotides with which it is not normally associated on a naturally occurring chromosome.

The polynucleotides of the invention can be provided as a linear molecule or within a circular molecule, and can be provided within autonomously replicating molecules (vectors) or within molecules without replication sequences. Expression of the polynucleotides can be regulated by their own or by other regulatory sequences known in the art. The polynucleotides of the invention can be introduced into suitable host cells using a variety of techniques available in the art, such as transferrin polycation-mediated DNA transfer, transfection with naked or encapsulated nucleic acids, liposome-mediated DNA transfer, intracellular transportation of DNA-coated latex beads, protoplast fusion, viral infection, electroporation, gene gun, calcium phosphate-mediated transfection, and the like,

The subject nucleic acid compositions can be used to, for example, produce polypeptides, as probes for the detection of mRNA of the invention in biological samples (*e.g*., extracts of human cells) to generate additional copies of the polynucleotides, to generate ribozymes or antisense oligonucleotides, and as single stranded DNA probes or as triple-strand forming oligonudeoddes. The probes described herein can be used to, for example, determine the presence or absence of the polynucleotide sequences as shown in SEQ ID NO:1 or variants thereof in a sample. These and other uses are described in more detail below.

### Use of Polynucleotides to Obtain Full-Length cDNA, Gene, and Promoter Region

Full-length cDNA molecules comprising the disclosed polynucleotides are obtained as follows. A polynucleotide comprising a sequence of SEQ ID NO:1, or a portion thereof comprising at least 12, 15, 18, or 20 nt, is used as a hybridization probe to detect hybridizing members of a cDNA library using probe design methods, cloning methods, and clone selection techniques such as those described in USPN 5,654,173. Libraries of cDNA are made from selected tissues, such as normal or tumor tissue, or from tissues of a mammal treated with, for example, a pharmaceutical agent Preferably, the tissue is the same as the tissue from which the polynucleotides of the invention were isolated, as both the polynucleotides described herein and the cDNA represent expressed genes. Most preferably, the cDNA library is made from the biological material described herein in the Examples. The choice of cell type for library construction can be made after the identity of the protein encoded by the gene corresponding to the polynucleotide of the invention is known. This will indicate which tissue and cell types are likely to express the related gene, and thus represent a suitable source for the mRNA for generating the cDNA. Where the provided polynucleotides are isolated from cDNA libraries, the libraries are prepared from mRNA of human colon cells, more preferably, human colon cancer cells, which cells can be obtained from patient tissue or can be a colon cell line, *e.g*., Km12L4-A.

Techniques for producing and probing nucleic acid sequence libraries are described; for example, in Sambrook et al., Molecular Cloning: A Laboratory Manual, 2nd Ed., (1989) Cold Spring Harbor Press, Cold Spring Harbor, NY. The cDNA can be prepared by using primers based on sequence from SEQ ID NO :1. In one embodiment, the cDNA library can be made from only poly-adenylated mRNA. Thus, poly-T primers can be used to prepare cDNA from the mRNA.

Members of the library that are larger than the provided polynucleotides, and preferably that encompass the complete coding sequence of the native message, are obtained: In order to confirm that the entire cDNA has been obtained, RNA protection experiments are performed as follows. Hybridization of a full-length cDNA to an mRNA will protect the RNA from RNase degradation. If the cDNA is not full length, then the portions of the mRNA that are not hybridized will be subject to RNase degradation. This is assayed, as is known in the art, by changes in electrophoretic mobility on polyacrylamide gels, or by detection of released monoribonucleotides. Sambrook et al., Molecular Cloning: A Laboratory Manual, 2nd Ed., (1989) Cold Spring Harbor Press, Cold Spring Harbor, NY. In order to obtain additional sequences 5' to the end of a partial cDNA, 5' RACE *(*PCR Protocols: A Guide to Methods and Applications, (1990) Academic Press, Inc.) can be performed

Genomic DNA is isolated using the provided polynucleotides in a manner similar to the isolation of full-length cDNAs. Briefly, the provided polynucleotides, or portions thereof, are used as probes to libraries of genomic DNA. Preferably, the library is obtained from the cell type that was used to generate the polynucleotides of the invention, but this is not essential. Most preferably, the genomic DNA is obtained from the biological material described herein in the Examples. Such libraries can be in vectors suitable for carrying large segments of a genome, such as P 1 or YAC, as described in detail in *Sambrook et al.,* 9.4-9.30. In addition, genomic sequences can be isolated from human BAC libraries, which are commercially available from Research Genetics, Inc., Huntsville, Alabama, USA, for example. In order to obtain additional 5' or 3' sequences, chromosome walking is performed, as described in *Sambrook et al.,* such that adjacent and overlapping fragments of genomic DNA are isolated. These are mapped and pieced together, as is known in the art, using restriction digestion enzymes and DNA ligase.

Using the polynucleotide sequences of the invention, corresponding full-length genes can be isolated using both classical and PCR methods to construct and probe cDNA libraries. Using either method, Northern blots, preferably, are performed on a number of cell types to determine which cell lines express the gene of interest at the highest level. Classical methods of constructing cDNA libraries are taught in Sambrook *et al., supra.* With these methods, cDNA can be produced from mRNA and inserted into viral or expression vectors. Typically, libraries of mRNA comprising poly(A) tails can be produced with poly(T) primers. Similarly, cDNA libraries can be produced using the instant sequences as primers.

PCR methods are used to amplify the members of a cDNA library that comprise the desired insert. In this case, the desired insert will contain sequence from the full length cDNA that corresponds to the instant polynucleotides. Such PCR methods include gene trapping and RACE methods. Gene trapping entails inserting a member of a cDNA library into a vector. The vector then is denatured to produce single stranded molecules. Next, a substrate-bound probe, such a biotinylated oligo, is used to trap cDNA inserts of interest. Biotinylated probes can be linked to an avidin-bound solid substrate. PCR methods can be used to amplify the trapped cDNA. To trap sequences corresponding to the full length genes, the labeled probe sequence is based on the polynucleotide sequences of the invention. Random primers or primers specific to the library vector can be used to amplify the trapped cDNA. Such gene trapping techniques are described in Gruber et al., WO 95/04745 and Gruber *et al.,* USPN 5,500,356. Kits are commercially available to perform gene trapping experiments from, for example, Life Technologies, Gaithersburg, Maryland, USA.

"Rapid amplification of cDNA ends," or RACE, is a PCR method of amplifying cDNAs from a number of different RNAs. The cDNAs are ligated to an oligonucleotide linker, and amplified by PCR using two primers. One primer is based on sequence from the instant polynucleotides, for which full length sequence is desired, and a second primer comprises sequence that hybridizes to the oligonucleotide linker to amplify the cDNA. A description of this methods is reported in WO 97/19110. In preferred embodiments of RACE, a common primer is designed to anneal to an arbitrary adaptor sequence ligated to cDNA ends (Apte and Siebert, Biotechniques (1993) 15:890-893; Edwards et al., Nuc. Acids Res. (1991) 19:5227-5232). When a single gene-specific RACE primer is paired with the common primer, preferential amplification of sequences between the single gene specific primer and the common primer occurs. Commercial cDNA pools modified for use in RACE are available.

Another PCR based method generates full-length cDNA library with anchored ends without needing specific knowledge of the cDNA sequence. The method uses lock-docking primers (I-VI), where one primer, poly TV (I-III) locks over the polyA tail of eukaryotic mRNA producing first strand synthesis and a second primer, polyGH (IV-VI) locks onto the polyC tail added by terminal deoxynucleotidyl transferase (TdT)(see, e.g, WO 96/40998).

The promoter region of a gene generally is located 5' to the initiation site for RNA polymerase II. Hundreds of promoter regions contain the "TATA" box, a sequence such as TATTA or TATAA, which is sensitive to mutations. The promoter region can be obtained by performing 5' RACE using a primer from the coding region of the gene. Alternatively, the cDNA can be used as a probe for the genomic sequence, and the region 5' to the coding region is identified by "walking up." If the gene is highly expressed or differentially expressed, the promoter from the gene can be of use in a regulatory construct for a heterologous gene.

Once the full-length cDNA or gene is obtained, DNA encoding variants can be prepared by site-directed mutagenesis, described in detail in Sambrook *et al.,* 15.3-15.63. The choice of codon or nucleotide to be replaced can be based on disclosure herein on optional changes in amino acids to achieve altered protein structure and/or function

As an alternative method to obtaining DNA or RNA from a biological material, nucleic acid comprising nucleotides having the sequence of one or more polynucleotides of the invention can be synthesized. Thus, the invention encompasses nucleic acid molecules ranging in length from 15 nt (corresponding to at least 15 contiguous nt of SEQ ID NO:1) up to a maximum length suitable for one or more biological manipulations, including replication and expression, of the nucleic acid molecule. The invention includes but is not limited to (a) nucleic acid having the size of a full gene, and comprising SEQ ID NO:1, (b) the nucleic acid of (a) also comprising at least one additional gene, operably linked to permit expression of a fusion protein; (c) an expression vector comprising (a) or (b); (d) a plasmid comprising (a) or (b) ; and (e) a recombinant viral particle comprising (a) or (b). Once provided with the polynucleotides disclosed herein, construction or preparation of (a) - (e) are well within the skill in the art

The sequence of a nucleic acid comprising at least 15 contiguous nt of SEQ ID NO:1 preferably the entire sequence of SEQ ID NO:1 is not limited and can be any sequence of A, T, G, and/or C (for DNA) and A, U, G, and/or C (for RNA) or modified bases thereof, including inosine and pseudouridine. The choice of sequence will depend on the desired function and can be dictated by coding regions desired, the intron-like regions desired, and the regulatory regions desired. Where the entire sequence of SEQ ID NO:1 is within the nucleic acid, the nucleic acid obtained is referred to herein as a polynucleotide comprising the sequence of SEQ ID NO:1.

### Expression of Polypeptide Encoded by Full-Length cDNA or Full-Length Gene

The provided polynucleotides (*e.g*., a polynucleotide comprising a sequence of SEQ ID NO:1), the corresponding cDNA, or the full-length gene is used to express a partial or complete gene product Constructs of polynucleotides comprising sequencer SEQ ID NO:1 can also be generated synthetically. Alternatively, single-step assembly of a gene and entire plasmid from large numbers of oligodeoxyribonucleotides is described by, *e.g.,* Stemmer et al., Gene (Amsterdam) (1995) 164(1):49-53. In this method, assembly PCR (the synthesis of long DNA sequences from large numbers of oligodeoxyribonucleotides (oligos)) is described. The method is derived from DNA shuffling (Stemmer, Nature (1994) 370:389-391), and does not rely on DNA.ligase, but instead relies on DNA polymerase to build increasingly longer DNA fragments during the assembly process.

Appropriate polynucleotide constructs are purified using standard recombinant DNA techniques as described in, for example, Sambrook et al.,Molecular Cloning: A Laboratory Manual 2nd Ed, (1989) Cold Spring Harbor Press, Cold Spring Harbor, NY, ead under current regulations described in United States Dept. of HHS; National Institute of Health (NIH) Guidelines for Recombinant DNA Research. The gene product encoded by a polynucleotide of the invention is expressed in any expression system, including, for example, bacterial, yeast, insect, amphibian and mammalian systems. Vectors, host cells and methods for obtaining expression in same are well known in the art. Suitable vectors and host cells are described in USPN 5,654,173.

Polynucleotide molecules comprising a polynucleotide sequence provided herein are generally propagated by placing the molecule in a vector. Viral and non-viral vectors are used, including plasmids. The choice of plasmid will depend on the type of cell in which propagation is desired and the purpose of propagation. Certain vectors are useful for amplifying and making large amounts of the desired DNA sequence. Other vectors are suitable for expression in cells in culture. Still other vectors are suitable for transfer and expression in cells in a whole animal or person. The choice of appropriate vector is well within the skill of the art. Many such vectors are available commercially. Methods for preparation of vectors comprising a desired sequence are well known in the art.

The polynucleotides set forth in SEQ ID NO:1 or their corresponding full-length polynucleotides are linked to regulatory sequences as appropriate to obtain the desired expression properties. These can include promoters (attached either at the 5' end of the sense strand or at the 3' end of the antisense strand), enhancers, terminators, operators, repressors, and inducers. The promoters can be regulated or constitutive. In some situations it may be desirable to use conditionally active promoters, such as tissue-specific or developmental stage-specific promoters. These are linked to the desired nucleotide sequence using the techniques described above for linkage to vectors. Any techniques known in the art can be used.

When any of the above host cells, or other appropriate host cells or organisms, are used to replicate and/or express the polynucleotides or nucleic acids of the invention, the resulting replicated nucleic acid, RNA, expressed protein or polypeptide, is within the scope of the invention as a product of the host cell or organism. The product is recovered by any appropriate means known in the art.

Once the gene corresponding to a selected polynucleotide is identified, its expression can be regulated in the cell to which the gene is native. For example, an endogenous gene of a cell can be regulated by an exogenous regulatory sequence as disclosed in USPN 5,641,670.

### Identification of Functional and Structural Motifs of Genes Screening Against Publicly Available Databases

Translations of the nucleotide sequence of the provided polynucleotides, cDNAs or full genes can be aligned with individual known sequences. Similarity with individual sequences can be used to determine the activity of the polypeptides encoded by the polynucleotides of the invention. Also, sequences exhibiting similarity with more than one individual sequence can exhibit activities that are characteristic of either or both individual sequences.

The full length sequences and fragments of the polynucleotide sequences of the nearest neighbors can be used as probes and primers to identify and isolate the full length sequence corresponding to provided polynucleotides. The nearest neighbors can indicate a tissue or cell type to be used to construct a library for the full-length sequences corresponding to the provided polynucleotides.

Typically, a selected polynucleotide is translated in all six frames to determine the best alignment with the individual sequences. The sequences disclosed herein in the Sequence Listing are in a 5' to 3' orientation and translation in three frames can be sufficient (with a few specific exceptions as described in the Examples). These amino acid sequences are referred to, generally, as query sequences, which will be aligned with the individual sequences. Databases with individual sequences are described in "Computer Methods for Macromolecular Sequence Analysis" Methods in Enzymology (1996) 266, Doolittle, Academic Press, Inc., a division of Harcourt Brace & Co., San Diego, California, USA. Databases include GenBank, EMBL, and DNA Database of Japan (DDBJ).

Query and individual sequences can be aligned using the methods and computer programs described above, and include BLAST 2.0, available over the world wide web at http://www.ncbi.nlm.nih.govBLAST/. See also Altschul, et al. Nucleic Acids Res. (1997) 25:3389-3402. Another alignment algorithm is Fasta, available in the Genetics Computing Group (GCG) package, Madison, Wisconsin, USA, a wholly owned subsidiary of Oxford Molecular Group, Inc. Other techniques for alignment are described in Doolittle, *supra.* Preferably, an alignment program that permits gaps in the sequence is utilized to align the sequences. The Smith-Waterman is one type of algorithm that permits gaps in sequence alignments. See Meth. Mol. Biol. (1997) 70: 173-187. Also, the GAP program using the Needleman and Wunsch alignment method can be utilized to align sequences.

An alternative search strategy uses MPSRCH software, which runs on a MASPAR computer. MPSRCH uses a Smith-Waterman algorithm to score sequences on a massively parallel computer. This approach improves ability to identify sequences that are distantly related matches, and is especially tolerant of small gaps and nucleotide sequence errors. Amino acid sequences encoded by the provided polynucleotides can be used to search both protein and DNA databases.

Results of individual and query sequence alignments can be divided into three categories: high similarity, weak similarity, and no similarity. Individual alignment results ranging from high similarity to weak similarity provide a basis for determining polypeptide activity and/or structure. Parameters for categorizing individual results include: percentage of the alignment region length where the strongest alignment is found, percent sequence identity, and p value. The percentage of the alignment region length is calculated by counting the number of residues of the individual sequence found in the region of strongest alignment, e.g., contiguous region of the individual sequence that contains the greatest number of residues that are identical to the residues of the corresponding region of the aligned query sequence. This number is divided by the total residue length of the query sequence to calculate a percentage. For example, a query sequence of 20 amino acid residues might be aligned with a 20 amino acid region of an individual sequence. The individual sequence might be identical to amino acid residues 5, 9-15, and 17-19 of the query sequence. The region of strongest alignment is thus the region stretching from residue 9-19, an 11 amino acid stretch. The percentage of the alignment region length is: 11 (length of the region of strongest alignment) divided by (query sequence length) 20 or 55%.

Percent sequence identity is calculated by counting the number of amino acid matches between the query and individual sequence and dividing total number of matches by the number of residues of the individual sequences found in the region of strongest alignment. Thus, the percent identity in the example above would be 10 matches divided by 11 amino acids, or approximately, 90.9%

P value is the probability that the alignment was produced by chance. For a single alignment, the p value can be calculated according to Karlin et al., Proc. Natl. Acad. Sci. (1990) 87:2264 and Karlin et al., Proc. Natl. Acad. Sci. (1993) 90. The p value of multiple alignments using the same query sequence can be calculated using an heuristic approach described in Altschul et al., Nat. Genet. (1994) 6:119. Alignment programs such as BLAST program can calculate the p value. See also Altschul et al., Nucleic Acids Res. (1997) 25:3389-3402.

Another factor to consider for determining identity or similarity is the location of the similarity or identity. Strong local alignment can indicate similarity even if the length of alignment is short. Sequence identity scattered throughout the length of the query sequence also can indicate a similarity between the query and profile sequences. The boundaries of the region where the sequences align can be determined according to Doolittle, *supra;* BLAST 2.0 (see, *e.g*., Altschul, et al. Nucleic Acids Res. (1997) 25:3389-3402) or FAST programs; or by determining the area where sequence identity is highest.

High Similarity, In general, in alignment results considered to be of high similarity, the percent of the alignment region length is typically at least about 55% of total length query sequence; more typically, at least about 58%; even more typically; at least about 60% of the total residue length of the query sequence. Usually, percent length of the alignment region can be as much as about 62%; more usually, as much as about 64%; even more usually, as much as about 66%. Further, for high similarity, the region of alignment, typically, exhibits at least about 75% of sequence identity; more typically, at least about 78%; even more typically; at least about 80% sequence identity. Usually, percent sequence identity can be as much as about 82%; more usually, as much as about 84%; even more usually, as much as about 86%.

The p value is used in conjunction with these methods. If high similarity is found, the query sequence is considered to have high similarity with a profile sequence when the p value is less than or equal to about 10⁻²; more usually; less than or equal to about 10⁻³ ; even more usually; less than or equal to about 10⁻⁴. More typically, the p value is no more than about 10⁻⁵; more typically; no more than or equal to about 10⁻¹⁰; even more typically; no more than or equal to about 10⁻¹⁵ for the query sequence to be considered high similarity.

Weak Similarity. In general, where alignment results considered to be of weak similarity, there is no minimum percent length of the alignment region nor minimum length of alignment. A better showing of weak similarity is considered when the region of alignment is, typically, at least about 15 amino acid residues in length; more typically, at least about 20; even more typically; at least about 25 amino acid residues in length. Usually, length of the alignment region can be as much as about 30 amino acid residues; more usually, as much as about 40; even more usually, as much as about 60 amino acid residues. Further, for weak similarity, the region of alignment, typically, exhibits at least about 35% of sequence identity; more typically, at least about 40%; even more typically; at least about 45% sequence identity. Usually, percent sequence identity can be as much as about 50%; more usually, as much as about 55%; even more usually, as much as about 60%.

If low similarity is found, the query sequence is considered to have weak similarity with a profile sequence when the p value is usually less than or equal to about 10⁻²; more usually; less than or equal to about 10-³; even more usually; less than or equal to about 10⁻⁴. More typically, the p value is no more than about 10⁻⁵; more usually; no more than or equal to about 10⁻¹⁰; even more usually; no more than or equal to about 10⁻¹⁵ for the query sequence to be considered weak similarity.

Similarity Determined by Sequence Identity Alone. Sequence identity alone can be used to determine similarity of a query sequence to an individual sequence and can indicate the activity of the sequence. Such an alignment, preferably, permits gaps to align sequences. Typically, the query sequence is related to the profile sequence if the sequence identity over the entire query sequence is at least about 15%; more typically, at least about 20%; even more typically, at least about 25%; even more typically, at least about 50%. Sequence identity alone as a measure of similarity is most useful when the query sequence is usually, at least 80 residues in length; more usually, 90 residues; even more usually, at least 95 amino acid residues in length. More typically, similarity can be concluded based on sequence identity alone when the query sequence is preferably 100 residues in length; more preferably, 120 residues in length; even more preferably, 150 amino acid residues in length.

Alignments with Profile and Multiple Aligned Sequences. Translations of the provided polynucleotides can be aligned with amino acid profiles that define either protein families or common motifs. Also, translations of the provided polynucleotides can be aligned to multiple sequence alignments (MSA) comprising the polypeptide sequences of members of protein families or motifs. Similarity or identity with profile sequences or MSAs can be used to determine the activity of the gene products (*e.g*., polypeptides) encoded by the provided polynucleotides or corresponding cDNA or genes. For example, sequences that show an identity or similarity with a chemokine profile or MSA can exhibit chemokine activities.

Profiles can designed manually by (1) creating an MSA, which is an alignment of the amino acid sequence of members that belong to the family and (2) constructing a statistical representation of the alignment. Such methods are described, for example, in Birney et al., Nucl. Acid Res. (1996) 24(14):2730-2739. MSAs of some protein families and motifs are publicly available. For example, http://genome.wustl.edu/Pfam/ includes MSAs of 547 different families and motifs. These MSAs are described also in Sonnhammer et al., Proteins (1997) 28: 405-420. Other sources over the world wide web include the site at http://www.embl-heidelberg.de/argos/ali/ali.html; alternatively, a message can be sent to ALI@EMBL-HEIDELBERG.DE for the information. A brief description of these MSAs is reported in Pascarella et al., Prot. Eng. (1996) 9(3):249-251. Techniques for building s from MSAs are described in Sonnhammer *et al., supra;* Birney *et al., supra;* and "Computer Methods for Macromolecular Sequence Analysis,"Methods in Enzymology (1996) 266, Doolittle, Academic Press, Inc., San Diego, California, USA.

Similarity between a query sequence and a protein family or motif can be determined by (a) comparing the query sequence against the profile and/or (b) aligning the query sequence with the members of the family or motif. Typically, a program such as Searchwise is used to compare the query sequence to the statistical representation of the multiple alignment, also known as a profile (see Birney *et al., supra*)*.* Other techniques to compare the sequence and profile are described in Sonnhammer *et al., supra* and Doolittle, *supra.*

Next, methods described by Feng et al., J. Mol. Evol. (1987) 25:351 and Higgins et al., CABIOS (1989) 5:151 can be used align the query sequence with the members of a family or motif, also known as a MSA. Sequence alignments can be generated using any of a variety of software tools. Examples include PileUp, which creates a multiple sequence alignment, and is described in Feng et al., J. Mol. Evol. (1987) 25:351. Another method, GAP, uses the alignment method of Needleman et al., J. Mol. Biol. (1970) 48:443. GAP is best suited for global alignment of sequences. A third method, BestFit, functions by inserting gaps to maximize the number of matches using the local homology algorithm of Smith et al., Adv. Appl. Math. (1981) 2:482. In general, the following factors are used to determine if a similarity between a query sequence and a profile or MSA exists: (1) number of conserved residues found in the query sequence, (2) percentage of conserved residues found in the query sequence, (3) number of frameshifts, and (4) spacing between conserved residues.

Some alignment programs that both translate and align sequences can make any number of frameshifts when translating the nucleotide sequence to produce the best alignment. The fewer frameshifts needed to produce an alignment, the stronger the similarity or identity between the query and profile or MSAs. For example, a weak similarity resulting from no frameshifts can be a better indication of activity or structure of a query sequence, than a strong similarity resulting from two frameshifts. Preferably, three or fewer frameshifts are found in an alignment; more preferably two or fewer frameshifts; even more preferably, one or fewer frameshifts; even more preferably, no frame shifts are found in an alignment of query and profile or MSAs.

Conserved residues are those amino acids found at a particular position in all or some of the family or motif members. Alternatively, a position is considered conserved if only a certain class of amino acids is found in a particular position in all or some of the family members. For example, the N-terminal position can contain a positively charged amino acid, such as lysine, arginine, or histidine.

Typically, a residue of a polypeptide is conserved when a class of amino acids or a single amino acid is found at a particular position in at least about 40% of all class members; more typically, at least about 50%; even more typically, at least about 60% of the members. Usually, a residue is conserved when a class or single amino acid is found in at least about 70% of the members of a family or motif; more usually, at least about 80%; even more usually, at least about 90%; even more usually, at least about 95%.

A residue is considered conserved when three unrelated amino acids are found at a particular position in the some or all of the members; more usually, two unrelated amino acids. These residues are conserved when the unrelated amino acids are found at particular positions in at least about 40% of all class member; more typically, at least about 50%; even more typically, at least about 60% of the members. Usually, a residue is conserved when a class or single amino acid is found in at least about 70% of the members of a family or motif; more usually, at least about 80%; even more usually, at least about 90%; even more usually, at least about 95%.

A query sequence has similarity to a profile or MSA when the query sequence comprises at least about 25% of the conserved residues of the profile or MSA; more usually, at least about 30%; even more usually; at least about 40%. Typically, the query sequence has a stronger similarity to a profile sequence or MSA when the query sequence comprises at least about 45% of the conserved residues of the profile or MSA; more typically, at least about 50%; even more typically; at least about 55%.

### Identification of Secreted & Membrane-Bound Polypeptides

Both secreted and membrane-bound polypeptides of the present invention are of particular interest. For example, levels of secreted polypeptides can be assayed in body fluids that are convenient, such as blood, plasma, serum, and other body fluids such as urine, prostatic fluid and semen. Membrane-bound polypeptides are useful for constructing vaccine antigens or inducing an immune response. Such antigens would comprise all or part of the extracellular region of the membrane-bound polypeptides. Because both secreted and membrane-bound polypeptides comprise a fragment of contiguous hydrophobic amino acids, hydrophobicity predicting algorithms can be used to identify such polypeptides.

A signal sequence is usually encoded by both secreted and membrane-bound polypeptide genes to direct a polypeptide to the surface of the cell. The signal sequence usually comprises a stretch of hydrophobic residues. Such signal sequences can fold into helical structures. Membrane-bound polypeptides typically comprise at least one transmembrane region that possesses a stretch of hydrophobic amino acids that can transverse the membrane. Some transmembrane regions also exhibit a helical structure. Hydrophobic fragments within a polypeptide can be identified by using computer algorithms. Such algorithms include Hopp & Woods,, Proc. Natl. Acad. Sci. USA (1981) 78:3824-3828; Kyte & Doolittle, J. Mol. Biol. (1982) 157: 105-132; and RAOAR algorithm, Degli Esposti et al., Eur. J. Biochem. (1990) 190: 207-219.

Another method of identifying secreted and membrane-bound polypeptides is to translate the polynucleotides of the invention in all six frames and determine if at least 8 contiguous hydrophobic amino acids are present. Those translated polypeptides with at least 8; more typically, 10; even more typically, 12 contiguous hydrophobic amino acids are considered to be either a putative secreted or membrane bound polypeptide. Hydrophobic amino acids include alanine, glycine, histidine, isoleucine, leucine, lysine, methionine, phenylalanine, proline, threonine, tryptophan, tyrosine, and valine.

### Identification of the Function of an Expression Product of a Full-Length Gene

Ribozymes, antisense constructs, and dominant negative mutants can be used to determine function of the expression product of a gene corresponding to a polynucleotide provided herein, and further can be used in inhibition of production of functional gene products encoded by a gene corresponding to a polynucleotide described herein. In the context of functional characterization of the encoded gene product, use of antisense, ribozymes, and/or dominant negative mutants is particularly useful where the provided polynucleotide exhibits no significant or substantial homology to a sequence encoding a gene of known function.

Antisense molecules and ribozymes can be constructed from synthetic polynucleotides. Typically, the phosphoramidite method of oligonucleotide synthesis is used. See Beaucage et al., Tet. Lett. (1981) 22:1859 and USPN 4,668,777. Automated devices for synthesis are available to create oligonucleotides using this chemistry. Examples of such devices include Biosearch 8600, Models 392 and 394 by Applied Biosystems, a division of Perkin-Elmer Corp., Foster City, California, USA; and Expedite by Perceptive Biosystems, Framingham, Massachusetts, USA. Synthetic RNA, phosphate analog oligonucleotides, and chemically derivatized oligonucleotides can also be produced, and can be covalently attached to other molecules. RNA oligonucleotides can be synthesized, for example, using RNA phosphoramidites. This method can be performed on an automated synthesizer, such as Applied Biosystems, Models 392 and 394, Foster City, California, USA.

Phosphorothioate oligonucleotides can also be synthesized for antisense construction. A sulfurizing reagent, such as tetraethylthiruam disulfide (TETD) in acetonitrile can be used to convert the internucleotide cyanoethyl phosphite to the phosphorothioate triester within 15 minutes at room temperature. TETD replaces the iodine reagent, while all other reagents used for standard phosphoramidite chemistry remain the same. Such a synthesis method can be automated using Models 392 and 394 by Applied Biosystems, for example.

Oligonucleotides of up to 200 nt can be synthesized, more typically, 100 nt, more typically 50 nt; even more typically 30 to 40 nt. These synthetic fragments can be annealed and ligated together to construct larger fragments. See, for example, *Sambrook et al., supra.* Trans-cleaving catalytic RNAs (ribozymes) are RNA molecules possessing endoribonuclease activity. Ribozymes are specifically designed for a particular target, and the target message must contain a specific nucleotide sequence. They are engineered to cleave any RNA species site-specifically in the background of cellular RNA. The cleavage event renders the mRNA unstable and prevents protein expression. Importantly, ribozymes can be used to inhibit expression of a gene of unknown function for the purpose of determining its function in an in *vitro* or *in vivo* context, by detecting the phenotypic effect. One commonly used ribozyme motif is the hammerhead, for which the substrate sequence requirements are minimal. Design of the hammerhead ribozyme, as well as therapeutic uses of ribozymes, are disclosed in Usman et al., Current Opin. Struct. Biol. (1996) 6:527. Methods for production of ribozymes, including hairpin structure ribozyme fragments, methods of increasing ribozyme specificity, and the like are known in the art.

The hybridizing region of the ribozyme can be modified or can be prepared as a branched structure as described in Horn and Urdea, Nucleic Acids Res. (1989) 17:6959. The basic structure of the ribozymes can also be chemically altered in ways familiar to those skilled in the art, and chemically synthesized ribozymes can be administered as synthetic oligonucleotide derivatives modified by monomeric units. In a therapeutic context, liposome mediated delivery of ribozymes improves cellular uptake, as described in Birikh et al., Eur. J. Biochem. (1997) 245:1.

Antisense nucleic acids are designed to specifically bind to RNA, resulting in the formation of RNA-DNA or RNA-RNA hybrids, with an arrest of DNA replication, reverse transcription or messenger RNA translation. Antisense polynucleotides based on a selected polynucleotide sequence can interfere with expression of the corresponding gene. Antisense polynucleotides are typically generated within the cell by expression from antisense constructs that contain the antisense strand as the transcribed strand. Antisense polynucleotides based on the disclosed polynucleotides will bind and/or interfere with the translation of mRNA comprising a sequence complementary to the antisense polynucleotide. The expression products of control cells and cells treated with the antisense construct are compared to detect the protein product of the gene corresponding to the polynucleotide upon which the antisense construct is based. The protein is isolated and identified using routine biochemical methods.

Given the extensive background literature and clinical experience in antisense therapy, one skilled in the art can use selected polynucleotides of the invention as additional potential therapeutics. The choice of polynucleotide can be narrowed by first testing them for binding to "hot spot" regions of the genome of cancerous colon cells. If a polynucleotide is identified as binding to a "hot spot", testing the polynucleotide as an antisense compound in the corresponding colon cancer cells is warranted.

As an alternative method for identifying function of the gene corresponding to a polynucleotide disclosed herein, dominant negative mutations are readily generated for corresponding proteins that are active as homomultimers. A mutant polypeptide will interact with wild-type polypeptides (made from the other allele) and form a non-functional multimer. Thus, a mutation is in a substrate-binding domain, a catalytic domain, or a cellular localization domain. Preferably, the mutant polypeptide will be overproduced. Point mutations are made that have such an effect. In addition, fusion of different polypeptides of various lengths to the terminus of a protein can yield dominant negative mutants. General strategies are available for making dominant negative mutants (see, *e.g*., Herskowitz, Nature (1987) 329:219). Such techniques can be used to create loss of function mutations, which are useful for determining protein function.

### Polypeptides and Variants Thereof

The polypeptides of the invention include those encoded by the disclosed polynucleotides, as well as nucleic acids that, by virtue of the degeneracy of the genetic code, are not identical in sequence to the disclosed polynucleotides. Thus, the invention includes within its scope a polypeptide encoded by a polynucleotide comprising the sequence of SEQ ID NO:1 or a variant thereof. Exemplary polypeptides encoded by an open reading frame of a polynucleotide described herein include SEQ ID NOS:2.

In general, the term "polypeptide" as used herein refers to both the full length polypeptide encoded by the recited polynucleotide, the polypeptide encoded by the gene represented by the recited polynucleotide, as well as portions or fragments thereof "Polypeptides" also includes variants of the naturally occurring proteins, where such variants are homologous or substantially similar to the naturally occurring protein, and can be of an origin of the same or different species as the naturally occurring protein (e.g., human, murine, or some other species that naturally expresses the recited polypeptide, usually a mammalian species). In general, variant polypeptides have a sequence that has at least about 80%, usually at least about 90%, and more usually at least about 98% sequence identity with a differentially expressed polypeptide of the invention, as measured by BLAST 2.0 using the parameters described above. The variant polypeptides can be naturally or non-naturally glycosylated, *i.e.,* the polypeptide has a glycosylation pattern that differs from the glycosylation pattern found in the corresponding naturally occurring protein.

The invention also encompasses homologs of the disclosed polypeptides (or fragments thereof) where the homologs are isolated from other species, *i.e.* other animal or plant species, where such homologs, usually mammalian species, *e.g*. rodents, such as mice, rats; domestic animals, e.g., horse, cow, dog, cat; and humans. By "homolog" is meant a polypeptide having at least about 35%, usually at least about 40% and more usually at least about 60% amino acid sequence identity to a particular differentially expressed protein as identified above, where sequence identity is determined using the BLAST 2.0 algorithm, with the parameters described *supra.*

In general, the polypeptides of the subject invention are provided in a non-naturally occurring environment, e.g. are separated from their naturally occurring environment. In certain embodiments, the subject protein is present in a composition that is enriched for the protein as compared to a control. As such, purified polypeptide is provided, where by purified is meant that the protein is present in a composition that is substantially free of non-differentially expressed polypeptides, where by substantially free is meant that less than 90%, usually less than 60% and more usually less than 50% of the composition is made up of non-differentially expressed polypeptides.

Also within the scope of the invention are variants; variants of polypeptides include mutants, fragments, and fusions. Mutants can include amino acid substitutions, additions or deletions. The amino acid substitutions can be conservative amino acid substitutions or substitutions to eliminate non-essential amino acids, such as to alter a glycosylation site, a phosphorylation site or an acetylation site, or to minimize misfolding by substitution or deletion of one or more cysteine residues that are not necessary for function. Conservative amino acid substitutions are those that preserve the general charge; hydrophobicity/ hydrophilicity, and/or steric bulk of the amino acid substituted.

Variants can be designed so as to retain or have enhanced biological activity of a particular region of the protein (*e.g*., a functional domain and/or, where the polypeptide is a member of a protein family, a region associated with a consensus sequence). Selection of amino acid alterations for production of variants can be based upon the accessibility (interior vs. exterior) of the amino acid (see, *e.g.,* Go et al, Int. J. Peptide Protein Res. (1980) 15:211), the thermostability of the variant polypeptide (see, *e.g*., Querol et al., Prot. Eng. (1996) 9:265), desired glycosylation sites (see, *e.g.,* Olsen and Thomsen, J. Gen. Microbiol. (1991) 137:579), desired disulfide bridges (see, *e.g.,* Clarke et al., Biochemistry (1993) 32:4322; and Wakarchuk et al., Protein Eng. (1994) 7:1379), desired metal binding sites (see, *e.g*., Toma et al., Biochemistry (1991) 30:97, and Haezerbrouck et al., Protein Eng. (1993) 6:643), and desired substitutions with in proline loops (see, *e.g.,* Masul et al., Appl. Env Microbiol. (1994) 60:3579). Cysteine-depleted muteins can be produced as disclosed in USPN 4,959,314.

Variants also include fragments of the polypeptides disclosed herein, particularly biologically active fragments and/or fragments corresponding to functional domains. Fragments of interest will typically be at least about 10 aa to at least about 15 aa in length, usually at least about 50 aa in length, and can be as long as 300 aa in length or longer, but will usually not exceed about 1000 aa in length, where the fragment will have a stretch of amino acids that is identical to a polypeptide encoded by a polynucleotide comprising a sequence of SEQ ID NO:1 or a homolog thereof. The protein variants described herein are encoded by polynucleotides that are within the scope of the invention. The genetic code can be used to select the appropriate codons to construct the corresponding variants.

### Computer-Related Embodiments

In general, a library of polynucleotides is a collection of sequence information, which information is provided in either biochemical form (*e.g*., as a collection of polynucleotide molecules), or in electronic form (*e.g.*, as a collection of polynucleotide sequences stored in a computer-readable form, as in a computer system and/or as part of a computer program). The sequence information of the polynucleotides can be used in a variety of ways, *e.g*., as a resource for gene discovery, as a representation of sequences expressed in a selected cell type *(e.g.,* cell type markers), and/or as markers of a given disease or disease state. In general, a disease marker is a representation of a gene product that is present in all cells affected by disease either at an increased or decreased level relative to a normal cell (*e.g*., a cell of the same or similar type that is not substantially affected by disease). For example, a polynucleotide sequence in a library can be a polynucleotide that represents an mRNA, polypeptide, or other gene product encoded by the polynucleotide, that is either overexpressed or underexpressed in a colon cell affected by cancer relative to a normal *(i.e.,* substantially disease-free) colon cell.

The nucleotide sequence information of the library can be embodied in any suitable form, *e.g.,* electronic or biochemical forms. For example, a library of sequence information embodied in electronic form comprises an accessible computer data file (or, in biochemical form, a collection of nucleic acid molecules) that contains the representative nucleotide sequences of genes that are differentially expressed (*e.g*., overexpressed or underexpressed) as between, for example, i) a cancerous colon cell and a normal colon cell; ii) a cancerous colon cell and a dysplastic colon cell; iii) a cancerous colon cell and a colon cell affected by a disease or condition other than cancer; iv) a metastatic cancerous colon cell and a normal colon cell and/or non-metastatic cancerous colon cell; v) a malignant cancerous colon cell and a non-malignant cancerous colon cell (or a normal colon cell) and/or vi) a dysplastic colon cell relative to a normal colon cell. Other combinations and comparisons of colon cells affected by various diseases or stages of disease will be readily apparent to the ordinarily skilled artisan. Biochemical embodiments of the library include a collection of nucleic acids that have the sequences of the genes in the library, where the nucleic acids can correspond to the entire gene in the library or to a fragment thereof, as described in greater detail below.

The polynucleotide libraries of the subject invention generally comprise sequence information of a plurality of polynucleotide sequences, where at least one of the polynucleotides comprises a sequence of SEQ ID NO:1. By plurality is meant at least 2, usually at least 3. and can include up.to all of SEQ ID NOS:1, 3, 5, 7, 9, 11-13, 15, 16, 18, 20, 22, 24, 26, 2Z and 29. The length and number of polynucleotides in the library will vary with the nature of the library, e.g., if the library is an oligonucleotide array, a CDNA array, a computer database of the sequence information, *etc*

Where the library is an electronic library, the nucleic acid sequence information can be present in a variety of media. "Media" refers to a manufacture, other than an isolated nucleic acid molecule, that contains the sequence information of the present invention. Such a manufacture provides the genome sequence or a subset thereof in a form that can be examined by means not directly applicable to the sequence as it exists in a nucleic acid. For example, the nucleotide sequence of the present invention, e.g. the nucleic acid sequences of the polynucleotides of SEQ ID NO: 1, can be recorded on computer readable media, *e.g*. any medium that can be read and accessed directly by a computer. Such media include, but are not limited to: magnetic storage media, such as a floppy disc, a hard disc storage medium, and a magnetic tape; optical storage media such as CD-ROM; electrical storage media such as RAM and ROM; and hybrids of these categories such as magnetic/optical storage media.

One of skill in the art can readily appreciate how any of the presently known computer readable mediums can be used to create a manufacture comprising a recording of the present sequence information. "Recorded" refers to a process for storing information on computer readable medium, using any such methods as known in the art. Any convenient data storage structure can be chosen, based on the means used to access the stored information. A variety of data processor programs and formats can be used for storage, e.g. word processing text file, database format, *etc.* In addition to the sequence information, electronic versions of the libraries of the invention can be provided in conjunction or connection with other computer-readable information and/or other types of computer-readable files (*e.g.,* searchable files, executable files, *etc,* including, but not limited to, for example, search program software, *etc. ).*

By providing the nucleotide sequence in computer readable form, the information can be accessed for a variety of purposes. Computer software to access sequence information is publicly available. For example, the gapped BLAST (Altschul et al. Nucleic Acids Res. (1997) 25:3389-3402) and BLAZE (Brutlag et al. Comp. Chem. (1993) 17:203) search algorithms on a Sybase system can be used to identify open reading frames (ORFs) within the genome that contain homology to ORFs from other organisms.

As used herein, "a computer-based system" refers to the hardware means, software means, and data storage means used to analyze the nucleotide sequence information of the present invention. The minimum hardware of the computer-based systems of the present invention comprises a central processing unit (CPU), input means, output means, and data storage means. A skilled artisan can readily appreciate that any one of the currently available computer-based system are suitable for use in the present invention. The data storage means can comprise any manufacture comprising a recording of the present sequence information as described above, or a memory access means that can access such a manufacture.

"Search means" refers to one or more programs implemented on the computer-based system, to compare a target sequence or target structural motif, or expression levels of a polynucleotide in a sample, with the stored sequence information. Search means can be used to identify fragments or regions of the genome that match a particular target sequence or target motif. A variety of known algorithms are publicly known and commercially available, *e.g*. MacPattern (EMBL), BLASTN and BLASTX (NCBI). A "target sequence" can be any polynucleotide or amino acid sequence of six or more contiguous nucleotides or two or more amino acids, preferably from about 10 to 100 amino acids or from about 30 to 300 nt A variety of comparing means can be used to accomplish comparison of sequence information from a sample (*e.g*., to analyze target sequences, target motifs, or relative expression levels) with the data storage means. A skilled artisan can readily recognize that any one of the publicly available homology search programs can be used as the search means for the computer based systems of the present invention to accomplish comparison of target sequences and motifs. Computer programs to analyze expression levels in a sample and in controls are also known in the art.

A "target structural motif," or "target motif," refers to any rationally selected sequence or combination of sequences in which the sequence(s) are chosen based on a three-dimensional configuration that is formed upon the folding of the target motif, or on consensus sequences of regulatory or active sites. There are a variety of target motifs known in the art Protein target motifs include, but arc not limited to, enzyme active sites and signal sequences. Nucleic acid target motifs include, but are not limited to, hairpin structures, promoter sequences and other expression elements such as binding sites for transcription factors.

A variety of structural formats for the input and output means can be used to input and output the information in the computer-based systems of the present invention. One format for an output means ranks the relative expression levels of different polynucleotides. Such presentation provides a skilled artisan with a ranking of relative expression levels to determine a gene expression profile.

As discussed above, the "library" of the invention also encompasses biochemical libraries of the polynucleotide of SEQ ID NO:1, *e.g.,* collections of nucleic acids representing the provided polynucleotides. The biochemical libraries can take a variety of forms, *e.g*., a solution of cDNAs, a pattern of probe nucleic acids stably associated with a surface of a solid support (*i.e*., an array) and the like. Of particular interest are nucleic acid arrays in which SEQ ID NO:1 is represented on the array. By array is meant a an article of manufacture that has at least a substrate with at least two distinct nucleic acid targets on one of its surfaces, where the number of distinct nucleic acids can be considerably higher, typically being at least 10 nt, usually at least 20 nt and often at least 25 nt A variety of different array formats have been developed and are known to those of skill in the art. The arrays of the subject invention find use in a variety of applications, including gene expression analysis, drug screening, mutation analysis and the like, as disclosed in the above-listed exemplary patent documents.

In addition to the above nucleic acid libraries, analogous libraries of polypeptides are also provided, where the polypeptides of the library will represent at least a portion of the polypeptides encoded by SEQ ID NO:1.

### Utilities

Polynucleotide probes, generally comprising at least 12 contiguous nt of a polynucleotide as shown in the Sequence Listing, are used for a variety of purposes, such as chromosome mapping of the polynucleotide and detection of transcription levels. Additional disclosure about preferred regions of the disclosed polynucleotide sequences is found in the Examples. A probe that hybridizes specifically to a polynucleotide disclosed herein should provide a detection signal at least 5-, 10-, or 20-fold higher than the background hybridization provided with other unrelated sequences.

Detection of Expression Levels. Nucleotide probes are used to detect expression of a gene corresponding to the provided polynucleotide. In Northern blots, mRNA is separated electrophoretically and contacted with a probe. A probe is detected as hybridizing to an mRNA species of a particular size. The amount of hybridization is quantitated to determine relative amounts of expression, for example under a particular condition. Probes are used for in situ hybridization to cells to detect expression. Probes can also be used *in vivo* for diagnostic detection of hybridizing sequences. Probes are typically labeled with a radioactive isotope. Other types of detectable labels can be used such as chromophores, fluors, and enzymes. Other examples of nucleotide hybridization assays are described in WO92/02526 and USPN 5,124,246.

Alternatively, the Polymerase Chain Reaction (PCR) is another means for detecting small amounts of target nucleic acids (see, *e.g*., Mullis et al., Meth. Enzymol. (1987) 155:335; USPN 4,683,195; and USPN 4,683,202). Two primer polynucleotides nucleotides that hybridize with the target nucleic acids are used to prime the reaction. The primers can be composed of sequence within or 3' and 5' to the polynucleotides of the Sequence Listing. Alternatively, if the primers are 3' and 5' to these polynucleotides, they need not hybridize to them or the complements. After amplification of the target with a thermostable polymerase, the amplified target nucleic acids can be detected by methods known in the art, *e.g.,* Southern blot. mRNA or cDNA can also be detected by traditional blotting techniques (*e.g.,* Southern blot, Northern blot, *etc.)* described in Sambrook et al., "Molecular Cloning: A Laboratory Manual" (New York, Cold Spring Harbor Laboratory, 1989) (*e.g*., without PCR amplification). In general, mRNA or cDNA generated from mRNA using a polymerase enzyme can be purified and separated using gel electrophoresis, and transferred to a solid support, such as nitrocellulose. The solid support is exposed to a labeled probe, washed to remove any unhybridized probe, and duplexes containing the labeled probe are detected.

Mapping. Polynucleotides of the present invention can be used to identify a chromosome on which the corresponding gene resides. Such mapping can be useful in identifying the function of the polynucleotide-related gene by its proximity to other genes with known function. Function can also be assigned to the polynucleotide-related gene when particular syndromes or diseases map to the same chromosome. For example, use of polynucleotide probes in identification and quantification of nucleic acid sequence aberrations is described in USPN 5,783,387. An exemplary mapping method is fluorescence in situ hybridization (FISH), which facilitates comparative genomic hybridization to allow total genome assessment of changes in relative copy number of DNA sequences (see, *e.g*., Valdes et al., Methods in Molecular Biology (1997) 68:1)*.* Polynucleotides can also be mapped to particular chromosomes using, for example, radiation hybrids or chromosome-specific hybrid panels. See Leach et al., Advances in Genetics, (1995) 33:63-99; Walter et al., Nature Genetics (1994) 7:22; Walter and Goodfellow, Trends in Genetics (1992) 9:352. Panels for radiation hybrid mapping are available from Research Genetics, Inc., Huntsville, Alabama, USA. Databases for markers using various panels are available via the world wide web at http:/F/shgo-www.stanford.edu; and http://www-genome.wi.mit.edulcgi-bin/contig/rhmapper.pl. The statistical program RHIVIAP can be used to construct a map based on the data from radiation hybridization with a measure of the relative likelihood of one order versus another. RHMAP is available via the world wide web at http://www.sph.umich.edu/group/statgen/software. In addition, commercial programs are available for identifying regions of chromosomes commonly associated with disease, such as cancer.

Tissue Typing or Profiling. Expression of specific mRNA corresponding to the provided polynucleotides can vary in different cell types and can be tissue-specific. This variation of mRNA levels in different cell types can be exploited with nucleic acid probe assays to determine tissue types. For example, PCR, branched DNA probe assays, or blotting techniques utilizing nucleic acid probes substantially identical or complementary to polynucleotides listed in the Sequence Listing can determine the presence or absence of the corresponding cDNA or mRNA.

Tissue typing can be used to identify the developmental organ or tissue source of a metastatic lesion by identifying the expression of a particular marker of that organ or tissue. If a polynucleotide is expressed only in a specific tissue type, and a metastatic lesion is found to express that polynucleotide, then the developmental source of the lesion has been identified. Expression of a particular polynucleotide can be assayed by detection of either the corresponding mRNA or the protein product. As would be readily apparent to any forensic scientist, the sequences disclosed herein are useful in differentiating human tissue from non-human tissue. In particular, these sequences are useful to differentiate human tissue from bird, reptile, and amphibian tissue, for example.

Use of Polymorphisms. A polynucleotide of the invention can be used in forensics, genetic analysis, mapping, and diagnostic applications where the corresponding region of a gene is polymorphic in the human population. Any means for detecting a polymorphism in a gene can be used, including, but not limited to electrophoresis of protein polymorphic variants, differential sensitivity to restriction enzyme cleavage, and hybridization to allele-specific probes.

### Antibody Production

Expression products of a polynucleotide of the invention, as well as the corresponding mRNA, cDNA, or complete gene, can be prepared and used for raising antibodies for experimental, diagnostic, and therapeutic purposes. For polynucleotides to which a corresponding gene has not been assigned, this provides an additional method of identifying the corresponding gene. The polynucleotide or related cDNA is expressed as described above, and antibodies are prepared. These antibodies are specific to an epitope on the polypeptide encoded by the polynucleotide, and can precipitate or bind to the corresponding native protein in a cell or tissue preparation or in a cell-free extract of an *in vitro* expression system.

Methods for production of antibodies that specifically bind a selected antigen are well known in the art. Immunogens for raising antibodies can be prepared by mixing a polypeptide encoded by a polynucleotide of the invention with an adjuvant, and/or by making fusion proteins with larger immunogenic proteins. Polypeptides can also be covalently linked to other larger immunogenic proteins, such as keyhole limpet hemocyanin. Immunogens are typically administered intradermally, subcutaneously, or intramuscularly to experimental animals such as rabbits, sheep, and mice, to generate antibodies. Monoclonal antibodies can be generated by isolating spleen cells and fusing myeloma cells to form hybridomas. Alternatively, the selected polynucleotide is administered directly, such as by intramuscular injection, and expressed *in vivo.* The expressed protein generates a variety of protein-specific immune responses, including production of antibodies, comparable to administration of the protein.

Preparations of polyclonal and monoclonal antibodies specific for polypeptides encoded by a selected polynucleotide are made using standard methods known in the art. The antibodies specifically bind to epitopes present in the polypeptides encoded by polynucleotides disclosed in the Sequence Listing. Typically, at least 6, 8, 10, or 12 contiguous amino acids are required to form an epitope. Epitopes that involve non-contiguous amino acids may require a longer polypeptide, e.g., at least 15, 25, or 50 amino acids. Antibodies that specifically bind to human polypeptides encoded by the provided polypeptides should provide a detection signal at least 5-, 10-, or 20-fold higher than a detection signal provided with other proteins when used in Western blots or other immunochemical assays. Preferably, antibodies that specifically bind polypeptides of the invention do not bind to other proteins in immunochemical assays at detectable levels and can immunoprecipitate the specific polypeptide from solution.

The invention also contemplates naturally occurring antibodies specific for a polypeptide of the invention. For example, serum antibodies to a polypeptide of the invention in a human population can be purified by methods well known in the art, *e.g*., by passing antiserum over a column to which the corresponding selected polypeptide or fusion protein is bound. The bound antibodies can then be eluted from the column, for example using a buffer with a high salt concentration.

In addition to the antibodies discussed above, the invention also contemplates genetically engineered antibodies, antibody derivatives (*e.g*., single chain antibodies, antibody fragments (*e.g*., Fab, *etc*.)), according to methods well known in the art.

### Diagnostic and Other Methods Involving Detection of Differentially Expressed Gene Products

The present invention provides methods of using the polynucleotides described herein. In specific non-limiting embodiments, the methods are useful for detecting colon cancer cells, facilitating diagnosis of cancer and the severity of a cancer (*e.g*., tumor grade, tumor burden, and the like) in a subject, facilitating a determination of the prognosis of a subject, and assessing the responsiveness of the subject to therapy (*e.g*., by providing a measure of therapeutic effect through, for example, assessing tumor burden during or following a chemotherapeutic regimen). Detection can be based on detection of a polynucleotide that is differentially expressed in a colon cancer cell, and/or detection of a polypeptide encoded by a polynucleotide that is differentially expressed in a colon cancer cell ("a polypeptide associated with colon cancer"). The detection methods of the invention can be conducted *in vitro* or *in vivo,* on isolated cells, or in whole tissues or a bodily fluid, *e.g*., blood, plasma, serum, urine, and the like).

In general, methods of the invention involving detection of a gene product (*e.g*., mRNA, cDNA generated from such mRNA, and polypeptides) involves contacting a sample with a probe specific for the gene product of interest. "Probe" as used herein in such methods is meant to refer to a molecule that specifically binds a gene product of interest (*e.g*., the probe binds to the target gene product with a specificity sufficient to distinguish binding to target over non-specific binding to non-target (background) molecules). "Probes" include, but are not necessarily limited to, nucleic acid probes (*e.g*., DNA, RNA, modified nucleic acid, and the like), antibodies (*e.g*., antibodies, antibody fragments that retain binding to a target epitope, single chain antibodies, and the like), or other polypeptide, peptide, or molecule (*e.g.*, receptor ligand) that specifically binds a target gene product of interest.

The probe and sample suspected of having the gene product of interest are contacted under conditions suitable for binding of the probe to the gene product. For example, contacting is generally for a time sufficient to allow binding of the probe to the gene product (*e.g.*, from several minutes to a few hours), and at a temperature and conditions of osmolarity and the like that provide for binding of the probe to the gene product at a level that is sufficiently distinguishable from background binding of the probe (*e.g.,* under conditions that minimize non-specific binding). Suitable conditions for probe-target gene product binding can be readily determined using controls and other techniques available and known to one of ordinary skill in the art.

In this embodiment, the probe can be a an antibody or other polypeptide, peptide, or molecule (*e.g*., receptor ligand) that specifically binds a target polypeptide of interest.

The detection methods can be provided as part of a kit. Thus, the invention further provides kits for detecting the presence and/or a level of a polynucleotide that is differentially expressed in a colon cancer cell (*e.g.,* by detection of an mRNA encoded by the differentially expressed gene of interest), and/or a polypeptide encoded thereby, in a biological sample. Procedures using these kits can be performed by clinical laboratories, experimental laboratories, medical practitioners, or private individuals. The kits of the invention for detecting a polypeptide encoded by a polynucleotide that is differentially expressed in a colon cancer cell comprise a moiety that specifically binds the polypeptide, which may be a specific antibody. The kits of the invention for detecting a polynucleotide that is differentially expressed in a colon cancer cell comprise a moiety that specifically hybridizes to such a polynucleotide. The kit may optionally provide additional components that are useful in the procedure, including, but not limited to, buffers, developing reagents, labels, reacting surfaces, means for detection, control samples, standards, instructions, and interpretive information.

### Detecting a polypeptide encoded by a polynucleotide that is differentially expressed in a colon cancer cell

In some embodiments, methods are provided for a colon cancer cell by detecting in the cell a polypeptide encoded by a gene differentially expressed in a colon cancer cell. Any of a variety of known methods can be used for detection, including, but not limited to, immunoassay, using antibody specific for the encoded polypeptide, e.g., by enzyme-linked immunosorbent assay (ELISA), radioimmunoassay (RIA), and the like; and functional assays for the encoded polypeptide, e.g., binding activity or enzymatic activity.

For example, an immunofluorescence assay can be easily performed on cells without first isolating the encoded polypeptide. The cells are first fixed onto a solid support, such as a microscope slide or microtiter well. This fixing step can permeabilize the cell membrane. The permeablization of the cell membrane permits the polypeptide-specific probe (*e.g*, antibody) to bind. Alternatively, where the polypeptide is secreted or membrane-bound, or is otherwise accessible at the cell-surface (*e.g*., receptors, and other molecule stably-associated with the outer cell membrane or otherwise stably associated with the cell membrane, such permeabilization may not be necessary.

Next, the fixed cells are exposed to an antibody specific for the encoded polypeptide. To increase the sensitivity of the assay, the fixed cells may be further exposed to a second antibody, which is labeled and binds to the first antibody, which is specific for the encoded polypeptide. Typically, the secondary antibody is detectably labeled, e.g., with a fluorescent marker. The cells which express the encoded polypeptide will be fluorescently labeled and easily visualized under the microscope. See, for example, Hashido et al. (1992) Biochem. Biophys. Res. Comm. 187:1241-1248.

As will be readily apparent to the ordinarily skilled artisan upon reading the present specification, the detection methods and other methods described herein can be readily varied. Such variations are within the intended scope of the invention. For example, in the above detection scheme, the probe for use in detection can be immobilized on a solid support, and the test sample contacted with the immobilized probe. Binding of the test sample to the probe can then be detected in a variety of ways, *e.g*., by detecting a detectable label bound to the test sample to facilitate detected of test sample-immobilized probe complexes.

The present invention further provides methods for detecting the presence of and/or measuring a level of a polypeptide in a biological sample, which polypeptide is encoded by a polynucleotide that represents a gene differentially expressed in cancer, particularly in a colon cancer cell, using a probe specific for the encoded polypeptide. In this embodiment, the probe can be a an antibody or other polypeptide, peptide, or molecule (*e.g*., receptor ligand) that specifically binds a target polypeptide of interest.

The methods generally comprise: a) contacting the sample with an antibody specific for a differentially expressed polypeptide in a test cell; and b) detecting binding between the antibody and molecules of the sample. The level of antibody binding (either qualitative or quantitative) indicates the cancerous state of the cell. For example, where the differentially expressed gene is increased in cancerous cells, detection of an increased level of antibody binding to the test sample relative to antibody binding level associated with a normal cell indicates that the test cell is cancerous.

Suitable controls include a sample known not to contain the encoded polypeptide; and a sample contacted with an antibody not specific for the encoded polypeptide, e.g., an anti-idiotype antibody. A variety of methods to detect specific antibody-antigen interactions are known in the art and can be used in the method, including, but not limited to, standard immunohistological methods, immunoprecipitation, an enzyme immunoassay, and a radioimmunoassay.

In general, the specific antibody will be detectably labeled, either directly or indirectly. Direct labels include radioisotopes; enzymes whose products are detectable (e.g., luciferase, β-galactosidase, and the like); fluorescent labels (e.g., fluorescein isothiocyanate, rhodamine, phycoerythrin, and the like); fluorescence emitting metals, e.g., ¹⁵²Eu, or others of the lanthanide series, attached to the antibody through metal chelating groups such as EDTA; chemiluminescent compounds, e.g., luminol, isoluminol, acridinium salts, and the like; bioluminescent compounds, e.g., luciferin, aequorin (green fluorescent protein), and the like.

The antibody may be attached (coupled) to an insoluble support, such as a polystyrene plate or a bead. Indirect labels include second antibodies specific for antibodies specific for the encoded polypeptide ("first specific antibody"), wherein the second antibody is labeled as described above; and members of specific binding pairs, e.g., biotin-avidin, and the like. The biological sample may be brought into contact with and immobilized on a solid support or carrier, such as nitrocellulose, that is capable of immobilizing cells, cell particles, or soluble proteins. The support may then be washed with suitable buffers, followed by contacting with a detectably-labeled first specific antibody. Detection methods are known in the art and will be chosen as appropriate to the signal emitted by the detectable label. Detection is generally accomplished in comparison to suitable controls, and to appropriate standards.

In some embodiments, the methods are adapted for use *in vivo,* e.g., to locate or identify sites where colon cancer cells are present. In these embodiments, a detectably-labeled moiety, e.g., an antibody, which is specific for a colon cancer-associated polypeptide is administered to an individual (e.g., by injection), and labeled cells are located using standard imaging techniques, including, but not limited to, magnetic resonance imaging, computed tomography scanning, and the like. In this manner, colon cancer cells are differentially labeled.

### Detecting a polynucleotide that represents a gene differentially expressed in a colon cancer cell

In some embodiments, methods are provided for detecting a colon cancer cell by detecting expression in the cell of a transcript or that is differentially expressed in a colon cancer cell. Any of a variety of known methods can be used for detection, including, but not limited to, detection of a transcript by hybridization with a polynucleotide that hybridizes to a polynucleotide that is differentially expressed in a colon cancer cell; detection of a transcript by a polymerase chain reaction using specific oligonucleotide primers; *in situ* hybridization of a cell using as a probe a polynucleotide that hybridizes to a gene that is differentially expressed in a colon cancer cell.

The methods can be used to detect and/or measure mRNA levels of a gene that is differentially expressed in a colon cancer cell. In some embodiments, the methods comprise: a) contacting a sample with a polynucleotide that corresponds to a differentially expressed gene described herein under conditions that allow hybridization; and b) detecting hybridization, if any. Detection of differential hybridization, when compared to a suitable control, is an indication of the presence in the sample of a polynucleotide that is differentially expressed in a colon cancer cell. Appropriate controls include, for example, a sample which is known not to contain a polynucleotide that is differentially expressed in a colon cancer cell, and use of a labeled polynucleotide of the same "sense" as the polynucleotide that is differentially expressed in a colon cancer cell . Conditions that allow hybridization are known in the art, and have been described in more detail above.

Detection can also be accomplished by any known method, including, but not limited to, *in situ* hybridization, PCR (polymerase chain reaction), RT-PCR (reverse transcription-PCR), and "Northern" or RNA blotting, or combinations of such techniques, using a suitably labeled polynucleotide. A variety of labels and labeling methods for polynucleotides are known in the art and can be used in the assay methods of the invention. Specific hybridization can be determined by comparison to appropriate controls.

Polynucleotide generally comprising at least 12 contiguous nt of a polynucleotide provided herein, as shown in the Sequence Listing or of the sequences of the genes corresponding to the polynucleotides of the Sequence Listing, are used for a variety of purposes, such as probes for detection of and/or measurement of, transcription levels of a polynucleotide that is differentially expressed in a colon cancer cell. Additional disclosure about preferred regions of the disclosed polynucleotide sequences is found in the Examples. A probe that hybridizes specifically to a polynucleotide disclosed herein should provide a detection signal at least 5-, 10-, or 20-fold higher than the background hybridization provided with other unrelated sequences. It should be noted that "probe" as used in this context of detection of nucleic acid is meant to refer to a polynucleotide sequence used to detect a differentially expressed gene product in a test sample. As will be readily appreciated by the ordinarily skilled artisan, the probe can be detectably labeled and contacted with, for example, an array comprising immobilized polynucleotides obtained from a test sample (e.g., mRNA). Alternatively, the probe can be immobilized on an array and the test sample detectably labeled These and other variations of the methods of the invention are well within the skill in the art and are within the scope of the invention.

Nucleotide probes are used to detect expression of a gene corresponding to the provided polynucleotide. In Northern blots, mRNA is separated electrophoretically and contacted with a probe. A probe is detected as hybridizing to an mRNA species of a particular size. The amount of hybridization can be quantitated to determine relative amounts of expression, for example under a particular condition. Probes are used for in situ hybridization to cells to detect expression. Probes can also be used *in vivo* for diagnostic detection of hybridizing sequences. Probes are typically labeled with a radioactive isotope. Other types of detectable labels can be used such as chromophores, fluorophoress, and enzymes. Other examples of nucleotide hybridization assays are described in WO92/02526 and USPN 5,124,246.

PCR is another means for detecting small amounts of target nucleic acids (see, e.g., Mullis et al., Meth. Enzymol. (1987) 155:335; USPN 4,683,195; and USPN 4,683,202). Two primer polynucleotides nucleotides that hybridize with the target nucleic acids are used to prime the reaction. The primers can be composed of sequence within or 3' and 5' to the polynucleotides of the Sequence Listing. Alternatively, if the primers are 3' and 5' to these polynucleotides, they need not hybridize to them or the complements. After amplification of the target with a thermostable polymerase, the amplified target nucleic acids can be detected by methods known in the art, e.g., Southern blot. mRNA or cDNA can also be detected by traditional blotting techniques (e.g., Southern blot, Northern blot, etc.) described in Sambrook et al., "Molecular Cloning: A Laboratory Manual" (New York, Cold Spring Harbor Laboratory, 1989) (e.g., without PCR amplification). In general, mRNA or cDNA generated from mRNA using a polymerase enzyme can be purified and separated using gel electrophoresis, and transferred to a solid support, such as nitrocellulose. The solid support is exposed to a labeled probe, washed to remove any unhybridized probe, and duplexes containing the labeled probe are detected.

Methods using PCR amplification can be performed on the DNA from a single cell, although it is convenient to use at least about 10⁵ cells. The use of the polymerase chain reaction is described in Saiki et al. (1985) Science 239:487, and a review of current techniques may be found in Sambrook, et al. Molecular Cloning: A Laboratory Manual, CSH Press 1989, pp.14.2-14.33. A detectable label may be included in the amplification reaction. Suitable detectable labels include fluorochromes,(e.g. fluorescein isothiocyanate (FITC), rhodamine, Texas Red, phycoerythrin, allophycocyanin, 6-carboxyfluorescein (6-FAM), 2',7'-dimethoxy-4',5'-dichloro-6-carboxyfluorescein, 6-carboxy-X-rhodamine (ROX), 6-carboxy-2',4',7',4,7-hexachlorofluorescein (HEX), 5-carboxyfluorescein (5-FAM) or N,N,N',N'-tetramethyl-6-carboxyrhodamine (TAMRA)), radioactive labels, (*e.g.* ³²P, ³⁵S, ³H, *etc.*), and the like. The label may be a two stage system, where the polynucleotides is conjugated to biotin, haptens, *etc.* having a high affinity binding partner, *e.g.* avidin, specific antibodies, *etc.,* where the binding partner is conjugated to a detectable label. The label may be conjugated to one or both of the primers. Alternatively, the pool of nucleotides used in the amplification is labeled, so as to incorporate the label into the amplification product.

### Arrays

Polynucleotide arrays provide a high throughput technique that can assay a large number of polynucleotides or polypeptides in a sample. This technology can be used as a tool to test for differential expression.

A variety of methods of producing arrays, as well as variations of these methods, are known in the art and contemplated for use in the invention. For example, arrays can be created by spotting polynucleotide probes onto a substrate *(e.g.,* glass, nitrocellulose, *etc.)* in a two-dimensional matrix or array having bound probes. The probes can be bound to the substrate by either covalent bonds or by non-specific interactions, such as hydrophobic interactions.

Samples of polynucleotides can be detectably labeled (*e.g.,* using radioactive or fluorescent labels) and then hybridized to the probes. Double stranded polynucleotides, comprising the labeled sample polynucleotides bound to probe polynucleotides, can be detected once the unbound portion of the sample is washed away. Alternatively, the polynucleotides of the test sample can be immobilized on the array, and the probes detectably labeled. Techniques for constructing arrays and methods of using these arrays are described in, for example, Schena et al. (1996) Proc Natl Acad Sci USA. 93(20):10614-9; Schena et al. (1995) Science 270(5235):467-70; Shalon et al. (1996) Genome Res. 6(7):639-45, USPN 5,807,522, EP 799 897; WO 97/29212; WO 97/27317; EP 785 280; WO 97/02357; USPN 5,593,839; USPN 5,578,832; EP 728 520; USPN 5,599,695; EP 721 016; USPN 5,556,752; WO 95/22058; and USPN 5,631,734.

Arrays can be used to, for example, examine differential expression of genes and can be used to determine gene function. For example, arrays can be used to detect differential expression of a gene corresponding to a polynucleotide described herein, where expression is compared between a test cell and control cell (*e.g*., cancer cells and normal cells). For example, high expression of a particular message in a cancer cell, which is not observed in a corresponding normal cell, can indicate a cancer specific gene product. Exemplary uses of arrays are further described in, for example, Pappalarado et al., Sem. Radiation Oncol. (1998) 8:217; and Ramsay Nature Biotechnol. (1998) 16:40. Furthermore, many variations on methods of detection using arrays are well within the skill in the art and within the scope of the present invention. For example, rather than immobilizing the probe to a solid support, the test sample can be immobilized on a solid support which is then contacted with the probe.

### Diagnosis Prognosis, Assessment of Therapy (Therametrics), and Management of Cancer

The polynucleotides described herein, as well as their gene products and corresponding genes and gene products, are of particular interest as genetic or biochemical markers (e.g., in blood or tissues) that will detect the earliest changes along the carcinogenesis pathway and/or to monitor the efficacy of various therapies and preventive interventions.

For example, the level of expression of certain polynucleotides can be indicative of a poorer prognosis, and therefore warrant more aggressive chemo- or radio-therapy for a patient or vice versa. The correlation of novel surrogate tumor specific features with response to treatment and outcome in patients can define prognostic indicators that allow the design of tailored therapy based on the molecular profile of the tumor. These therapies include antibody targeting, antagonists (*e.g*., small molecules), and gene therapy.

Determining expression of certain polynucleotides and comparison of a patients profile with known expression in normal tissue and variants of the disease allows a determination of the best possible treatment for a patient, both in terms of specificity of treatment and in terms of comfort level of the patient. Surrogate tumor markers, such as polynucleotide expression, can also be used to better classify, and thus diagnose and treat, different forms and disease states of cancer. Two classifications widely used in oncology that can benefit from identification of the expression levels of the genes corresponding to the polynucleotides described herein are staging of the cancerous disorder, and grading the nature of the cancerous tissue.

The polynucleotides that correspond to differentially expressed genes, as well as their encoded gene products, can be useful to monitor patients having or susceptible to cancer to detect potentially malignant events at a molecular level before they are detectable at a gross morphological level. In addition, the polynucleotides described herein, as well as the genes corresponding to such polynucleotides, can be useful as therametrics, *e.g*., to assess the effectiveness of therapy by using the polynucleotides or their encoded gene products, to assess, for example, tumor burden in the patient before, during, and after therapy.

Furthermore, a polynucleotide identified as corresponding to a gene that is differentially expressed in, and thus is important for, one type of cancer can also have implications for development or risk of development of other types of cancer, *e.g*., where a polynucleotide represents a gene differentially expressed across various cancer types. Thus, for example, expression of a polynucleotide corresponding to a gene that has clinical implications for metastatic colon cancer can also have clinical implications for breast cancer or ovarian cancer.

Staging. Staging is a process used by physicians to describe how advanced the cancerous state is in a patient. Staging assists the physician in determining a prognosis, planning treatment and evaluating the results of such treatment. Staging systems vary with the types of cancer, but generally involve the following "TNM" system: the type of tumor, indicated by T; whether the cancer has metastasized to nearby lymph nodes, indicated by N; and whether the cancer has metastasized to more distant parts of the body, indicated by M. Generally, if a cancer is only detectable in the area of the primary lesion without having spread to any lymph nodes it is called Stage I. If it has spread only to the closest lymph nodes, it is called Stage II. In Stage II, the cancer has generally spread to the lymph nodes in near proximity to the site of the primary lesion. Cancers that have spread to a distant part of the body, such as the liver, bone, brain or other site, are Stage IV, the most advanced stage.

The polynucleotides and corresponding genes and gene products described herein can facilitate fine-tuning of the staging process by identifying markers for the aggressiveness of a cancer, *e.g*. the metastatic potential, as well as the presence in different areas of the body. Thus, a Stage II cancer with a polynucleotide signifying a high metastatic potential cancer can be used to change a borderline Stage II tumor to a Stage III tumor, justifying more aggressive therapy. Conversely, the presence of a polynucleotide signifying a lower metastatic potential allows more conservative staging of a tumor.

Grading of cancers. Grade is a term used to describe how closely a tumor resembles normal tissue of its same type. The microscopic appearance of a tumor is used to identifyy tumor grade based on parameters such as cell morphology, cellular organization, and other markers of differentiation. As a general rule, the grade of a tumor corresponds to its rate of growth or aggressiveness, with undifferentiated or high-grade tumors generally being more aggressive than well differentiated or low-grade tumors. The following guidelines are generally used for grading tumors: 1) GX Grade cannot be assessed; 2) G1 Well differentiated; G2 Moderately well differentiated; 3) G3 Poorly differentiated; 4) G4 Undifferentiated. The polynucleotides of the Sequence Listing, and their corresponding genes and gene products, can be especially valuable in determining the grade of the tumor, as they not only can aid in determining the differentiation status of the cells of a tumor, they can also identify factors other than differentiation that are valuable in determining the aggressiveness of a tumor, such as metastatic potential.

Detection of colon cancer. The polynucleotides corresponding to genes that exhibit the appropriate expression pattern can be used to detect colon cancer in a subject. Colorectal cancer is one of the most common neoplasms in humans and perhaps the most frequent form of hereditary neoplasia. Prevention and early detection are key factors in controlling and curing colorectal cancer. Colorectal cancer begins as polyps, which are small, benign growths of cells that form on the inner lining of the colon. Over a period of several years, some of these polyps accumulate additional mutations and become cancerous. Multiple familial colorectal cancer disorders have been identified, which are summarized as follows: 1) Familial adenomatous polyposis (FAP); 2) Gardner's syndrome; 3) Hereditary nonpolyposis colon cancer (HNPCC); and 4) Familial colorectal cancer in Ashkenazi Jews.

The expression of appropriate polynucleotides can be used in the diagnosis, prognosis and management of colorectal cancer. Detection of colon cancer can be determined using expression levels of any of these sequences alone or in combination with the levels of expression. Determination of the aggressive nature and/or the metastatic potential of a colon cancer can be determined by comparing levels of one or more gene products of the genes corresponding to the polynucleotides described herein, and comparing total levels of another sequence known to vary in cancerous tissue, *e.g*., expression of p53, DCC, ras, FAP (see, e.g., Fearon ER, et al., Cell (1990) 61 (5):759*;* Hamilton SR et al., Cancer (1993) 72:957; Bodmer W, et al., Nat Genet. (1994) 4(3):217; Fearon ER, Ann N Y Acad Sci. (1995) 768:101)*.*

For example, development of colon cancer can be detected by examining the level of expression of a gene corresponding to a polynucleotides described herein to the levels of oncogenes (e.g. ras) or tumor suppressor genes (*e.g*. FAP or p53). Thus expression of specific marker polynucleotides can be used to discriminate between normal and cancerous colon tissue, to discriminate between colon cancers with different cells of origin, to discriminate between colon cancers with different potential metastatic rates, etc. For a review of markers of cancer, see, *e.g*., Hanahan et al. (2000) Cell 100:57-70.

### Treatment of colon cancer

The invention further provides methods for reducing growth of colon cancer cells. The methods provide for decreasing the expression of a gene that is differentially expressed in a colon cancer cell or decreasing the level of and/or decreasing an activity of a colon cancer-associated polypeptide. In general, the methods comprise contacting a colon cancer cell with a substance that modulates (1) expression of a gene that is differentially expressed in colon cancer; or (2) a level of and/or an activity of a colon cancer-associated polypeptide. "Reducing growth of colon cancer cells" includes, but is not limited to, reducing proliferation of colon cancer cells, and reducing the incidence of a non-cancerous colon cell becoming a cancerous colon cell. Whether a reduction in colon cancer cell growth has been achieved can be readily determined using any known assay, including, but not limited to, [³H]-thymidine incorporation; counting cell number over a period of time; detecting and/or measuring a marker associated with colon cancer (e.g., CEA, CA 19-9, and LASA).

The present invention provides methods for treating colon cancer, generally comprising administering to an individual in need thereof a substance that reduces colon cancer cell growth, in an amount sufficient to reduce colon cancer cell growth and treat the colon cancer. Whether a substance, or a specific amount of the substance, is effective in treating colon cancer can be assessed using any of a variety of known diagnostic assays for colon cancer, including, but not limited to, sigmoidoscopy, proctoscopy, rectal examination, colonoscopy with biopsy, contrast radiographic studies, CAT scans, angiography, and detection of a tumor marker associated with colon cancer in the blood of the individual. The substance can be administered systemically or locally. Thus, in some embodiments, the substance is administered locally, and colon cancer growth is decreased at the site of administration. Local administration may be useful in treating, e.g., a solid tumor.

A substance that reduces colon cancer cell growth can be targeted to a colon cancer cell. Thus, in some embodiments, the invention provides a method of delivering a drug to a colon cancer cell, comprising administering a drug-antibody complex to a subject, wherein the antibody is specific for a colon cancer-associated polypeptide, and the drug is one that reduces colon cancer cell growth, a variety of which are known in the art. Targeting can be accomplished by coupling (e.g., linking, directly or via a linker molecule, either covalently or non-covalently, so as to form a drug-antibody complex) a drug to an antibody specific for a colon cancer-associated polypeptide. Methods of coupling a drug to an antibody are well known in the art and need not be elaborated upon herein.

### Identification of Therapeutic Targets and Anti-Cancer Therapeutic Agents

The present invention also encompasses methods for identification of agents having the ability to modulate activity of a differentially expressed gene product, as well as methods for identifying a differentially expressed gene product as a therapeutic target for treatment of cancer, especially colon cancer.

### Candidate agents

Identification of compounds that modulate activity of a differentially expressed gene product can be accomplished using any of a variety of drug screening techniques. Such agents are candidates for development of cancer therapies. Of particular interest are screening assays for agents that has tolerable toxicity for normal, non-cancerous human cells. The screening assays of the invention are generally based upon the ability of the agent to modulate an activity of a differentially expressed gene product and/or to inhibit or suppress phenomenon associated with cancer (*e.g*., cell proliferation, colony formation, cell cycle arrest, metastasis, and the like).

The term "agent" as used herein describes any molecule, *e.g*. protein or pharmaceutical, with the capability of modulating a biological activity of a gene product of a differentially expressed gene. Generally a plurality of assay mixtures are run in parallel with different agent concentrations to obtain a differential response to the various concentrations. Typically, one of these concentrations serves as a negative control, *i.e.* at zero concentration or below the level of detection.

Candidate agents encompass numerous chemical classes, though typically they are organic molecules, preferably small organic compounds having a molecular weight of more than 50 and less than about 2,500 daltons. Candidate agents comprise functional groups necessary for structural interaction with proteins, particularly hydrogen bonding, and typically include at least an amine, carbonyl, hydroxyl or carboxyl group, preferably at least two of the functional chemical groups. The candidate agents often comprise cyclical carbon or heterocyclic structures and/or aromatic or polyaromatic structures substituted with one or more of the above functional groups. Candidate agents are also found among biomolecules including, but not limited to: peptides, saccharides, fatty acids, steroids, purines, pyrimidines, derivatives, structural analogs or combinations thereof.

Candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts (including extracts from human tissue to identify endogenous factors affecting differentially expressed gene products) are available or readily produced. Additionally, natural or synthetically produced libraries and compounds are readily modified through conventional chemical, physical and biochemical means, and may be used to produce combinatorial libraries. Known pharmacological agents may be subjected to directed or random chemical modifications, such as acylation, alkylation, esterification, amidification, *etc*. to produce structural analogs.

Exemplary candidate agents of particular interest include, but are not limited to, antisense polynucleotides, and antibodies, soluble receptors, and the like. Antibodies and soluble receptors are of particular interest as candidate agents where the target differentially expressed gene product is secreted or accessible at the cell-surface (*e.g*., receptors and other molecule stably-associated with the outer cell membrane).

### Screening of candidate agents

Screening assays can be based upon any of a variety of techniques readily available and known to one of ordinary skill in the art. In general, the screening assays involve contacting a cancerous cell (preferably a cancerous colon cell) with a candidate agent, and assessing the effect upon biological activity of a differentially expressed gene product. The effect upon a biological activity can be detected by, for example, detection of expression of a gene product of a differentially expressed gene (*e.g*., a decrease in mRNA or polypeptide levels, would in turn cause a decrease in biological activity of the gene product). Alternatively or in addition, the effect of the candidate agent can be assessed by examining the effect of the candidate agent in a functional assay. For example, where the differentially expressed gene product is an enzyme, then the effect upon biological activity can be assessed by detecting a level of enzymatic activity associated with the differentially expressed gene product. The functional assay will be selected according to the differentially expressed gene product. In general, where the differentially expressed gene is increased in expression in a cancerous cell, agents of interest are those that decrease activity of the differentially expressed gene product.

Assays described infra can be readily adapted in the screening assay embodiments of the invention. Exemplary assays useful in screening candidate agents include, but are not limited to, hybridization-based assays (*e.g*., use of nucleic acid probes or primers to assess expression levels), antibody-based assays (*e.g*., to assess levels of polypeptide gene products), binding assays (*e.g*., to detect interaction of a candidate agent with a differentially expressed polypeptide, which assays may be competitive assays where a natural or synthetic ligand for the polypeptide is available), and the like. Additional exemplary assays include, but are not necessarily limited to, cell proliferation assays, antisense knockout assays, assays to detect inhibition of cell cycle, assays of induction of cell death/apoptosis, and the like. Generally such assays are conducted *in vitro,* but many assays can be adapted for *in vivo* analyses, *e.g*., in an animal model of the cancer.

### Identification of therapeutic targets

In another embodiment, the invention contemplates identification of differentially expressed genes and gene products as therapeutic targets. In some respects, this is the converse of the assays described above for identification of agents having activity in modulating (*e.g*., decreasing or increasing) activity of a differentially expressed gene product

In this embodiment, therapeutic targets are identified by examining the effect(s) of an agent that can be demonstrated or has been demonstrated to modulate a cancerous phenotype (*e.g.*, inhibit or suppress or prevent development of a cancerous phenotype). Such agents are generally referred to herein as an "anti-cancer agent", which agents encompass chemotherapeutic agents. For example, the agent can be an antisense oligonucleotide that is specific for a selected gene transcript For example, the antisense oligonucleotide may have a sequence corresponding to a sequence of a differentially expressed gene described herein, *e.g*., a sequence of one of SEQ ID NO: 1.

Assays for identification of therapeutic targets can be conducted in a variety of ways using methods that are well known to one of ordinary skill in the art. For example, a test cancerous cell that expresses or overexpresses a differentially expressed gene is contacted with an anti-cancer agent, the effect upon a cancerous phenotype and a biological activity of the candidate gene product assessed. The biological activity of the candidate gene product can be assayed be examining, for example, modulation of expression of a gene encoding the candidate gene product (*e.g.,* as dectected by, for example, an increase or decrease in transcript levels or polypeptide levels), or modulation of an enzymatic or other activity of the gene product. The cancerous phenotype can be, for example, cellular proliferation, loss of contact inhibition of growth (*e.g*., colony formation), tumor growth (in *vitro* or in vivo), and the like. Alternatively or in addition, the effect of modulation of a biological activity of the candidate target gene upon cell death/apoptosis or cell cycle regulation can be assessed.

Inhibition or suppression of a cancerous phenotype, or an increase in cell/death apoptosis as a result of modulation of biological activity of a candidate gene product indicates that the candidate gene product is a suitable target for cancer therapy. Assays described infra can be readily adapted in for assays for identification of therapeutic targets. Generally such assays are conducted *in vitro,* but many assays can be adapted for *in vivo* analyses, *e.g*., in an appropriate, art-accepted animal model of the cancer.

### Identification of Peptide Analogs and Antagonists

Polypeptides encoded by differentially expressed genes identified herein can be used to screen peptide libraries to identify binding partners, such as receptors, from among the encoded polypeptides. Peptide libraries can be synthesized according to methods known in the art (see, e.g., USPN 5,010,175 , and WO 91/17823).

Agonists or antagonists of the polypeptides if the invention can be screened using any available method known in the art, such as signal transduction, antibody binding, receptor binding, mitogenic assays, chemotaxis assays, etc. The assay conditions ideally should resemble the conditions under which the native activity is exhibited *in vivo,* that is, under physiologic pH, temperature, and ionic strength. Suitable agonists or antagonists will exhibit strong inhibition or enhancement of the native activity at concentrations that do not cause toxic side effects in the subject. Agonists or antagonists that compete for binding to the native polypeptide can require concentrations equal to or greater than the native concentration, while inhibitors capable of binding irreversibly to the polypeptide can be added in concentrations on the order of the native concentration.

Such screening and experimentation can lead to identification of a polypeptide binding partner, such as a receptor, encoded by a gene or a cDNA corresponding to a polynucleotide described herein, and at least one peptide agonist or antagonist of the binding partner. Such agonists and antagonists can be used to modulate, enhance, or inhibit receptor function in cells to which the receptor is native, or in cells that possess the receptor as a result of genetic engineering. Further, if the receptor shares biologically important characteristics with a known receptor, information about agonist/antagonist binding can facilitate development of improved agonists/antagonists of the known receptor.

### Pharmaceutical Compositions and Therapeutic Uses

Pharmaceutical compositions of the invention can comprise polypeptides, antibodies, or polynucleotides (including antisense nucleotides and ribozymes) of the claimed invention in a therapeutically effective amount. The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation is determined by routine experimentation and is within the judgment of the clinician. For purposes of the present invention, an effective dose will generally be from about 0.01 mg/ kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which can be administered without undue toxicity. Suitable carriers can be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art. Pharmaceutically acceptable carriers in therapeutic compositions can include liquids such as water, saline, glycerol and ethanol. Auxiliary substances, such as wetting or emulsifying agents, pH buffering substances, and the like, can also be present in such vehicles.

Typically, the therapeutic compositions are prepared as injectables, either as liquid solutions or suspensions; solid forms suitable for solution in, or suspension in, liquid vehicles prior to injection can also be prepared. Liposomes are included within the definition of a pharmaceutically acceptable carrier. Pharmaceutically acceptable salts can also be present in the pharmaceutical composition, e.g., mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Delivery Methods. Once formulated, the compositions of the invention can be (1) administered directly to the subject (*e.g*., as polynucleotide or polypeptides); or (2) delivered *ex vivo,* to cells derived from the subject *(e.g.,* as in *ex vivo* gene therapy). Direct delivery of the compositions will generally be accomplished by parenteral injection, *e.g*., subcutaneously, intraperitoneally, intravenously or intramuscularly, intratumoral or to the interstitial space of a tissue. Other modes of administration include oral and pulmonary administration, suppositories, and transdermal applications, needles, and gene guns or hyposprays. Dosage treatment can be a single dose schedule or a multiple dose schedule.

Methods for the *ex vivo* delivery and reimplantation of transformed cells into a subject are known in the art and described in *e.g*., International Publication No. WO 93/14778. Examples of cells useful in *ex vivo* applications include, for example, stem cells, particularly hematopoetic, lymph cells, macrophages, dendritic cells, or tumor cells. Generally, delivery of nucleic acids for both *ex vivo* and *in vitro* applications can be accomplished by, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Once a gene corresponding to a polynucleotide of the invention has been found to correlate with a proliferative disorder, such as neoplasia, dysplasia, and hyperplasia, the disorder can be amenable to treatment by administration of a therapeutic agent based on the provided polynucleotide, corresponding polypeptide or other corresponding molecule (*e.g*., antisense, ribozyme, *etc.).*

The dose and the means of administration of the inventive pharmaceutical compositions are determined based on the specific qualities of the therapeutic composition, the condition, age, and weight of the patient, the progression of the disease, and other relevant factors. For example, administration of polynucleotide therapeutic compositions agents of the invention includes local or systemic administration, including injection, oral administration, particle gun or catheterized administration, and topical administration. Preferably, the therapeutic polynucleotide composition contains an expression construct comprising a promoter operably linked to a polynucleotide of at least 12, 22, 25, 30, or 35 contiguous nt of the polynucleotide disclosed herein.

Various methods can be used to administer the therapeutic composition directly to a specific site in the body. For example, a small metastatic lesion is located and the therapeutic composition injected several times in several different locations within the body of tumor. Alternatively, arteries which serve a tumor are identified, and the therapeutic composition injected into such an artery, in order to deliver the composition directly into the tumor. A tumor that has a necrotic center is aspirated and the composition injected directly into the now empty center of the tumor. The antisense composition is directly administered to the surface of the tumor, for example, by topical application of the composition. X-ray imaging is used to assist in certain of the above delivery methods.

Receptor-mediated targeted delivery of therapeutic compositions containing an antisense polynucleotide, subgenomic polynucleotides, or antibodies to specific tissues can also be used. Receptor-mediated DNA delivery techniques are described in, for example, Findeis et al., Trends Biotechnol. (1993) 11:202; Chiou et al., Gene Therapeutics: Methods And Applications Of Direct Gene Transfer (J.A. Wolff, ed.) (1994); Wu et al., J. Biol. Chem. (1988) 263:621; Wu et al., J. Biol. Chem. (1994) 269:542; Zenke et al., Proc. Natl. Acad. Sci. (USA) (1990) 87:3655*;* Wu et al., J. Biol. Chem. (1991) 266:338. Therapeutic compositions containing a polynucleotide are administered in a range of about 100 ng to about 200 mg of DNA for local administration in a gene therapy protocol. Concentration ranges of about 500 ng to about 50 mg, about 1 µg to about 2 mg, about 5 µg to about 500 µg, and about 20 µg to about 100 µg of DNA can also be used during a gene therapy protocol. Factors such as method of action (*e.g*., for enhancing or inhibiting levels of the encoded gene product) and efficacy of transformation and expression are considerations which will affect the dosage required for ultimate efficacy of the antisense subgenomic polynucleotides. Where greater expression is desired over a larger area of tissue, larger amounts of antisense subgenomic polynucleotides or the same amounts readministered in a successive protocol of administrations, or several administrations to different adjacent or close tissue portions of, for example, a tumor site, may be required to effect a positive therapeutic outcome. In all cases, routine experimentation in clinical trials will determine specific ranges for optimal therapeutic effect. For polynucleotide related genes encoding polypeptides or proteins with anti-inflammatory activity, suitable use, doses, and administration are described in USPN 5,654,173.

The therapeutic polynucleotides and polypeptides of the present invention can be delivered using gene delivery vehicles. The gene delivery vehicle can be of viral or non-viral origin (see generally, Jolly, Cancer Gene Therapy (1994) 1:51; Kimura, Human Gene Therapy (1994) 5:845; Connelly, Human Gene Therapy (1995) 1:185; and Kaplitt, Nature Genetics (1994) 6:148). Expression of such coding sequences can be induced using endogenous mammalian or heterologous promoters. Expression of the coding sequence can be either constitutive or regulated.

Viral-based vectors for delivery of a desired polynucleotide and expression in a desired cell are well known in the art. Exemplary viral-based vehicles include, but are not limited to, recombinant retroviruses (see, *e.g*., WO 90/07936; WO 94/03622; WO 93/25698; WO 93/25234; USPN 5, 219,740; WO 93/11230; WO 93/10218; USPN 4,777,127; GB Patent No. 2,200,651; EP 0 345 242; and WO 91/02805), alphavirus-based vectors (*e.g*., Sindbis virus vectors, Semliki forest virus (ATCC VR-67; ATCC VR-1247), Ross River virus (ATCC VR-373; ATCC VR-1246) and Venezuelan equine encephalitis virus (ATCC VR-923; ATCC VR-1250; ATCC VR 1249; ATCC VR-532), and adeno-associated virus (AAV) vectors (see, e.g., WO 94/12649, WO 93/03769; WO 93/19191; WO 94/28938; WO 95/11984 and WO 95/00655). Administration of DNA linked to killed adenovirus as described in Curiel, Hum. Gene Ther. (1992) 3:147 can also be employed.

Non-viral delivery vehicles and methods can also be employed, including, but not limited to, polycationic condensed DNA linked or unlinked to killed adenovirus alone (see, *e.g*., Curiel, Hum. Gene Ther. (1992) 3:147); ligand-linked DNA (see, *e.g.,* Wu, J Biol. Chem. (1989) 264:16985); eukaryotic cell delivery vehicles cells (see, *e.g*., USPN 5,814,482; WO 95/07994; WO 96/17072; WO 95/30763; and WO 97/42338) and nucleic charge neutralization or fusion with cell membranes. Naked DNA can also be employed. Exemplary naked DNA introduction methods are described in WO 90/11092 and USPN 5,580,859. Liposomes that can act as gene delivery vehicles are described in USPN 5,422,120; WO 95/13796; WO 94/23697; WO 91/14445; and EP 0524968. Additional approaches are described in Philip, Mol. Cell Biol. (1994) 14:2411, and in Woffendin, Proc. Natl. Acad. Sci. (1994) 91:1581

Further non-viral delivery suitable for use includes mechanical delivery systems such as the approach described in Woffendin et al., Proc. Natl. Acad. Sci. USA (1994) 91 (24):11581*.* Moreover, the coding sequence and the product of expression of such can be delivered through deposition of photopolymerized hydrogel materials or use of ionizing radiation (see, *e.g*., USPN 5,206,152 and WO 92/11033). Other conventional methods for gene delivery that can be used for delivery of the coding sequence include, for example, use of hand-held gene transfer particle gun (see, *e.g*., USPN 5,149,655); use of ionizing radiation for activating transferred gene (see, *e.g.*, USPN 5,206,152 and WO 92/11033).

The present invention will now be illustrated by reference to the following examples which set forth particularly advantageous embodiments. However, it should be noted that these embodiments are illustrative and are not to be construed as restricting the invention in any way.

### EXAMPLES

The following examples are offered primarily for purposes of illustration. It will be readily apparent to those skilled in the art that the formulations, dosages, methods of administration, and other parameters of this invention may be further modified or substituted in various ways without departing from the spirit and scope of the invention.

### Example 1: Source of Biological Materials and Overview of Polynucleotides Expressed by the Biological Materials

In order to identify genes that are differentially expressed in colon cancer, cDNA libraries were prepared from several different cell lines and tissue sources. Table 1 provides a summary of these libraries, including the shortened library name (used hereafter), the mRNA source used to prepared the cDNA library, the "nickname" of the library that is used in the tables below (in quotes), and the approximate number of clones in the library. cDNA libraries were prepared according to methods well known in the art, and the sequences of the cDNA inserts were determined using well known methods.

| **Table 1**. Description of cDNA Libraries | | |
|---|---|---|
| Library | Description | Number of Clones |
| 1 | Human Colon Cell Line Km12 L4: High Metastatic Potential (derived from Km12C) | 308731 |
| 2 | Human Colon Cell Line Km12C: Low Metastatic Potential | 284771 |
| 3 | Human Breast Cancer Cell Line MDA-MB-231: High Metastatic Potential; micromets in lung | 326937 |
| 4 | Human Breast Cancer Cell Line MCF7: Non-Metastatic | 318979 |
| 8 | Human Lung Cancer Cell Line MV-522: High Metastatic Potential | 223620 |
| 9 | Human Lung Cancer Cell Line UCP-3: Low Metastatic Potential | 312503 |
| 12 | Human microvascular endothelial cells (HMEC) - UNTREATED (PCR (OligodT) cDNA library) | 41938 |
| 13 | Human microvascular endothelial cells (HMEC) - bFGF TREATED (PCR (OligodT) cDNA library) | 42100 |
| 14 | Human microvascular endothelial cells (HMEC) - VEGF TREATED (PCR (OligodT) cDNA library) | 42825 |
| 15 | Normal Colon - UC#2 Patient (MICRODISSECTED PCR (OligodT) cDNA library) | 282718 |
| 16 | Colon Tumor - UC#2 Patient (MICRODISSECTED PCR (OligodT) cDNA library) | 298829 |
| 17 | Liver Metastasis from Colon Tumor of UC#2 Patient (MICRODISSECTED PCR (OligodT) cDNA library) | 303462 |
| 18 | Normal Colon - UC#3 Patient (MICRODISSECTED PCR (OligodT) cDNA library) | 36216 |
| 19 | Colon Tumor - UC#3 Patient (MICRODISSECTED PCR (OligodT) cDNA library) | 41388 |
| 20 | Liver Metastasis from Colon Tumor of UC#3 Patient (MICRODISSECTED PCR (OligodT) cDNA library) | 30956 |
| 21 | GRRpz Cells derived from normal prostate epithelium | 164801 |
| 22 | WOca Cells derived from Gleason Grade 4 prostate cancer epithelium | 162088 |
| 23 | Normal Lung Epithelium of Patient #1006 (MICRODISSECTED PCR (OligodT) cDNA library) | 306198 |
| 24 | Primary tumor, Large Cell Carcinoma of Patient #1006 (MICRODISSECTED PCR (OligodT) cDNA library) | 309349 |
| 25 | Normal Prostate Epithelium from Patient IF97-26811 | 279437 |
| 26 | Prostate Cancer Epithelium Gleason 3+3 Patient IF97-26811 | 269366 |

The KM12L4 cell line is derived from the KM12C cell line (Morikawa, et al, Cancer Research (1988) 48:6863). The KM12C cell line, which is poorly metastatic (low metastatic) was established in culture from a Dukes' stage B₂ surgical specimen (Morikawa et al. Cancer Res. (1988) 48:6863). The KML4-A is a highly metastatic subline derived from KM12C (Yeatman et al. Nucl. Acids. Res. (1995) 23:4007; Bao-Ling et al. Proc. Annu. Meet. Am. Assoc. Cancer. Res. (1995) 21:3269). The KM12C and KM12C-derived cell lines (*e.g*., KM12L4, KM12L4-A, *etc.)* are well-recognized in the art as a model cell line for the study of colon cancer (see, *e.g*., Moriakawa *et al., supra;* Radinsky et al. Clin. Cancer Res. (1995) 1:19; Yeatman *et al.,* (1995) *supra;* Yeatman et al. Clin. Exp. Metastasis (1996) 14:246).

The MDA-MB-231 cell line was originally isolated from pleural effusions (Cailleau, J. Natl. Cancer. Inst. (1974) 53:661), is of high metastatic potential, and forms poorly differentiated adenocarcinoma grade II in nude mice consistent with breast carcinoma. The MCF7 cell line was derived from a pleural effusion of a breast adenocarcinoma and is non-metastatic. These cell lines are well-recognized in the art as models for the study of human breast and lung cancer (see, *e.g*., Chandrasekaran et al., Cancer Res. (1979) 39:870; Gastpar et al., J Med Chem (1998) 41:4965; Ranson et al., Br J Cancer (1998) 77:1586; Kuang et al., Nucleic Acids Res (1998) 26:1116. The samples of libraries 15-20 are derived from two different patients (UC#2 and UC#3). The GRRpz and WOca cell lines were provided by Dr. Donna M. Peehl, Department of Medicine, Stanford University School of Medicine. GRRpz was derived from normal prostate epithelium. The WOca cell line is a Gleason Grade 4 cell line.

Each of the libraries is composed of a collection of cDNA clones that in turn are representative of the mRNAs expressed in the indicated mRNA source. In order to facilitate the analysis of the millions of sequences in each library, the sequences were assigned to clusters. The concept of "cluster of clones" is derived from a sorting/grouping of cDNA clones based on their hybridization pattern to a panel of roughly 300 7bp oligonucleotide probes (see Drmanac et al., Genomics (1996) 37(1):29). Random cDNA clones from a tissue library are hybridized at moderate stringency to 300 7bp oligonucleotides. Each oligonucleotide has some measure of specific hybridization to that specific clone. The combination of 300 of these measures of hybridization for 300 probes equals the "hybridization signature" for a specific clone. Clones with similar sequence will have similar hybridization signatures. By developing a sorting/grouping algorithm to analyze these signatures, groups of clones in a library can be identified and brought together computationally. These groups of clones are termed "clusters".

Depending on the stringency of the selection in the algorithm (similar to the stringency of hybridization in a classic library cDNA screening protocol), the "purity" of each cluster can be controlled. For example, artifacts of clustering may occur in computational clustering just as artifacts can occur in "wet-lab" screening of a cDNA library with 400 bp cDNA fragments, at even the highest stringency. The stringency used in the implementation of cluster herein provides groups of clones that are in general from the same cDNA or closely related cDNAs. Closely related clones can be a result of different length clones of the same cDNA, closely related clones from highly related gene families, or splice variants of the same cDNA.

Differential expression for a selected cluster was assessed by first determining the number of cDNA clones corresponding to the selected cluster in the first library (Clones in 1^{st}), and the determining the number of cDNA clones corresponding to the selected cluster in the second library (Clones in 2^{nd}). Differential expression of the selected cluster in the first library relative to the second library is expressed as a "ratio" of percent expression between the two libraries. In general, the "ratio" is calculated by: 1) calculating the percent expression of the selected cluster in the first library by dividing the number of clones corresponding to a selected cluster in the first library by the total number of clones analyzed from the first library; 2) calculating the percent expression of the selected cluster in the second library by dividing the number of clones corresponding to a selected cluster in a second library by the total number of clones analyzed from the second library; 3) dividing the calculated percent expression from the first library by the calculated percent expression from the second library. If the "number of clones" corresponding to a selected cluster in a library is zero, the value is set at 1 to aid in calculation. The formula used in calculating the ratio takes into account the "depth" of each of the libraries being compared, i.e., the total number of clones analyzed in each library.

As a result of this library comparison, 17 polynucleotides, listed as SEQ ID NOS:1, 3, 5, 7, 9, 11-13, 15, 16, 18, 20, 22, 24, 26, 27 and 29 in the accompanying Sequence Listing and summarized in Table 2, were identified as corresponding to genes differentially expressed in colon cancer patient tissues. Table 2 provides: 1) the sequence identification number ("SEQ ID NO of polynucleotide") assigned to each sequence for use in the present specification; 2) the cluster identification number ("CLUSTER"); 3) the Candidation Idnetification number; 4) ththe CHIR number (which serves as tha cross-reference to antisense oligos discussed below), with, for examplek CHIR7 having corresponding oligos CHIR7-2AS (antibsense) and CHIR7-RC (reverse control); 5) the sequence name ("SEQ NAME") used as an internal identifier of the sequence; 6) the name assigned to the clone from which the sequence was isolated ("CLONE ID"); 7) the first nucleotide of the start and stop codons of identified open reading frames ("ORF start" and "ORF stop"); and 8) the sequence identification number ("SEQ ID NO of encoded polypeptide") assigned to the encoded polypeptide, where appropriate. Because the provided polynucleotides represent partial mRNA transcripts, two or more polynucleotides of the invention may represent different regions of the same mRNA transcript and the same gene. Thus, if two or more sequences are identified as belonging to the same clone, then either sequence can be used to obtain the full-length mRNA or gene.

| **Table 2.** Polynucleotide sequence identificaton and characterization | | | | | | | |
|---|---|---|---|---|---|---|---|
| **SEQ ID NO** | **CLUSTER** | **Candidate ID** | **CHIR** | **SEQ** NAME | **ORF** | | **SEQ ID NO** of encoded polypeptide |
| | | | | | **start** | **stop** | |
| 1 | 719 | 196 | CHIR-7 | SK1 | 21 | 396 | 2 |
| 3 | 9083 | 181 | CHIR-8 | SK2 | 219 | 693 | 4 |
| 5 | 115762 | 188 | CHIR-16 | SK5 | 5 | 1760 | 6 |
| 7 | 1665 | 195 | CHIR-9 | 1665 long | 78 | 642 | 8 |
| 9 | 1665 | 195 | CHIR-9 | 1665 short | 79 | 232 | 10 |
| 11 | 2334 | | | SK8 partial | | | |
| 12 | 2334 | | | SK8 full length | | | |
| 13 | 3376 | 118 | CHIR-11 | SK19 | 79 | 376 | 14 |
| 15 | 376130 | | | Junc2 | 181, 363, 731 | 361, 542, 911 | |
| 16 | 402380 | 202 | CHIR-33 | XD4 | 16 | 538 | 17 |
| 18 | 726682 | 198 | CHIR-43 | XD1 | 2 | 551 | 19 |
| 20 | 552930 | 174 | CHIR-42 | XD7 | 240 | 585 | 21 |
| 22 | 454001 | 161 | CHIR-29 | XD10 | 53 | 1700 | 23 |
| 24 | 378805 | 163 | CHIR-31 | XD11 | 10 | 400 | 25 |
| 26 | 374641 | 160 | CHIR-32 | 374641 long (Junc4) | 33,420 | 183, 615 | |
| 27 | 374641 | 160 | CHIR-32 | 374641 short (XD6) | 324 | 519 | 28 |
| 29 | 374641 | 160 | CHIR-32 | 374641 electronic | 40,388 | 190, 583 | |

Table 3 summarizes polynucleotides that correspond to genes differentially expressed in colon tissue from a single patient.

| **Table 3** | | | | | | | |
|---|---|---|---|---|---|---|---|
| **SEQ ID NO** | **CLUSTER** | **Normal (Lib15) Clones** | **Tumor (Lib16) Clones** | **High Met (Lib17) Clones** | **Tumor/ Normal (Lib16/Lib15)** | **High Met/ Normal (Lib17/Lib15)** | **High Met/ Tumor (Lib17/Lib16)** |
| 1 | 719 | 0 | 20 | 27 | 20 | 27 | 1 |
| 3 | 9083 | 0 | 10 | 14 | 10 | 14 | 1 |
| 5 | 115762 | 0 | 6 | 7 | 6 | 7 | 1 |
| 7 | 1665 | 4 | 14 | 20 | 3.5 | 5 | 1 |
| 12 | 2334 | 0 | 6 | 1 | 6 | 1 | 0 |
| 13 | 3376 | 3 | 20 | 19 | 7 | 6 | 1 |
| 15 | 376130 | 0 | 9 | 15 | 9 | 15 | 2 |
| 16 | 402380 | 0 | 15 | 2 | 15 | 2 | 0 |
| 18 | 726682 | 0 | 52 | 0 | 52 | 0 | 0 |
| 20 | 552930 | 1 | 14 | 2 | 14 | 2 | 0 |
| 22 | 454001 | 0 | 8 | 13 | 8 | 13 | 2 |
| 24 | 378805 | 1 | 12 | 12 | 12 | 12 | 1 |
| 26 | 374641 | 9 | 47 | 129 | 5 | 14 | 3 |

### Example 2: Analysis and Characterization of Polynucleotides of the Invention

Several of the provided polynucleotides contain one or more putative open reading frames (ORFs) encoding a gene product. The start and stop sites for these ORFs are listed in Table 2.

SEQ ID NO:15 contains three ORFs. The first ORF extends from nucleotide 181 to nucleotide 361. The second ORF extends from nucleotide 363 to nucleotide 542. The third ORF extends from nucleotide 731 to nucleotide 911.

SEQ ID NO:26 contains a 39-nucleotide insertion sequence (from nucleotide 269 to nucleotide 307) and two ORFs. The first ORF extends from nucleotide 33 to nucleotide 183. The second ORF extends from nucleotide 420 to nucleotide 615.

SEQ ID NO:29 is an electronic sequence according to the 5'-RACE result and contains two ORFs. The first ORF extends from nucleotide 40 to nucleotide 190. The second ORF extends from nucleotide 388 to nucleotide 583.

### Example 3: Members of Protein Families

Translations of the provided polynucleotides were aligned with amino acid profiles that define either protein families or common motifs. Several of the polynucleotides of the invention were found to encode polypeptides having characteristics of a polypeptide belonging to a known protein family (and thus represent new members of these protein families) and/or comprising a known functional domain. Similarity between a query sequence and a protein family or motif was determined by (a) comparing the query sequence against the profile and/or (b) aligning the query sequence with the members of the family or motif.

Each of the profile hits is described in more detail below. Table 4 provides the corresponding SEQ ID NO of the provided polynucleotides that encode gene products with similarity or identity to the profile sequences. Similarity (strong or weak) is also noted in Table 4. The acronyms for the profiles (provided in parentheses) are those used to identify the profile in the Pfam and Prosite databases. The Pfam database can be accessed through any of the following URLS: http://pfam.wustl.edu/index.html; http://www.sanger.ac.uk/ Software/Pfam/; and http://www.cgr.ki.se/Pfam/. The Prosite database can be accessed athttp://www.expasy.ch/prosite/.

**Table 4. Profile hits.**

| **SEQ ID NO** | **CLUSTER** | **Profile** | **Description** | **Similarity** |
|---|---|---|---|---|
| 1 | 719 | | Glycosyl hydrolase | weak |
| 3 | 9083 | ANK | Ankyrin repeats | strong |
| 5 | 115762 | 7tm_1 | 7 transmembrane receptor (rhodopsin family) | weak |
| 11 | 2334 | EFhand | EF-hand | strong |
| 12 | 2334 | Efhand | EF-hand | strong |
| 15 | 376130 | | Endogenous retrograde protease/integrase | |
| 16 | 402380 | Rrm | RNA recognition motif. (aka RRM, RBD, or RNP domain) | |

### Glycosyl hydrolase family 5 (GLYCOSYL HYDROL F5; Pfam Accession No.

PS00659; PDOC00565). SEQ ID NO: 1 corresponds to a gene encoding a polypeptide having homology to polypeptides of the glycosyl hydrolase family 5 (Henrissat Biochem. J. (1991) 280:309-316) (also known as the cellulase family A (Henrissat et al. Gene (1989) 81:83-95)). The members of this family participate in the degradation of cellulose and xylans, and are generally found in bacteria, fungi, and yeast. The consensus pattern for members of this family is: [LIV]-[LIVMFYWGA](2)-[DNEQG]-[LIVMGST]-x-N-E-[PV]-[RHDNSTLIVFY] (where E is a putative active site residue).

SEQ ID NO: 1 corresponds to a gene encoding a member of one of the families of glycosyl hydrolases (Henrissat et al. Biochem. J. (1993) 293:781-788). These enzymes contain at least one conserved glutamic acid residue (or aspartic acid residue) which has been shown to be directly involved in glycosidic bond cleavage by acting as a nucleophile.

Ank Repeats (ANK; Pfam Accession No. PF0023). SEQ ID NO:3 corresponds to a gene encoding an Ank repeat-containing protein. The ankyrin motif is a 33 amino acid sequence named after the protein ankyrin which has 24 tandem 33-amino-acid motifs. Ank repeats were originally identified in the cell-cycle-control protein cdc10 (Breeden et al., Nature (1987) 329:651). Proteins containing ankyrin repeats include ankyrin, myotropin, I-kappaB proteins, cell cycle protein cdc10, the Notch receptor (Matsuno et al., Development (1997) 124(21):4265); G9a (or BAT8) of the class III region of the major histocompatibility complex (Biochem J. 290:811-818, 1993), FABP, GABP, 53BP2, Lin12, glp-1, SW14, and SW16. The functions of the ankyrin repeats are compatible with a role in protein-protein interactions (Bork, Proteins (1993) 17(4):363; Lambert and Bennet, Eur. J. Biochem. (1993) 211: 1; Kerr et al., Current Op. Cell Biol. (1992) 4:496; Bennet et al., J. Biol. Chem. (1980) 255:6424).

Seven Transmembrane Integral Membrane Proteins -- Rhodopsin Family (7tm 1: Pfam Accession No. PF00001). SEQ ID NO:3 corresponds to a gene encoding a polypeptide that is a member of the seven transmembrane (7tm) receptor rhodopsin family. G-protein coupled receptors of the (7tm) rhodopsin family (also called R7G) are an extensive group of hormones, neurotransmitters, and light receptors which transduce extracellular signals by interaction with guanine nucleotide-binding (G) proteins (Strosberg A.D. Eur. J. Biochem. (1991) 196:1, Kerlavage A.R Curr. Opin. Struct. Biol. (1991) 1:394, Probst, et al., DNA Cell Biol. (1992) 11:1, Savarese, et al., Biochem. J. (1992) 283:1, http://www.gcrdb.uthscsa.edu/, http://swift.embl-heidelberg.de/7tm/. The consensus pattern that contains the conserved triplet and that also spans the major part of the third transmembrane helix is used to detect this widespread family of proteins: [GSTALIVMFYWC]-[GSTANCPDE]-{EDPKRH}-x(2)-[LIVMNQGA]-x(2)- [LIVMFT]-[GSTANC]-[LIVMFYWSTAC]-[DENH]-R-[FYWCSH]-x(2)-[LIVM].

EF Hand (EFhand: Pfam Accession No. PF00036). SEQ ID NOS:11 and 12 correspond to genes encoding a protein in the family of EF-hand proteins. Many calcium-binding proteins belong to the same evolutionary family and share a type of calcium-binding domain known as the EF-hand (Kawasaki et al., Protein. Prof (1995) 2:305-490). This type of domain consists of a twelve residue loop flanked on both sides by a twelve residue alpha-helical domain. In an EF-hand loop the calcium ion is coordinated in a pentagonal bipyramidal configuration. The six residues involved in the binding are in positions 1, 3, 5, 7, 9 and 12; these residues are denoted by X, Y, Z, -Y, -X and -Z. The invariant Glu or Asp at position 12 provides two oxygens for liganding Ca (bidentate ligand). The consensus pattern includes the complete EF-hand loop as well as the first residue which follows the loop and which seem to always be hydrophobic: D-x-[DNS]-{ILVFYW}-[DENSTG]-[DNQGHRK]-{GP}-[LIVMC]-[DENQSTAGC]-x(2)-[DE]-[LIVMFYW].

Endogenous retroviral protease/integrase. SEQ ID NO:15 corresponds to a gene encoding a polypeptide having a domain homologous to a human endogenous retrovirus protease/integrase domain of a retroviral pol protein.

RNA Recognition Motif (rrm; Pfam Accession No. PF00076). SEQ ID NO:16 corresponds to a gene encoding an RNA recognition motif, also known as an RRM, RBD, or RNP domain. This domain, which is about 90 amino acids long, is contained in eukaryotic proteins that bind single-stranded RNA (Bandziulis et al. Genes Dev. (1989) 3:431-437; Dreyfuss et al. Trends Biochem. Sci. (1988) 13:86-91). Two regions within the RNA-binding domain are highly conserved: the first is a hydrophobic segment of six residues (which is called the RNP-2 motif), the second is an octapeptide motif (which is called RNP-1 or RNP-CS). The consensus pattern is: [RK]-G-{EDRKHPCG}-[AGSCI]-[FY]-[LIVA]-x-[FYLM].

### Example 4: Detection and Quantification of Polynucleotides of the Invention

The polynucleotides of the invention were detected and quantified in patient tissue samples by reverse transcriptase PCR (RT-PCR). Total RNA amplifications were performed using the LightCycler™ thermal cycling system (Roche Diagnostics) in a standard PCR reaction containing the provided primers and the dsDNA-binding dye SYBR Green I. PCR amplifacaiotn was monitored by fluroescence dye SYBR Green I, which fluroesces only when bound to double-stranded DNA. The specific of the products was verified by melting curve analysis.

Standard Preparation. 1 µg human placenta total RNA (Clontech, Palo Alto, CA) was reverse-transcribed at 42°C for 1 hour then heated at 94°C for 5 minutes in a total reaction volume of 20 µl (1st-Strand™ cDNA Synthesis Kit, Clontech). The reaction mix was used as 1x template standard. Serial dilutions from 1x template standard were then prepared: 10⁻¹x, 10⁻²x, 10⁻³x, 10⁻⁴x, 10⁻⁵x, 10⁻⁶x template standards.

Total RNA Sample Preparation. The patient tissue samples were shipped in frozen TRIZOL reagent. The samples were homogenized in TRIZOL reagent. Chloroform was then added to isolate RNA, followed by RNA precipitation with isopropanol. The RNA precipitates were washed with 75% ethanol, dried in air, then dissolved in RNase-free distilled water. Before reverse-transcription, RNA samples were treated with DNase I (RNase-free) (2 U/µl, Ambion, Austin, TX) and cleaned up using RNeasy Mini Kit (Qiagen, Santa Clarita, CA).

RT-PCR Total RNA samples were reverse-transcribed with oligo-dT₁₈ primer (1st-StrandTM cDNA Synthesis Kit, Clontech). PCR was performed using the following gene-specific primers:

| | | | |
|---|---|---|---|
| SK1: | forward primer | 5'- AGGAGTTTCTGAGGACCATGCAC -3' | (SEQ ID NO:30) |
| | reverse primer | 5'- TCAAGGGTTGGGGATACACACG -3' | (SEQ ID NO:31) |
| SK2: | forward primer | 5'- CTTGCTTGCTTTCTTCTCTGGC -3' | (SEQ ID NO:32) |
| | reverse primer | 5'- AGTCTGGAAATCCACATGACCAAG - 3' | (SEQ ID NO:33) |
| SK5: | forward primer | 5'- CCCAATGAGGAACCTAAAGTTGC -3' | (SEQ ID NO:34) |
| | reverse primer | 5'- GGTGCCAAATCTGGACTCTTGTC -3' | (SEQ ID NO:35) |
| 1665: | forward primer | 5'- GATCCATTTTCAGCAGTGCTCTG -3' | (SEQ ID NO:36) |
| | reverse primer | 5'- CAGTGTTCACAGAAGGGGTACTCAC -3' | (SEQ ID NO:37) |
| SK8: | forward primer | 5'- ACGAGAGCGACACGGACAAG -3' | (SEQ ID NO:38) |
| | reverse primer | 5'- TCTGAGGCTGTGGCAGGTGC -3' | (SEQ ID NO:39) |
| SK19: | forward primer | 5'- CCAGTCTTTGCCAACTCGTGC -3' | (SEQ ID NO:40) |
| | reverse primer | 5'- TTCGATCTTCAAACTGTGCCTTG -3' | (SEQ ID NO:4 1) |
| Junc2: | forward primer | 5'- TTGGCAACCAGACCAGCATC -3' | (SEQ ID NO:42) |
| | reverse primer | 5'- TTTCCCATAGGTGTGAGTGGCG -3' | (SEQ ID NO:43) |
| XD4: | forward primer | 5'- GACTGGTGTTTTGTTCGGGGTC -3' | (SEQ ID NO:44) |
| | reverse primer | 5'- TTTGTCCAAGGCTGCATGGTC -3' | (SEQ ID NO:45) |
| XD1: | forward primer | 5'- TGCCCTGGTTAAGCCAGAAGTC -3' | (SEQ ID NO:46) |
| | reverse primer | 5'- AGCTTCACTTTGGTCTTGACGG -3' | (SEQ ID NO:47) |
| XD7: | forward primer | 5'- GGTCATCTGCATCAAGGTTGGC -3' | (SEQ ID NO:48) |
| | reverse primer | 5'- GGTTCGTAACCGTGACTTCAGG -3' | (SEQ ID NO:49) |
| XD10: | forward primer | 5'- GCATCCTTTTCCAGTCTTCCG -3' | (SEQ ID NO:50) |
| | reverse primer | 5'- TGCAGCAAACATGCCTGAGC -3' | (SEQ ID NO:51) |
| XD11: | forward primer | 5'- TGTTCCACGAGCAAAGCATGTG -3' | (SEQ ID NO:52) |
| | reverse primer | 5'- ATCCTTCTTCCACTCCCGCTTC -3' | (SEQ ID NO:53) |
| 37641: | forward primer | 5'- TCGGCTTGACTACACTGTGTGG -3' | (SEQ ID NO:54) |
| | reverse primer | 5'- TACAAAGACCACTGGGAGGCTG -3' | (SEQ ID NO:55) |
| β-actin: | forward primer | 5'- CGGGAAATCGTGCGTGACATTAAG - 3' | (SEQ ID NO:56) |
| | reverse primer | 5'- TGATCTCCTTCTGCATCCTGTCGG -3' | (SEQ ID NO:57) |
| GAPDH: | forward primer | 5'- TTTGGCTACAGCAACAGGGTG -3' | (SEQ ID NO:5 8) |
| | reverse primer | 5'- TGTGAGGAGGGGAGATTCAGTG -3' | (SEQ ID NO:59) |

β-actin and GAPDH were used as positive controls. All PCR products are 150-250 bp. The 20-µl PCR reaction mix in each LightCycler™ capillary contained 2 µl of 10x PCR buffer 11, 3 mM MgCl₂ (Perkin-Elmer, Foster City, CA), 140 µM dNTP, 1:50000 of SYBR Green 1,0.25 mg/ml BSA, 1 unit of Taq polymerase (Boehringer Mannheim, Indianapolis, IN), 0.175 µM each primer, 2 µl of RT reaction mix. The PCR amplification began with 20-second denaturation at 95°C, followed by 45 cycles of denaturation at 95°C for 5 seconds, annealing at 60°C for 1 second and extension at 72°C for 30 seconds. At the end of final cycle, PCR products were annealed at 60°C for 5 seconds, then slowly heated to 95°C at 0.2°C/second, to measure melting curve of specific PCR products. All experiments were performed in duplicate.

Data analysis was performed using LightCycler™ software (Roche Diagnostics) with quantification and melting curve options. Fluorescence is normalized relative to positive and negative controls.

Overexpression of genes in colon cancer patient whole tissue. Results provided in the tables below include fluoresence data for polynucleotides isolated from colon tissue samples that were harvested directly, not microdissected (*i.e.,* whole tissue), and amplified using the indicated primers. Normal, primary tumor and metastatic cell types are denoted as N, PT and Met, respectively. Overexpression was determined by comparing either metastatic cells or primary tumor cells, or both, to normal cells. The results for each gene corresponding to the indicated clusters in each patient sample are summarized in the tables below. All values are adjusted to levels relative to beta-actin control.

| Cluster#719 (SK1): overexpression detected in 4 of 6 patients (67%) | | | |
|---|---|---|---|
| Patients | N | PT | MET |
| UC#1 | 0.022 | 0.117 | 0.364 |
| UC#2 | 0.121 | 0.109 | 0.142 |
| UC#4 | 0.083 | 0.053 | 0.078 |
| UC#7 | 0.042 | 0.199 | 0.145 |
| UC#8 | 0.215 | 0.515 | 0.794 |
| UC#9 | 0.233 | 0.585 | 0.613 |

| Cluster#9083 (SK2): overexpression inf 3 or 4 patients (75%) | | | |
|---|---|---|---|
| Patients | N | PT | MET |
| UC#1 | 0.0021 | 0.0013 | 0.0078 |
| UC#2 | 0.008 | 0.012 | 0.014 |
| UC#4 | 0.0021 | 0.0022 | 0.0026 |
| UC#7 | 0.0009 | 0.0021 | 0.0039 |

| Cluster#115762 (SK5): overexpression in 5 of 6 patients (83%) | | | |
|---|---|---|---|
| Patients | N | PT | MET |
| UC#1 | 0.0053 | 0.0159 | 0.044 |
| UC#2 | 0.0195 | 0.0174 | 0.0269 |
| UC#4 | 0.022 | 0.033 | 0.034 |
| UC#7 | 0.013 | 0.028 | 0.025 |
| UC#8 | 0.0275 | 0.105 | 0.143 |
| UC#9 | 0.0336 | 0.0595 | 0.0541 |

| Cluster# 1665: overexpression in 4 of 6 patients (67%) | | | |
|---|---|---|---|
| Patients | N | PT | MET |
| UC# 1 | 0.00006 | 0.0003 | 0.002 |
| UC#2 | 0.0015 | 0.001 | 0.0012 |
| UC#4 | 0.0016 | 0.0013 | 0.0016 |
| UC#7 | 0.00003 | 0.0003 | 0.0012 |
| UC#8 | 0.0016 | 0.0122 | 0.0154 |
| UC#9 | 0.006 | 0.057 | 0.097 |

| Cluster#2334 (SK8): overexpression in 4 of 6 patients (67%) | | | |
|---|---|---|---|
| Patients | N | PT | MET |
| UC# 1 | 0.011 | 0.022 | 0.017 |
| UC#2 | 0.0266 | 0.0317 | 0.026 |
| UC#4 | 0.02 | 0.006 | 0.01 |
| UC#7 | 0.046 | 0.093 | 0.042 |
| UC#8 | 0.042 | 0.168 | 0.472 |
| UC#9 | 0.208 | 0.322 | 0.29 |

| Cluster#3376 (SK19): overexpression in 4 of 6 patients (67%) | | | |
|---|---|---|---|
| Patients | N | PT | MET |
| UC#1 | 0.00018 | 0.00042 | 0.0012 |
| UC#2 | 0.002 | 0.0025 | 0.0016 |
| UC#4 | 0.0013 | 0.0012 | 0.002 |
| UC#7 | 0.00024 | 0.00055 | 0.00062 |
| UC#8 | 0.0003 | 0.00127 | 0.0023 |
| UC#9 | 0.001 | 0.0075 | 0.009 |

| Cluster#376130 (Junc2): overexpression in 3 of 4 patients (75%) | | | |
|---|---|---|---|
| Patients | N | PT | MET |
| UC#1 | 0.00871 | 0.0111 | 0.0142 |
| UC#2 | 0.000567 | 0.00663 | 0.0163 |
| UC#4 | 0.000107 | 0.00048 | 0.000237 |
| UC#7 | 0.0000401 | 0.000259 | 0.00159 |

| Cluster#402380 (XD4): overexpression in 2 of 4 patients (50%) | | | |
|---|---|---|---|
| Patients | N | PT | MET |
| UC# 1 | 0.0763 | 0.123 | 0.2 |
| UC#2 | 0.0867 | 0.0629 | 0.069 |
| UC#4 | 0.0735 | 0.0672 | 0.0664 |
| UC#7 | 0.0559 | 0.112 | 0.139 |

| Cluster#726682 (XD1): overexpression in 0 of 4 patients | | | |
|---|---|---|---|
| Patients | N | PT | MET |
| UC#1 | 0.0679 | 0.0822 | 0.136 |
| UC#2 | 0.175 | 0.124 | 0.147 |
| UC#4 | 0.2 | 0.145 | 0.145 |
| UC#7 | 0.108 | 0.144 | 0.114 |

| Cluster#552930 (XD7): overexpression in 1 of 4 patients (25%) | | | |
|---|---|---|---|
| Patients | N | PT | MET |
| UC# 1 | 0.018 | 0.019 | 0.0902 |
| UC#2 | 0.204 | 0.161 | 0.212 |
| UC#4 | 0.299 | 0.25 | 0.238 |
| UC#7 | 0.246 | 0.409 | 0.248 |

| Cluster#454001 (XD10): overexpression in 2 of 4 patients) | | | |
|---|---|---|---|
| Patients | N | PT | MET |
| UC#1 | 0.0197 | 0.0363 | 0.0587 |
| UC#2 | 0.0514 | 0.0451 | 0.069 |
| UC#4 | 0.0587 | 0.0889 | 0.096 |
| UC#7 | 0.0342 | 0.1 | 0.0705 |

| Cluster#378805 (XD11): overexpression in 1 of 4 patients) | | | |
|---|---|---|---|
| Patients | N | PT | MET |
| UC#1 | 0.00117 | 0.00269 | 0.00697 |
| UC#2 | 0.00864 | 0.00371 | 0.00672 |
| UC#4 | 0.0098 | 0.00525 | 0.00497 |
| UC#7 | 0.00912 | 0.00989 | 0.0127 |

| Cluster#374641: overexpression in 3 of 4 patients (75%) | | | |
|---|---|---|---|
| Patients | N | PT | MET |
| UC# 1 | 0.0124 | 0.163 | 0.0947 |
| UC#2 | 0.28 | 0.317 | 0.544 |
| UC#4 | 0.685 | 1.809 | 1.996 |
| UC#7 | 0.569 | 1.714 | 1.073 |

Overexpression of genes in colon cancer patient epithelium. Results provided in the tables below include fluorescence data for polynucleotides isolated from colon epithelial cells that were prepared by the epithelial shakeoff method to obtain >97% pure epithelium without stroma. Normal, precancerous (adenomatous polyp), and primary tumor cell types are denoted as N, polyp and PT, respectively. Overexpression was determined by comparing either primary tumor cells or precancerous cells, or both, to normal cells. All values are adjusted to levels relative to beta-actin control.

| Cluster#719 (SK1): overexpression in 4 of 4 patients (100%) | | | |
|---|---|---|---|
| Patients | N | Polyp | PT |
| UW#17 | 0.0924 | 0.117 | N/A |
| UW#18 | 0.0864 | N/A | 0.327 |
| UW#19 | 0.151 | N/A | 0.227 |
| UW#20 | 0.0624 | 0.162 | 0.164 |

| Cluster# 115762 (SK5): overexpression in 4 of 4 patients (100%). | | | |
|---|---|---|---|
| Patients | N | Polyp | PT |
| UW#17 | 0.00724 | 0.0122 | N/A |
| UW#18 | 0.0156 | N/A | 0.111 |
| UW#19 | 0.0158 | N/A | 0.0461 |
| UW#20 | 0.00728 | 0.0187 | 0.0306 |

| Cluster#1665: overexpression in 4 of 4 patients (100%) | | | |
|---|---|---|---|
| Patients | N | Polyp | PT |
| UW#17 | 0.0041 | 0.0306 | N/A |
| UW#18 | 0.0029 | N/A | 0.0357 |
| UW#19 | 0.0045 | N/A | 0.0357 |
| UW#20 | 0.0028 | 0.025 | 0.047 |

| Cluster#2334 (SK8) overexpressed in 1 of 4 patients (25%) | | | |
|---|---|---|---|
| Patients | N | Polyp | PT |
| UW#17 | 0.1835 | 0.041 | N/A |
| UW#18 | 0.0638 | N/A | 0.0927 |
| UW#19 | 0.04 | N/A | 0.04 |
| UW#20 | 0.2236 | 0.0576 | 0.0454 |

| Cluster#3376 (SK19) overexpressed in 4 of 4 patients (100%) | | | |
|---|---|---|---|
| Patients | N | Polyp | PT |
| UW#17 | 0.0053 | 0.012 | N/A |
| UW#18 | 0.0028 | N/A | 0.0084 |
| UW#19 | 0.003 | N/A | 0.0135 |
| UW#20 | 10.0023 | 10.023 | 10.012 |

### Example 5: Northern Blot Analysis

Differential gene expression in cancerous colon cells can be further confirmed by other techniques, such as Northern blot analysis. Northern analysis can be accomplished by methods well-known in the art. Briefly, rapid-Hyb buffer (Amersham Life Science, Little Chalfont, England) with 5 mg/ml denatured single stranded sperm DNA is pre-warmed to 65°C and human colon tumor total RNA blots (Invitrogen, Carlsbad, CA) are pre-hybridized in the buffer with shaking at 65°C for 30 minutes. Gene-specific DNA probes (50 ng per reaction) labeled with [α-32P]dCTP (3000Ci/mmol, Amersham Pharmacia Biotech Inc., Piscataway, NJ) (Prime-It RmT Kit, Stratagene, La Jolla, CA) and purified with ProbeQuantTM G-50 Micro Columns (Amersham Pharmacia Biotech Inc.) are added and hybridized to the blots with shaking at 65°C for overnight. The blots are washed in 2x SSC, 0.1%(w/v) SDS at room temperature for 20 minutes, twice in 1x SSC, 0.1%(w/v) SDS at 65°C for 15 minutes, then exposed to Hyperfilms (Amersham Life Science).

### Example 6: Analysis of expression of gene corresponding to SK2 (cluster 9083 (c9083)) (SEQ ID NO:3) in colorectal carcinoma

The expression of the gene comprising the sequence of SK2, which clusters to cluster i.d. no. 9083, was examined by quantitative PCR in several cancer cell lines, including a number of colorectal carcinoma cell lines. The cells in which expression was tested are summarized below.

| **Cell Line** | **Tissue Source** | **Cell Line** | **Tissue Source** |
|---|---|---|---|
| MDA-MB-231 | Human breast; high metastatic potential (micromets in lung; adenocarcinoma; pleural effusion | Caco-2 | Human colorectal adenocarcinoma |
| MDA-MB-435 | Human breast, high metastatic potential (macrometastases in lung) | SW620 | Human colorectal adenocarcinoma; from metastatic site (lymph node) |
| MCF-7 | Human breast; non-metastatic | LS 174T | High metastatic potential human colorectal adenocarcinoma |
| MDA-MB-468 | Human breast; adenocarcinoma | LOVO | Human colorectal adenocarcinoma; colon; from metastatic site (colon) |
| Alab | Human breast, metastatic | HT29 | Human colorectal adenocarcinoma; colon |
| SKOV3 | Human ovarian adenocarcinoma | SW480 | Human colorectal adenocarcinoma; colon |
| OVCAR3 | Human ovarian adenocarcinoma | HCT116 | Human colorectal carcinoma; colon |
| KM 12C | Human colon; low metastatic potential | Colo 320DN | Human colorectal adenocarcinoma; colon |
| KM12L4 | Human colon; high metastatic potential (derived from Km12C) | T84 | Human colorectal carcinoma; colon; from metastatic site (lung) |
| DU 145 | Human prostate; carcinoma; from metastatic site: brain | HCT15 | Human colorectal adenocarcinoma; colon |
| HT 1080 | Human sarcoma cell line; | CCD 112 | Human colorectal adenocarcinoma, low metastatic potential |
| HMVEC | Primary human microvascular endothelial cells | DLD1 | Human colon; colorectal adenocarcinoma |
| 185B4 | normal breast epithelial cells; chemically transformed | 293 | kidney epithelial cells |
| LNCAP | prostate carcinoma; metastasis to left supraclavicular lymph | GRDP | primary prostate epithelium |
| U373MG | glioblastoma cell | IMR90 | primary lung fibroblast |
| WOCA | primary prostate epithelium | PC3 | prostate cancer; androgen receptor negative |

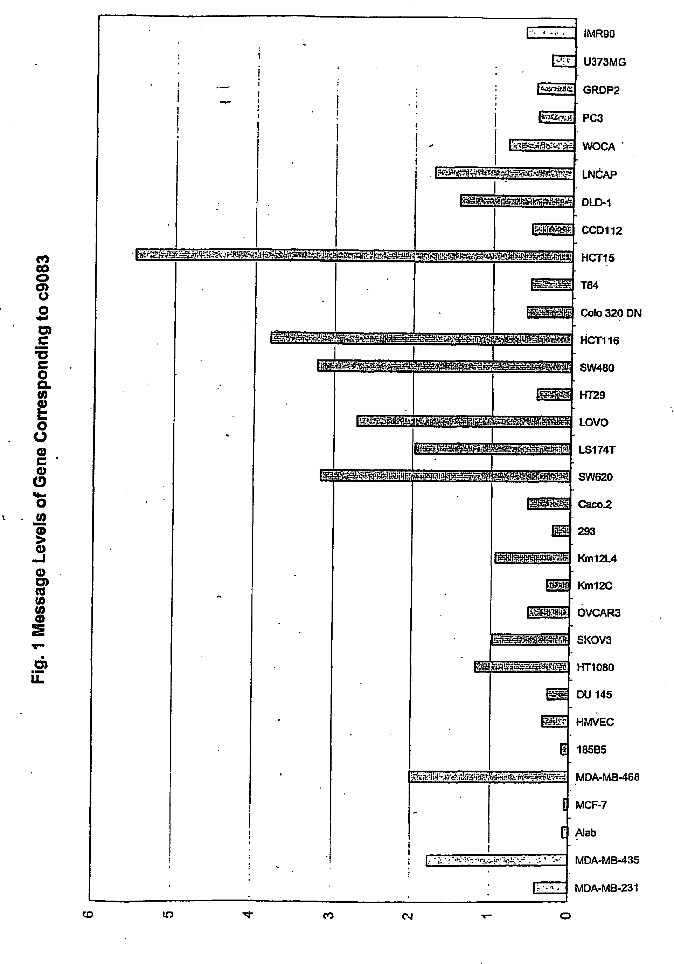

Quantitative real-time PCR was performed by first isolating RNA from cells using a Roche RNA Isolation kit according to manufacturer's directions. One microgram of RNA was used to synthesize a first-strand cDNA using MMLV reverse transcriptase (Ambion) using the manufacturers buffer and recommended concentrations of oligo dT, nucleotides, and Rnasin. This first-strand cDNA served as a template for quantitative real-time PCR using the Roche light-cycler as recommended in the machine manual. The gene corresponding to SK2 (C9083) (SEQ ID NO:3) was amplified with forward primer: 5'-cgctgacctcaaccag-3' (SEQ ID NO:60) and reverse primer: 5'-ctgtttgcccgttcttattac-3' (SEQ ID NO:61). Product was quantified based on the cycle at which the amplification entered the linear phase of amplification in comparison to an internal standard and using the software supplied by the manufacturer. Small differences in amounts or total template in the first-strand cDNA reaction were eliminated by normalizing to amount of actin amplified in a separate quantitative PCR reaction using the forward primer 5'-CGGGAAATCGTGCGTGACATTAAG-3' (SEQ ID NO:56) and the reverse primer: 5'-TGATCTCCTTCTGCATCCTGTCGG-3' (SEQ ID NO:57). The results are shown in Fig. 1

### Example 7: Functional analysis of gene corresponding to SK2 (c9083) (SEQ ID NO:3)

In order to further assess the role of the gene corresponding to SK2 (c9083) (SEQ ID NO:3), the functional information on the gene corresponding to this sequence was obtained using antisense knockout technology. In short, the cell type to be tested, SW620 or HT1080 cells which express the polypeptide encoded by the gene corresponding to c9083, were plated to approximately 60-80% confluency on 6-well or, for proliferation assays, 96-well dishes. Antisense or reverse control oligonucleotide was diluted to 2 µM in optimem and added to optimem into which the delivery vehicle, lipitoid 116-6 in the case of SW620 cells or 1:1 lipitoid 1:cholesteroid 1 in the case of HT1080 cells, had been diluted. The oligo/ delivery vehicle mixture was then further diluted into medium with serum on the cells. The final concentration of oligonucleotide for all experiments was 300 nM, and the final ratio of oligo to delivery vehicle for all experiments was 1.5 nmol lipitoid/µg oligonucleotide. Cells were transfected overnight at 37 C and the transfection mixture was replaced with fresh medium the next morning.

The following antisense oligonucleotides were tested for the ability to deplete c9083 (SEQ ID NO:3) RNA:

| **Olig Name** | **Sequence** | **Nucleotides** |
|---|---|---|
| CHIR-8-4AS C9083:P0463 | ATTTGGGCATCACTGGCTACAAGCA (SEQ ID NO:64) | 25 |
| CHIR-8-4RC C9083:P0463RC | ACGAACATCGGTCACTACGGGTTTA (SEQ ID NO:65) | 25 |
| CHIR-8-5AS C9083:P0157 | CAGAGAGGTGAGACACTCGCCGCA (SEQ ID NO:66) | 24 |
| CHIR-8-SRC C9083:P0157RC | ACGCCGCTCACAGAGTGGAGAGAC (SEQ ID NO:67) | 24 |
| RC: reverse control oligos (control oligos); AS: antisense oligos (test) | | |

The effect of the oligonucleotide on the cells was assessed by both quantitation of PCR levels as described above, and in proliferation assays using amount of DNA as quantified with the Stratagene Quantos™ kit to determine cell number.

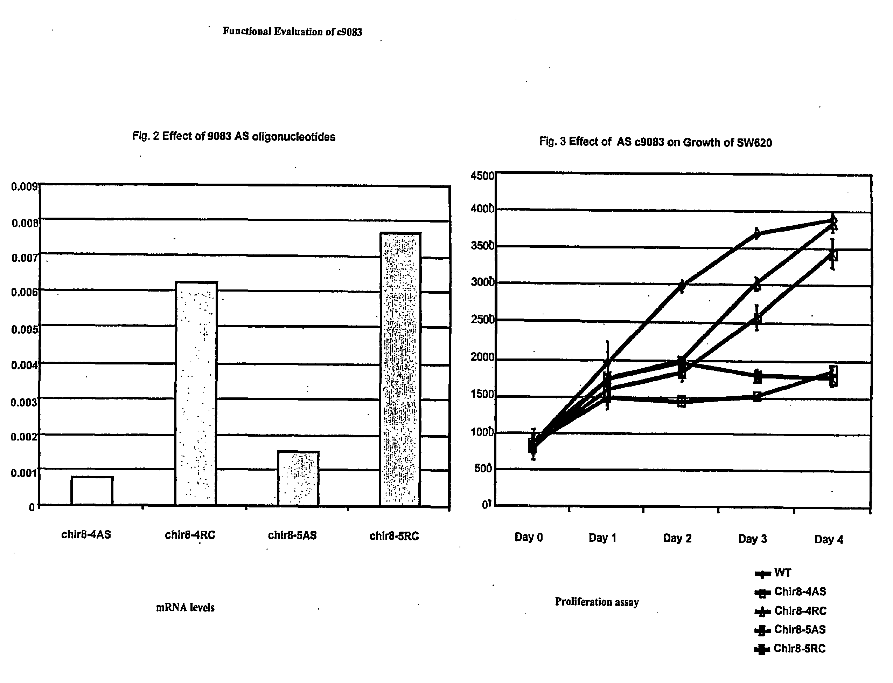

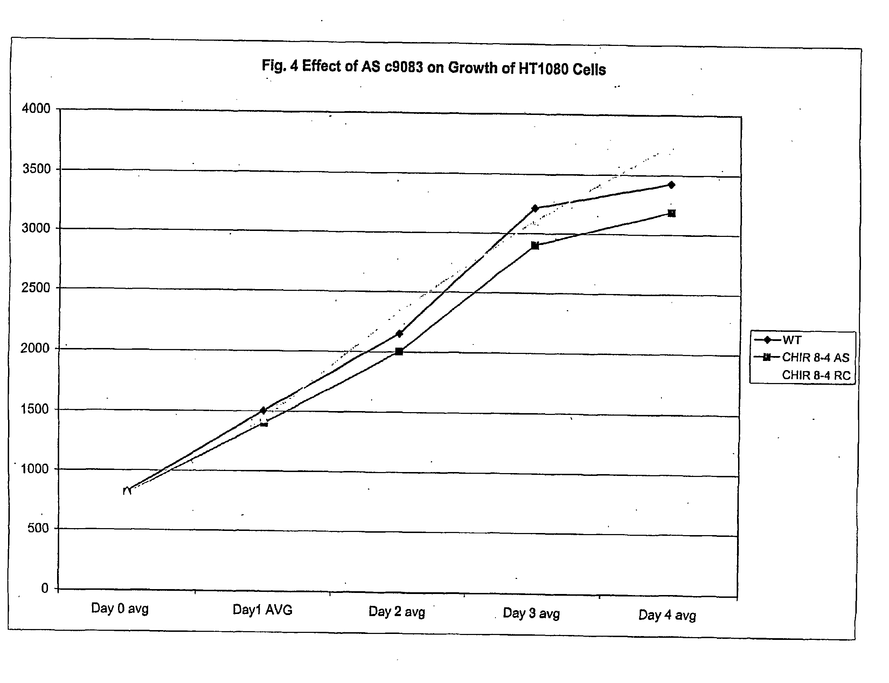

The results of the mRNA level quantitation are shown in Fig. 2. The effects of the oligonucleotides upon proliferation over a four day period are shown in Figs. 3 and 4. Cells without oligonucleotide treatment (WT) served as a control. The oligo CHIR-8-4AS was most effective in decreasing mRNA for the gene corresponding to 9083c. Transfection of these oligos into SW620 cells resulted in a decreased rate of proliferation relative to matched reverse control oligos, with CHIR 8-4 being somewhat more effective than CHIR-8-5 (Fig. 3). Significantly, the same antisense oligonucleotide had no effect on growth of a fibrosarcoma cell line, HT1080 (Fig. 4). This indicates that the functional role of the gene corresponding to c9083 is tissue-specific, and further that the gene corresponding to c9083 has a specific effect on growth.

The oligos were next tested for their effect on colony formation in a soft agar assay. Soft agar assays were conducted by first establishing a bottom layer of 2 ml of 0.6% agar in media plated fresh within a few hours of layering on the cells. The cell layer was formed on the bottom layer by removing cells transfected as described above (either an antisense k-Ras oligo as a positive control), CHIR-8-4, CHIR-8-5, CHIR-8-4RC, or CHIR-8-SRC) from plates using 0.05% trypsin and washing twice in media. The cells were counted in a Coulter counter, and resuspended to 10⁶ per ml in media. 10 µl aliquots are placed with media in 96-well plates (to check counting with WST1), or diluted further for soft agar assay. 2000 cells are plated in 800 µl 0.4% agar in duplicate wells above 0.6% agar bottom layer. After the cell layer agar solidifies, 2 ml of media is dribbled on top and antisense or reverse control oligo is added without delivery vehicles. Fresh media and oligos are added every 3-4 days. Colonies are formed in 10 days to 3 weeks. Fields of colonies were counted by eye. WST-1 metabolism values can be used to compensate for small differences in starting cell number. Larger fields can be scanned for visual record of differences.

Both the CHIR-8-4 and CHIR-8-5 antisense oligos led to decreased colony size and number compared to the control CHIR-8-4RC and CHIR-8-SRC oligos. These results further validate the gene corresponding to c9083 (SEQ ID NO:3) as a target for therapeutic intervention.

### Example 8: Effect of antisense oligonucleotides on message levels for target genes

The effect of antisense oligonucleotides upon message levels for the genes corresponding to the sequences and clusters described herein was analyzed using antisense knockout technology as described for c9083 in the Example above. Specifically, antisense oligos for genes corresponding to each of c719, c1665, c3376, c115762, c454001, c3788805, and c776682 were prepared as described above. Once synthesized and quantitated, the oligomers were screened for efficiency of a transcript knock-out in a panel of cancer cell lines. The efficiency of the knock-out was determined by analyzing mRNA levels using lightcycler quantification. The oligomers that resulted in the highest level of transcript knock-out, wherein the level was at least about 50%, preferably about 80-90%, up to 95% or more up to undetectable message, were selected for use in a cell-based proliferation assay, an anchorage independent growth assay, and an apoptosis assay.

SW620 cells, which express the polypeptide encoded by the corresponding genes to be analyzed, were plated to approximately 60-80% confluency on 6-well or, for proliferation assays, 96-well dishes. For each transfection mixture, a carrier molecule, preferably a lipitoid or cholesteroid, was prepared to a working concentration of 0.5 mM in water, sonicated to yield a uniform solution, and filtered through a 0.45 µm PVDF membrane. The antisense or control oligonucleotide was then prepared to a working concentration of 100 µM in sterile Millipore water. The oligonucleotide was further diluted in OptiMEM™ (GibcoBRL), in a microfuge tube, to 2 µM, or approximately 20 µg oligo/ml of OptiMEM™. In a separate microfuge tube, lipitoid or cholesteroid, typically in the amount of about 1.5-2 nmol lipitoid/µg antisense oligonucleotide, was diluted into the same volume of OptiMEM™ used to dilute the oligonucleotide. The diluted antisense oligonucleotide was immediately added to the diluted lipitoid and mixed by pipetting up and down. Oligonucleotide was added to the cells to a final concentration of 30 nM.

The level of target mRNA that corresponds to a target gene of interest in the transfected cells was quantitated in the cancer cell lines using the Roche LightCycler™ real-time PCR machine. Values for the target mRNA were normalized versus an internal control (e.g., beta-actin). For each 20 µl reaction, extracted RNA (generally 0.2-1 µg total) was placed into a sterile 0.5 or 1.5 ml microcentrifuge tube, and water was added to a total volume of 12.5 µl. To each tube was added 7.5 µl of a buffer/enzyme mixture, prepared by mixing (in the order listed) 2.5 µl H₂O, 2.0 µl 10X reaction buffer, 10 µl oligo dT (20 pmol), 1.0 µl dNTP mix (10 mM each), 0.5 µl RNAsin® (20u) (Ambion, Inc., Hialeah, FL), and 0.5 µl MMLV reverse transcriptase (50u) (Ambion, Inc.). The contents were mixed by pipetting up and down, and the reaction mixture was incubated at 42°C for 1 hour. The contents of each tube were centrifuged prior to amplification.

An amplification mixture was prepared by mixing in the following order: 1X PCR buffer II, 3 mM MgCl₂, 140 µM each dNTP, 0.175 pmol each oligo, 1:50,000 dil of SYBR® Green, 0.25 mg/ml BSA, 1 unit *Taq* polymerase, and H₂O to 20 µl. (PCR buffer II is available in 10X concentration from Perldn-Elmer, Norwalk, CT). In 1X concentration it contains 10 mM Tris pH 8.3 and 50 mM KCl. SYBR® Green (Molecular Probes, Eugene, OR) is a dye which fluoresces when bound to double stranded DNA. As double stranded PCR product is produced during amplification, the fluorescence from SYBR® Green increases. To each 20 µl aliquot of amplification mixture, 2 µl of template RT was added, and amplification was carried out according to standard protocols.

The following antisense oligonucleotides were tested for the ability to deplete the message levels of the gene corresponding to the indicated cluster. Target Gene : Oligo Location provides the name of the cluster to which the target gene is assigned and the name of the oligo used. AS indicates antisense; RC indicates reverse control. Data for the genes corresponding to c9083 are provided for comparison.

| **Target Gene:Oligo Location** | **Oligo Sequence** | **SEQ ID NO:** | **% KO of Message** |
|---|---|---|---|
| c719:1-AS | TTGGTGTCATTGGGTCAAGGGTTGG | 68 | 85% |
| C719:1-RC | GGTTGGGAACTGGGTTACTGTGGTT | 69 | |
| c719:2-AS | ACAGGGCAGATACGGACCTCGGTG | 70 | 93% |
| c719:2-RC | GTGGCTCCAGGCATAGACGGGACA | 71 | |
| c719:3-AS | TTGTGGGTAAGCAGTTTCATGTCGC | 72 | 67% |
| c719:3-RC | CGCTGTACTTTGACGAATGGGTGTT | 73 | |
| c719:4-AS | CCTGGATCAGACGCAAGTTATCGGC | 74 | 85% |
| c719:4-RC | CGGCTATTGAACGCAGACTAGGTCC | 75 | |
| C9083:4-AS | ATTTGGGCATCACTGGCTACAAGCA | 64 | 83.0 |
| C9083:4-RC | ACGAACATCGGTCACTACGGGTTTA | 65 | |
| C9083:5-AS | CAGAGAGGTGAGACACTCGCCGCA | 66 | 73.0 |
| C9083:5-RC | ACGCCGCTCACAGAGTGGAGAGAC | 67 | |
| C1665:1-AS | CTACTCCCCACACTTCATCGCCAGG | 76 | 73.0 |
| C1665:1-RC | GGACCGCTACTTCACACCCCTCATC | 77 | |
| C1665:2-AS | CTCTTGATACTCCAGCGGCAAACCA | 78 | 81.0 |
| C1665:2-RC | ACCAAACGGCGACCTCATAGTTCTC | 79 | |
| c3376:1-AS | GCGCCCAAGCCGTTCGTTCTTAAG | 80 | 78.0 |
| c3376:1-RC | GAATTCTTGCTTGCCGAACCCGCG | 81 | |
| c3376:2-AS | CCAGGTAGGCACGAGTTGGCAAAGA | 82 | 97.0 |
| c3376:2-RC | AGAAACGGTTGAGCACGGATGGACC | 83 | |
| c3376:3-AS | GCCATTGAAGATGCCCAGATCCCAC | 84 | 56.0 |
| c3376:3-RC | CACCCTAGACCCGTAGAAGTTACCG | 85 | |
| c3376:4-AS | CCTGCGTTTGTCCCTCCAGCATCT | 86 | 93.0 |
| c3376:4-RC | TCTACGACCTCCCTGTTTGCGTCC | 87 | |
| c3376:5-AS | AAGTCACAGTCCCCGGATACCAGTC | 88 | 88.0 |
| c3376:5-RC | CTGACCATAGGCCCCTGACACTGAA | 89 | |
| c115762:1-AS | TTGTCGCTTTGGCAGGCATAAAACC | 90 | 97.5 |
| c115762:2-AS | TCTGGTCATCAACTTGCTTTCCGTG | 91 | 99.0 |
| c115762:3-AS | CAGTGTTTCGTGGTGTGCTCTGTGG | 92 | 98.0 |
| c115762:4-AS | GCTCACCATCCGGGCACCAAGCA | 93 | 97.0 |
| c115762:5-AS | TGAGAGACAGTGTTTCGTGGTGTGC | 94 | 93.0 |
| 454001:1-AS | TGCCTTCACACGCTTGGTTATCTTC | 95 | 0 |
| 454001:2-AS | GACAACATCGGAGGCTTCAATCACC | 96 | 0 |
| 454001:3-AS | GTTGAGGCTCTGAACACCACTGTTG | 97 | 0 |
| 454001:4-AS | GTTTGGCAGCACCTTCAACATTTGG | 98 | 87 |
| 454001:5-AS | AGCAGTTTGGCAGCACCTTCAACA | 99 | 92 |
| 454001:1-RC | CTTCTATTGGTTCGCACACTTCCGT | 100 | |
| 454001:2-RC | CCACTAACTTCGGAGGCTACAACAG | 101 | |
| 454001:3-RC | GTTGTCACCACAAGTCTCGGAGTTG | 102 | |
| 454001:4-RC | GGTTTACAACTTCCACGACGGTTTG | 103 | |
| 454001:5-RC | ACAACTTCCACGACGGTTTGACGA | 104 | |
| 378805:1-AS | ATCTGGCATGGACGGATGAGCGAA | 105 | 41.0 |
| 378805:2-AS | GCTGGGTGGTTTCCGAACTCAACG | 106 | 97 |
| 378805:3-AS | GTCCCAATCACCTTCCCCACAATCC | 107 | 65.0 |
| 378805:4-AS | TCAGATCCTTCTTCCACTCCCGCTT | 108 | 100.0 |
| 378805:5-AS | TGCTCGTGGAACAGGTAAAGCTCTG | 109 | 98 |
| 378805:1-RC | AAGCGAGTAGGCAGGTACGGTCTA | 110 | |
| 378805:2-RC | GCAACTCAAGCCTTTGGTGGGTCG | 111 | |
| 378805:3-RC | CCTAACACCCCTTCCACTAACCCTG | 112 | |
| 378805:4-RC | TTCGCCCTCACCTTCTTCCTAGACT | 113 | |
| 378805:5-RC | GTCTCGAAATGGACAAGGTGCTCGT | 114 | |
| 776682:1-AS | AGCTTCACTTTGGTCTTGACGGCAT | 115 | 81 |
| 776682:2-AS | CGGAGGGAAGTCAAGTCAGCCACA | 116 | 60 |
| 776682:3-AS | CGGCATTCACCCTCTCCAGCACCT | 117 | 89 |
| 776682:4-AS | CCTCCACCTGTTTGCGGGCTTCC | 118 | 61 |
| 776682:5-AS | CCACATTGAGGGAGTCCTCTTGCAA | 119 | 80 |
| 776682:1-RC | TACGGCAGTTCTGGTTTCACTTCGA | 120 | |
| 776682:2-RC | ACACCGACTGAACTGAAGGGAGGC | 121 | |
| 776682:3-RC | TCCACGACCTCTCCCACTTACGGC | 122 | |
| 776682:5-RC | CCTTCGGGCGTTTGTCCACCTCC | 123 | |
| 402380:P464:4-AS | CCCCGAACAAAACACCAGTCAACG | 124 | 94 |
| 402380:P464:4-RC | GCAACTGACCACAAAACAAGCCCC | 125 | |
| 402380:P414:5 AS | GGCCATTGAGTCCCTCCATAGCAGC | 126 | 92 |
| 402380:P414:5-RC | CGACGATACCTCCCTGAGTTACCGG | 127 | |

The effect of the oligonucleotide on the cells was assessed by quantitation of PCR levels. The results of the mRNA level quantitation are summarized in the table immediately above.

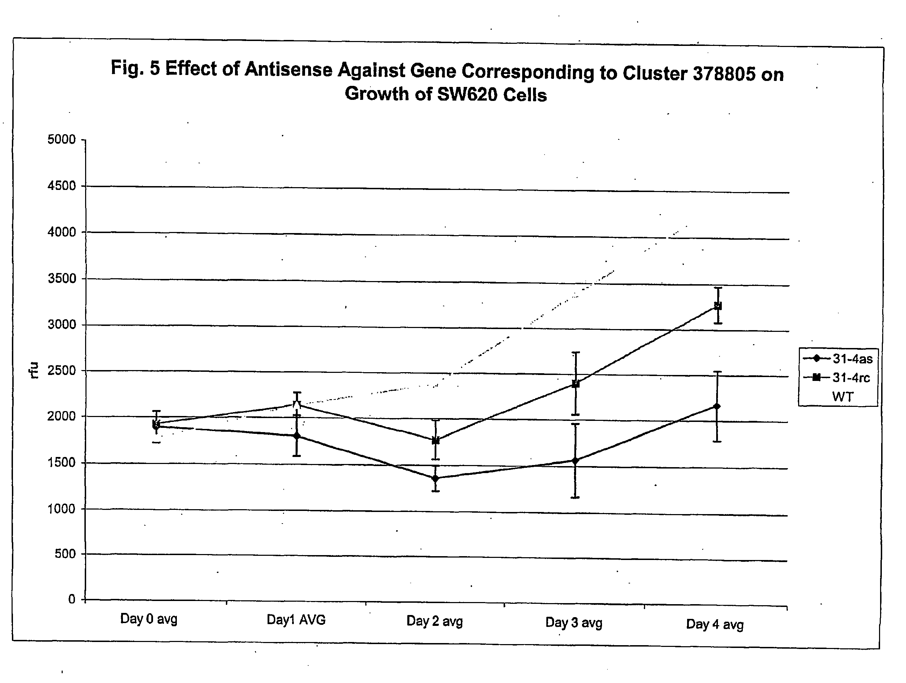

The effect of the loss of message for each gene above can be assessed in cell-based assays as described in Example 7 above. One such use of the antisense oligonucleotide described by SEQ ID NO: 108 resulted in an inhibition of proliferation of SW620 cells when used as described in the transfection and proliferation assay protocols in Example 7 (Fig. 5).

### Example 9: The Effect of Expression of Genes Corresponding to c3376 and 402380 upon on Proliferation

The effect of expression of genes corresponding to c3376 (gene corresponding to SEQ ID NO: 13) and 402380 (gene corresponding to SEQ ID NO: 16) on the inhibition of cell proliferation was assessed in SW620 colon colorectal carcinoma cells.

Cells were plated to approximately 60-80% confluency in 96-well dishes. Antisense or reverse control oligonucleotide was diluted to 2 µM in OptiMEM™ and added to OptiMEM™ into which the delivery vehicle, lipitoid 116-6 in the case of SW620 cells or 1:1 lipitoid 1:cholesteroid 1 in the case of MDA-MB-231 cells, had been diluted. The oligo/delivery vehicle mixture was then further diluted into medium with serum on the cells. The final concentration of oligonucleotide for all experiments was 300 nM, and the final ratio of oligo to delivery vehicle for all experiments was 1.5 nmol hpitoid/µg oligonucleotide.

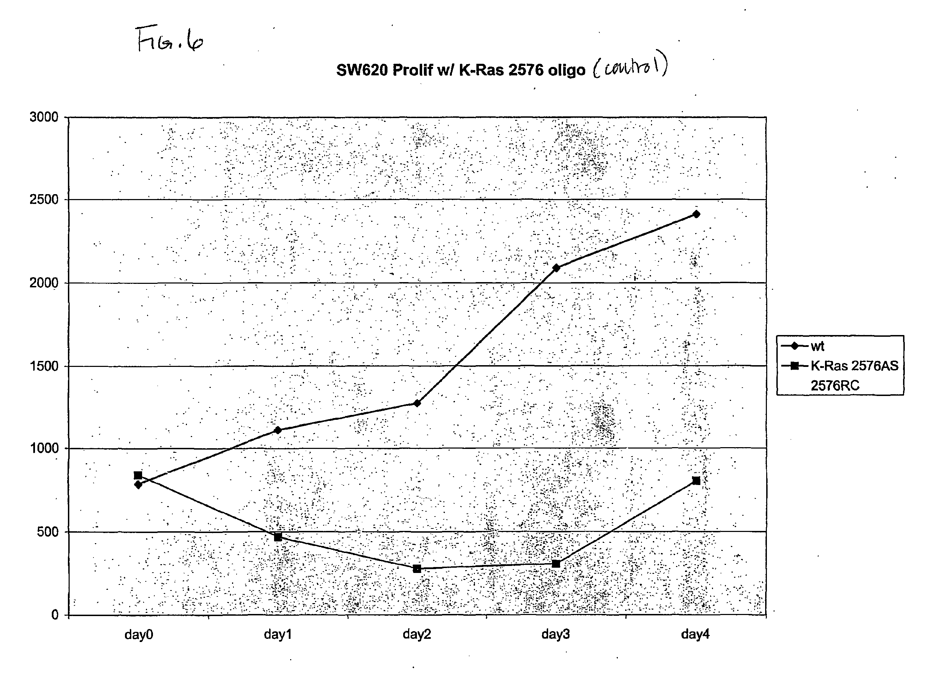

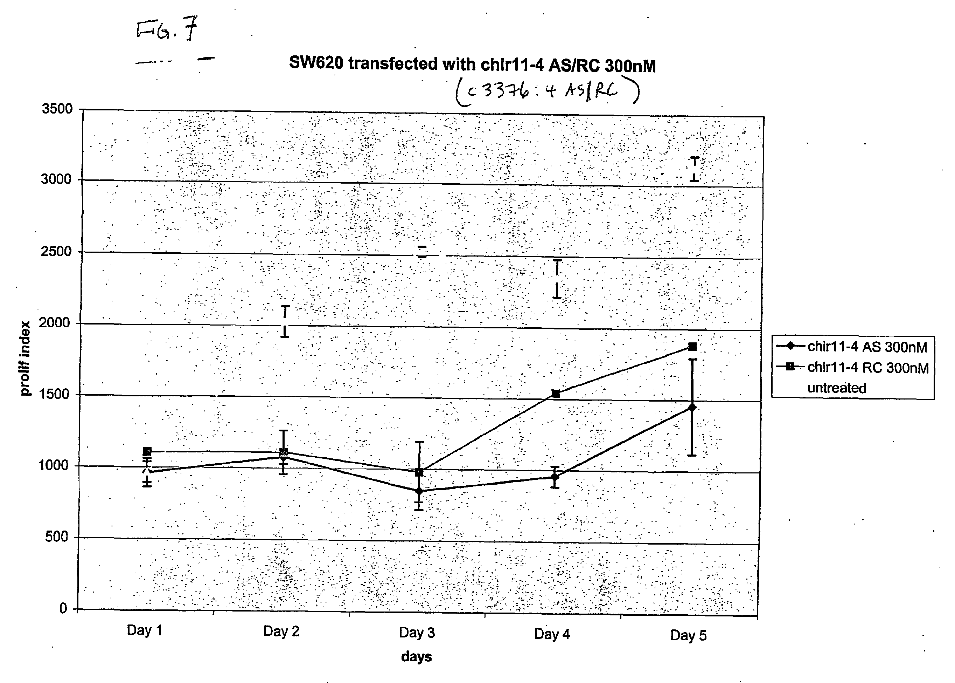

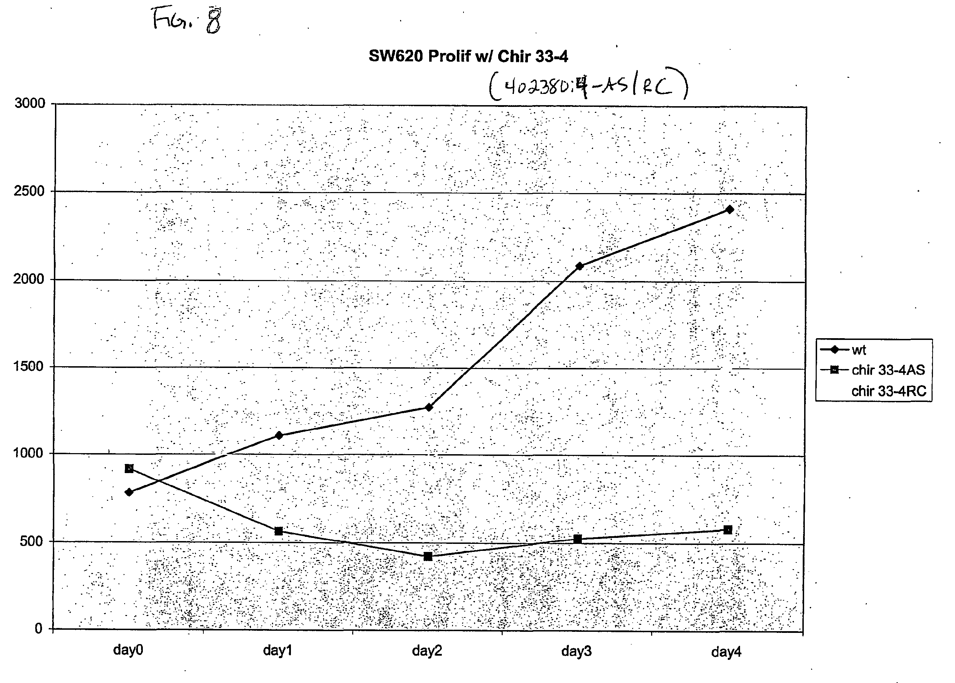

Antisense oligonucleotides were prepared as described above. Cells were transfected overnight at 37°C and the transfection mixture was replaced with fresh medium the next morning. Transfection was carried out as described above in Example 8. Proliferaton was measured using the colormetric reagent WST-1 according to methods well known in the art. The results of the antisense experiments are shown in Figs. 6-9. The values on the y-axis represent relative fluorescent units. Antisense and reverse control oligos to K-Ras served as a control to demonstrate the assay worked as expected (Fig. 6).

### Example 10: Effect of Gene Expression on Colony Formation in Soft Agar

The effect of expression of the gene corresponding to 402380 (gene corresponding to SEQ ID NO: 16) upon colony formation of SW620 cells was tested in a soft agar assay. Soft agar assays were conducted by first establishing a bottom layer of 2 ml of 0.6% agar in media plated fresh within a few hours of layering on the cells. The cell layer was formed on the bottom layer by removing cells transfected as described above from plates using 0.05% trypsin and washing twice in media. The cells were counted in a Coulter counter, and resuspended to 10⁶ per ml in media. 10 µl aliquots were placed with media in 96-well plates (to check counting with WST-1), or diluted further for the soft agar assay. 2000 cells were plated in 800 µl 0.4% agar in duplicate wells above 0.6% agar bottom layer. After the cell layer agar solidified, 2 ml of media was dribbled on top and antisense or reverse control oligo (produced as described above) was added without delivery vehicles. Fresh media and oligos were added every 3-4 days. Colonies formed in 10 days to 3 weeks. Fields of colonies were counted by eye. Wst-1 metabolism values were used to compensate for small differences in starting cell number. Larger fields can be scanned for visual record of differences.

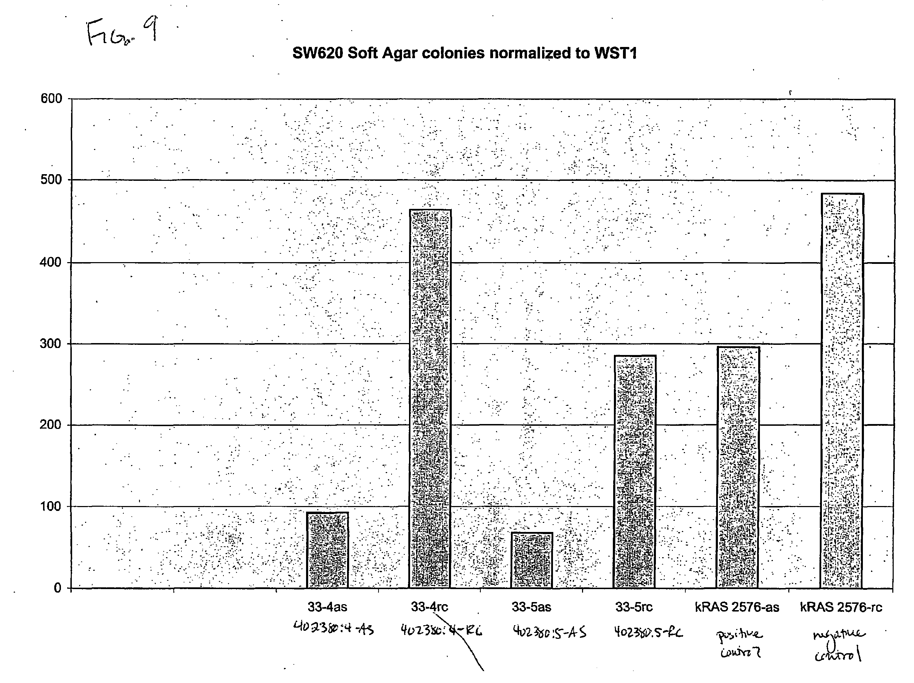

The results are shown in Fig. 9. The y-axis represents the number of cells per a defined sector, using WST-1 to facilitate cell count and normalized to a control. Antisense and reverse control oligos to K-Ras (kRAS 2576-as and kRAS 2576-rc) served as controls to demonstrate the assay worked as expected.

### Example 11: Effect of Gene Expression upon Cell Death

Effect of expression of the genes corresponding to cluster 719 (gene corresponding to SEQ ID NO:1, CHIR-7); cluster 9083 (gene corresponding to SEQ ID NO:3, CHIR-8); cluster 1665 (gene corresponding to SEQ ID NOS:7 and 9, CHIR-9); cluster 3376 (gene corresponding to SEQ ID NO: 13, CHIR 11); cluster 115762 (gene corresponding to SEQ IDNO:5, CHIR 16); and cluster 402380 (gene corresponding to SEQ ID NO:16, CHIR-33) upon cell death in an lactatae dehydrobenase (LDH) cytotoxitity assay was examined in HT1080 cells (a human fibrosarcoma cell line), SW620 cells, and metastatic breast cancer cell lines (MDA-MB-231 ("231 ")) cells. The lactate dehydrogenase (LDH) cytotoxicity assay essentially as follows:

The lactate dehydrogenase (LDH) cytotoxicity assay was performed essentially as follows:
Day 1: Cells were seeded in 4 separate 96 well plates, typically 5000 cells/well and incubated at 37°C and 5% CO₂.
Day 2: Cells were transfected with the anti-sense as well as the reverse complement controls, essentially as described in Example 4. One plate (day 0) was left untransfected as a seeding control.

The transfection was carried out using a lipid vehicle for delivery as described in WO 01/16306, Briefly, the transfection used agents known as "lipitoids"and"cholesteroids", described, for example, in PCT publications WO 01/16306, WO 98/06437 and WO 99/08711.

These lipid-cationic peptoid conjugates are shown in these references to be effective reagents for the delivery of plasmid DNA to cells in vitro. Any of the carriers described in the above-referenced applications are suitable for use in transfection of the oligonucleotides described herein.

These compounds may be prepared by conventional solution or solid-phase synthesis. In one such procedure, as described in WO 99108711, cited above, the N-terminus of a resin-bound peptoid is acylated with a spacer such as Fmocaminohexanoic acid or Fmoc-3-alanine. After removal of the Fmoc group, the primary amino group is reacted with cholesterol chloroformate to form a carbamate linkage. The product is then cleaved from the resin with trifluoroacetic acid and purified by reverse-phase HPLC. A fatty acid-derived lipid moiety, such as a phospholipid, may be used in place of the steroid moiety. The steroid or other lipid moiety may also be linked to the peptoid moiety by other linkages, of any effective length, readily available to the skilled practitioner.

Depending on the cell type, different lipid vehicles were used for different lengths of time for transfection. However, the transfection time did not exceed 24 hrs. The transfection was carried out in complete medium and the final anti-sense oligonucleotide concentration was 300 nM per well. In the wells with drug, the drug was added to the culture at the beginning of the transfection.

Starting on day 3: cells were recovered, 1 plate/day and release of LDH into the supernatant as well as LDH in intact cells was measured using a kit from Roche according to manufacturer's instructions (Roche Diagnostics, Basel, Switzerland) (data labeled as day 1, 2, 3).

For each sample, were analyzed by examining the relative level of released LDH compared to total LDH, wherein an increase as a portion of total LDH signifies increased cell death (due to a higher proportion of released LDH in the media). The data was assessed qualitatively by comparison to an untreated control (no oligo). This assay allowed a determination as to whether antisense-induced loss of message for a particular gene causes death of cells when used alone, or wheter this loss of message sensitizes cells to the effects of a drug.

The results are shown in the table immediately below.

| | **HT1080** | **SW620** | **231** |
|---|---|---|---|
| chir7-2 | negative | negative | |
| chir8-4 | positive | weakly positive | |
| chir9-5 | | positive | |
| chirl 1-2 | | negative | |
| chir 16-4 | | negative | |
| chir33-4 | very weakly positive | strong positive | very weakly positive |

### Example 12: Detection of Differential Expression Using Arrays

mRNA isolated from samples of cancerous and normal colon tissue obtained from patients were analyzed to identify genes differentially expressed in cancerous and normal cells. Normal and cancerous cells collected from cryopreserved patient tissues were isolated using laser capture microdissection (LCM) techniques, which techniques are well known in the art (see, e.g., Ohyama et al. (2000) Biotechniques 29:530-6; Curran et al. (2000) Mol. Pathol. 53:64-8; Suarez-Quian et al. (1999) Biotechniques 26:328-35; Simone et al. (1998) Trends Genet 14:272-6; Conia et al. (1997) J. Clin. Lab. Anal. 11:28-38; Emmert-Buck et al. (1996) Science 274:998-1001).

Table 5 (inserted before the claims) provides information about each patient from which the samples were isolated, including: the "Patient ID" and "Path ReportID", which are numbers assigned to the patient and the pathology reports for identification purposes; the "Group" to which the patients have been assigned; the anatomical location of the tumor ("Anatom Loc"); the "Primary Tumor Size"; the "Primary Tumor Grade"; the identification of the histopathological grade ("Histopath Grade); a description of local sites to which the tumor had invaded ("Local Invasion"); the presence of lymph node metastases ("Lymph Node Met"); the incidence of lymph node metastases (provided as a number of lymph nodes positive for metastasis over the number of lymph nodes examined) ("Incidence Lymphnode Met"); the "Regional Lymphnode Grade"; the identification or detection of metastases to sites distant to the tumor and their location ("Distant Met & Loc"); a description of the distant metastases ("Descrip Distant Met"); the grade of distant metastasis ("Dist Met Grade); and general comments about the patient or the tumor ("Comments"). Adenoma was not described in any of the patients; adenoma dysplasia (described as hyperplasia by the pathologist) was described in Patient ID No. 695. Extranodal extensions were described in two patients, Patient ID Nos. 784 and 791. Lymphovascular invasion was described in seven patients, Patient ID Nos. 128, 278, 517, 534, 784, 786, and 791. Crohn's-like infiltrates were described in seven patients, Patient ID Nos. 52, 264, 268, 392, 393, 784, and 791.

### Identification of differentially expressed genes

cDNA probes were prepared from total RNA isolated from the patient cells described above. Since LCM provides for the isolation of specific cell types to provide a substantially homogenous cell sample, this provided for a similarly pure RNA sample.

Total RNA was first reverse transcribed into cDNA using a primer containing a T7 RNA polymerase promoter, followed by second strand DNA synthesis. cDNA was then transcribed *in vitro* to produce antisense RNA using the T7 promoter-mediated expression (see, e.g., Luo et al. (1999) Nature Med 5:117-122), and the antisense RNA was then converted into cDNA. The second set of cDNAs were again transcribed *in vitro,* using the T7 promoter, to provide antisense RNA. Optionally, the RNA was again converted into cDNA, allowing for up to a third round of T7-mediated amplification to produce more antisense RNA. Thus the procedure provided for two or three rounds of *in vitro* transcription to produce the final RNA used for fluorescent labeling.

Fluorescent probes were generated by first adding control RNA to the antisense RNA mix, and producing fluorescently labeled cDNA from the RNA starting material. Fluorescently labeled cDNAs prepared from the tumor RNA sample were compared to fluorescently labeled cDNAs prepared from normal cell RNA sample. For example, the cDNA probes from the normal cells were labeled with Cy3 fluorescent dye (green) and the cDNA probes prepared from the tumor cells were labeled with Cy5 fluorescent dye (red), and vice versa.

Each array used had an identical spatial layout and control spot set. Each microarray was divided into two areas, each area having an array with, on each half, twelve groupings of 32 x 12 spots, for a total of about 9,216 spots on each array. The two areas are spotted identically which provide for at least two duplicates of each clone per array.

Polynucleotides corresponding to the differentially expressed genes describedherein for use on the arrays were obtained from both publicly available sources and from cDNA libraries generated from selected cell lines and patient tissues. PCR products of from about 0.5kb to 2.0 kb amplified from these sources were spotted onto the array using a Molecular Dynamics Gen III spotter according to the manufacturer's recommendations. The first row of each of the 24 regions on the array had about 32 control spots, including 4 negative control spots and 8 test polynucleotides. The test polynucleotides were spiked into each sample before the labeling reaction with a range of concentrations from 2-600 pg/slide and ratios of 1:1. For each array design, two slides were hybridized with the test samples reverse-labeled in the labeling reaction. This provided for about four duplicate measurements for each clone, two of one color and two of the other, for each sample.

The differential expression assay was performed by mixing equal amounts of probes from tumor cells and normal cells of the same patient. The arrays were prehybridized by incubation for about 2 hrs at 60°C in 5X SSC/0.2% SDS/1 mM EDTA, and then washed three times in water and twice in isopropanol. Following prehybridization of the array, the probe mixture was then hybridized to the array under conditions of high stringency (overnight at 42°C in 50% formamide, 5X SSC, and 0.2% SDS. After hybridization, the array was washed at 55°C three times as follows: 1) first wash in 1X SSC/0.2% SDS; 2) second wash in 0.1X SSC/0.2% SDS; and 3) third wash in 0.1X SSC.

The arrays were then scanned for green and red fluorescence using a Molecular Dynamics Generation III dual color laser-scanner/detector. The images were processed using BioDiscovery Autogene software, and the data from each scan set normalized to provide for a ratio of expression relative to normal. Data from the microarray experiments was analyzed according to the algorithms described in U.S. application serial no. 60/252,358, filed November 20, 2000, by E.J. Moler, M.A. Boyle, and F.M. Randazzo, and entitled "Precision and accuracy in cDNA microarray data.

The experiment was repeated, this time labeling the two probes with the opposite color in order to perform the assay in both "color directions." Each experiment was sometimes repeated with two more slides (one in each color direction). The level fluorescence for each sequence on the array expressed as a ratio of the geometric mean of 8 replicate spots/genes from the four arrays or 4 replicate spots/gene from 2 arrays or some other permutation. The data were normalized using the spiked positive controls present in each duplicated area, and the precision of this normalization was included in the final determination of the significance of each differential. The fluorescent intensity of each spot was also compared to the negative controls in each duplicated area to determine which spots have detected significant expression levels in each sample.

A statistical analysis of the fluorescent intensities was applied to each set of duplicate spots to assess the precision and significance of each differential measurement, resulting in a p-value testing the null hypothesis that there is no differential in the expression level between the tumor and normal samples of each patient. During initial analysis of the microarrays, the hypothesis was accepted if p > 10⁻³, and the differential ratio was set to 1.000 for those spots. All other spots have a significant difference in expression between the tumor and normal sample. If the tumor sample has detectable expression and the normal does not, the ratio is truncated at 1000 since the value for expression in the normal sample would be zero, and the ratio would not be a mathematically useful value (e.g., infinity). If the normal sample has detectable expression and the tumor does not, the ratio is truncated to 0.001, since the value for expression in the tumor sample would be zero and the ratio would not be a mathematically useful value. These latter two situations are referred to herein as "on/off." Database tables were populated using a 95% confidence level (p>0.05).

The results are provided in Table 6 below. The table includes: 1) the SEQ ID NO; 2) the sample identification (Sample ID); 3) the spot identification number ("SpotID"); and 4) the percentage of patients tested in which expression levels of the gene was at least 2-fold greater in cancerous tissue than in matched normal tissue ("ColonPatients pvalcorrected 95 _>=2x"). The ratios of differential expression is expressed as a normalized hybridization signal associated with the tumor probe divided by the normalized hybridization signal with the normal probe. Thus, a ratio greater than 1 indicates that the gene product is increased in expression in cancerous cells relative to normal cells, while a ratio of less than 1 indicates the opposite.

| **Table 6** | | | |
|---|---|---|---|
| **SEQ ID NO** | **SampleID** | **Chip Spot Id** | **ColonPatients pvalcorrected 95 >=2x** |
| **1** | RG:727787:Order7TM31:E07 | 29912 | 82.14 |
| **7** | M00055209C:B07 | 24297 | 30.30 |
| **9** | M00056908A:H05 | 21544 | 42.42 |
| **13** | M00057000D:E08 | 21592 | 30.30 |
| **27** | RG:1418951:Order7TM11:D12 | 33623 | 78.57 |
| **29** | RG:1418951:Order7TM11:D12 | 33623 | 78.57 |
| **22** | M00001346C:A05 | 243 | 55 |
| **22** | M00054893C:D03 | 21952 | 30 |

These data provide evidence that the genes represented by the polynucleotides having the indicated sequences are differentially expressed in colon cancer.

Those skilled in the art will recognize, or be able to ascertain, using not more than routine experimentation, many equivalents to the specific embodiments of the invention described herein. Such specific embodiments and equivalents are intended to be encompassed by the following claims.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

Deposit Information. A deposit of biologically pure cultures of the following viruses was made with the American Type Culture Collection, 10801 University Blvd., Manassa, VA 20110-2209, under the provisions of the Budapest Treaty, on or before the filing date of the present application. The accession number indicated was assigned after successful viability testing, and the requisite fees were paid. Access to said cultures will be available during pendency of the patent application to one determined by the Commissioner to be entitled to such under 37 C.F.R § 1.14 and 35 U.S.C. § 122. All restriction on availability of said cultures to the public will be irrevocably removed upon the granting of a patent based upon the application. Moreover, the designated deposits will be maintained for a period of thirty (30) years from the date of deposit, or for five (5) years after the last request for the deposit; or for the enforceable life of the U.S. patent, whichever is longer. Should a culture become nonviable or be inadvertently destroyed, or, in the case of plasmid-containing strains, lose its plasmid, it will be replaced with a viable culture(s) of the same taxonomic description.

These deposits are provided merely as a convenience to those of skill in the art, and are not an admission that a deposit is required. The nucleic acid sequences of these plasmids, as well as the amino sequences of the polypeptides encoded thereby, are controlling in the event of any conflict with the description herein. A license may be required to make, use, or sell the deposited materials, and no such license is hereby granted.

In addition, pools of selected clones, as well as libraries containing specific clones, were assigned an "ES" number (internal reference) and deposited with the ATCC. Table 7 below provides the ATCC Accession Nos. of the deposited clones, all of which were deposited on or before the filing date of the application.

**Table 7. Pools of Clones and Libraries Deposited with the ATCC**

| **Sequence Name** | **Clones** | **CMCC** | **ATCC** |
|---|---|---|---|
| SK1 | SK-1 | 5162 | PTA-1360 |
| SK2 | SK-2 | 5163 | PTA-1361 |
| SK5 | SK-5 | 5164 | PTA-1362 |
| 1665 short | 1665 short | 5165 | PTA-1363 |
| 1665 long | 1665 long | 5166 | PTA-1363 |
| sk19 | SK-19 | 5167 | PTA-1364 |
| Junc2 | Junc2-6 | 5168 | PTA-1365 |
| XD4 | XD4b | 5169 | PTA-1366 |
| XD1 | XD1b | 5170 | PTA-1367 |
| XD7 | XD7c | 5171 | PTA-1368 |
| XD10 | XD10b | 5172 | PTA-1369 |
| XD11 | XD11b | 5173 | PTA-1370 |
| Junc4 | Junc4-2 | 5174 | PTA-1371 |

CMCC refers to applicant's internal reference number.

Retrieval of Individual Clones from Deposit of Pooled Clones. Where the ATCC deposit is composed of a pool of cDNA clones or a library of cDNA clones, the deposit was prepared by first transfecting each of the clones into separate bacterial cells. The clones in the pool or library were then deposited as a pool of equal mixtures in the composite deposit. Particular clones can be obtained from the composite deposit using methods well known in the art. For example, a bacterial cell containing a particular clone can be identified by isolating single colonies, and identifying colonies containing the specific clone through standard colony hybridization techniques, using an oligonucleotide probe or probes designed to specifically hybridize to a sequence of the clone insert (e.g., a probe based upon unmasked sequence of the encoded polynucleotide having the indicated SEQ ID NO). The probe should be designed to have a Tₘ of approximately 80°C (assuming 2°C for each A or T and 4°C for each G or C). Positive colonies can then be picked, grown in culture, and the recombinant clone isolated. Alternatively, probes designed in this manner can be used to PCR to isolate a nucleic acid molecule from the pooled clones according to methods well known in the art, *e.g.,* by purifying the cDNA from the deposited culture pool, and using the probes in PCR reactions to produce an amplified product having the corresponding desired polynucleotide sequence.

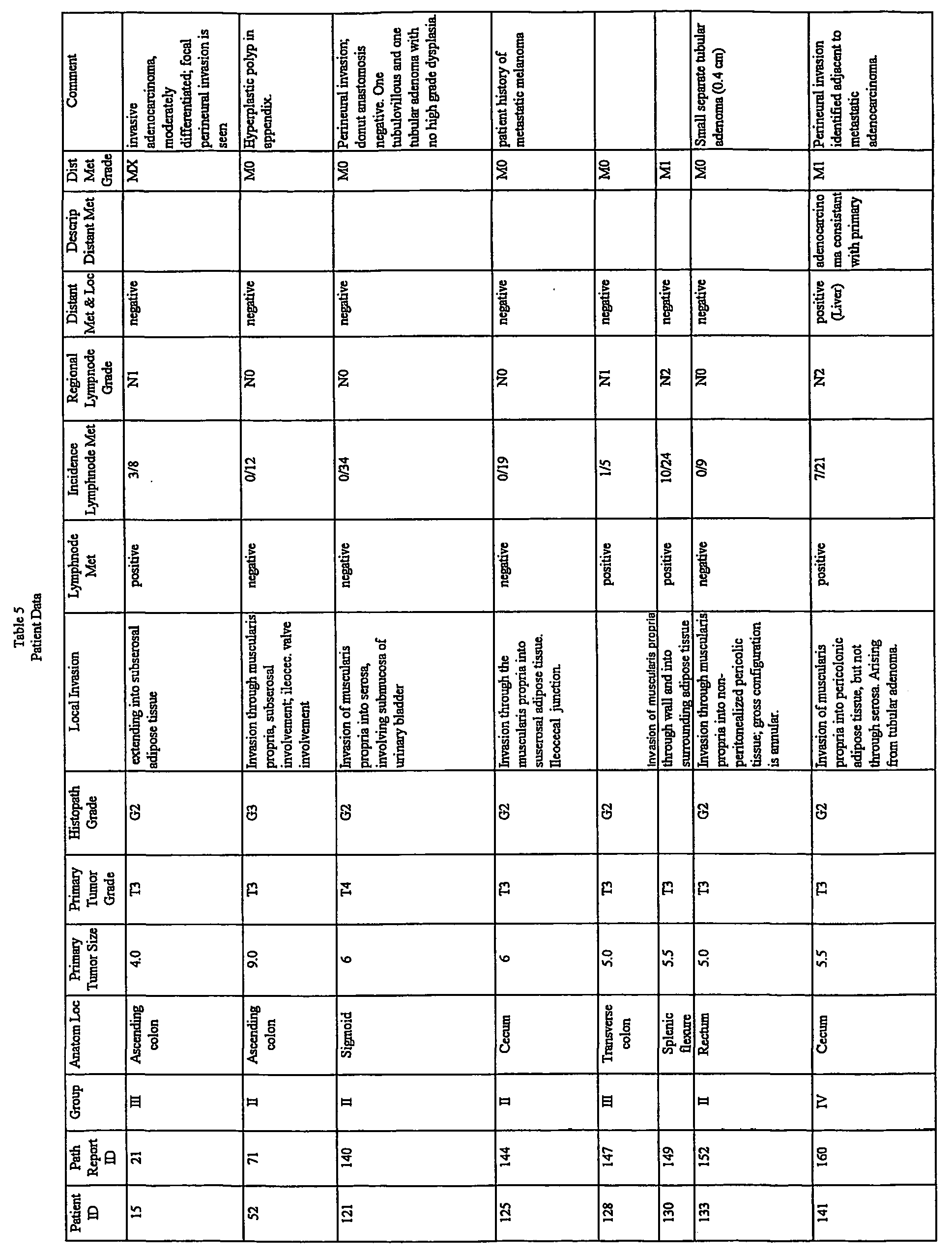

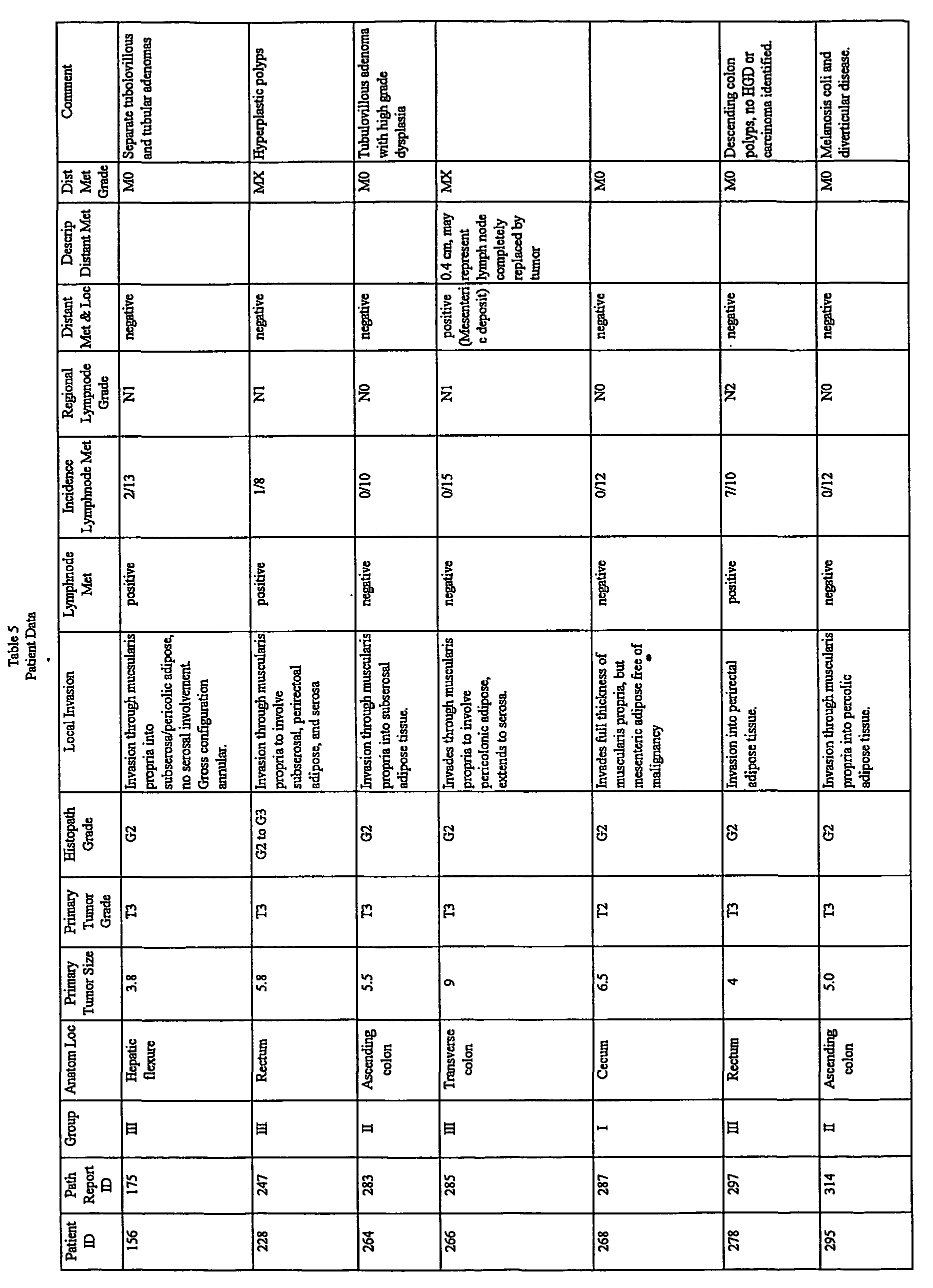

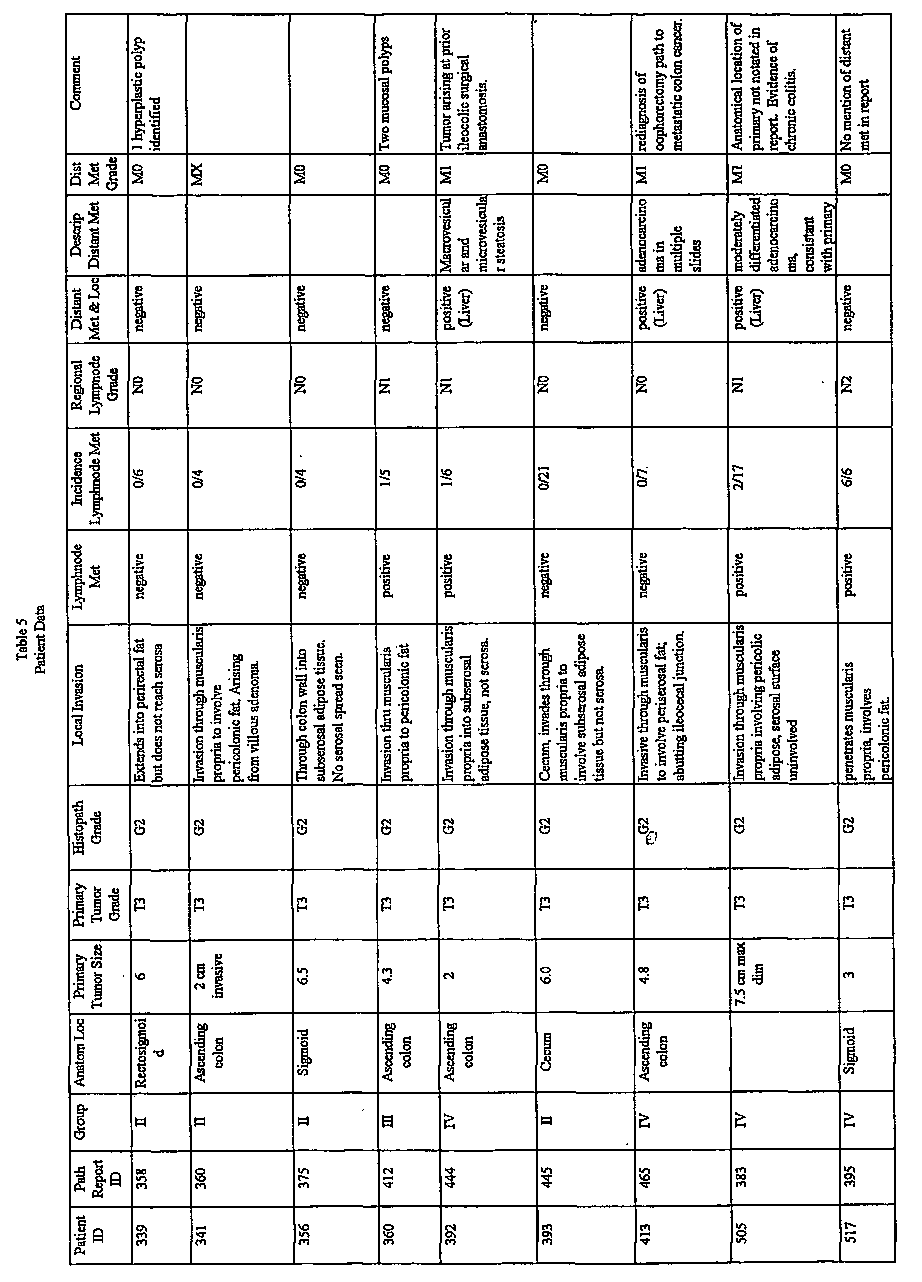

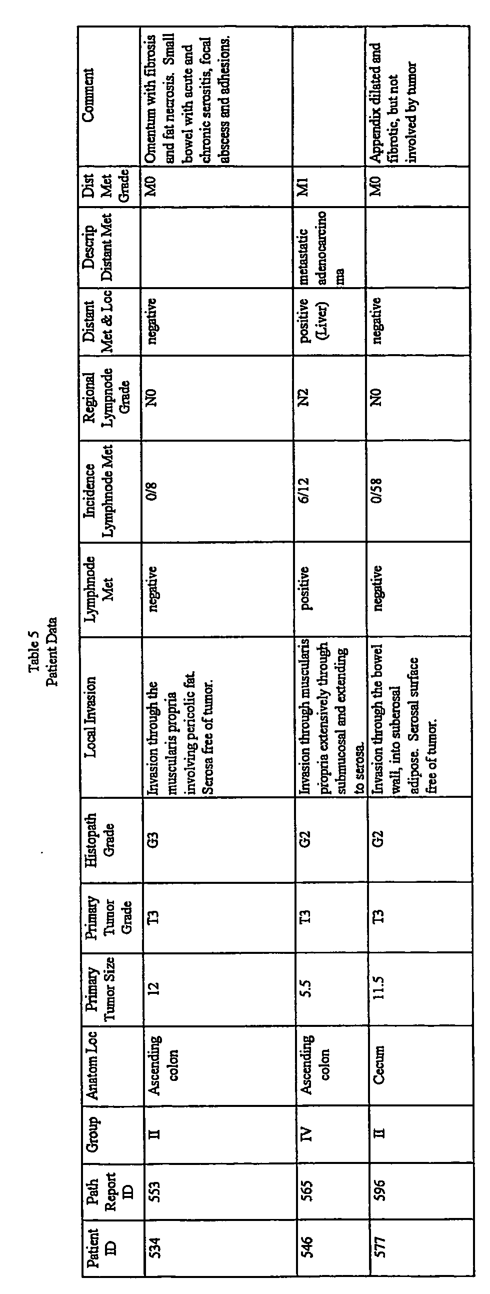

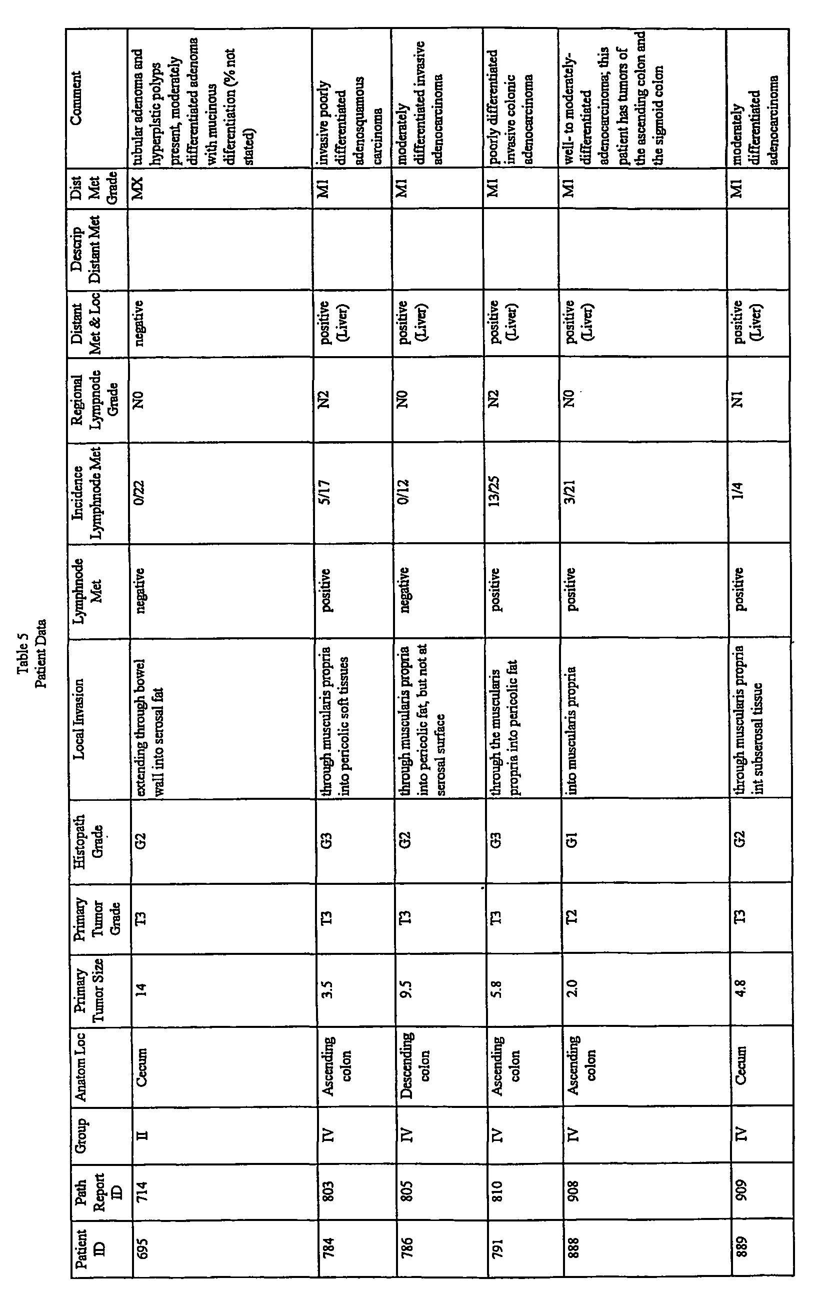

## Claims

1. A method for detecting a cancerous colon cell comprising:
contacting a sample obtained from a test colon cell with a probe for detection of a gene product of a gene differentially expressed in colon cancer, wherein the gene comprises the sequence of SEQ ID NO: 1, said contacting being for a time sufficient for binding of the probe to the gene product; and
comparing a level of the probe binding to the sample with a level of probe binding to a control sample obtained from a control colon cell, wherein the control colon cell is of known cancerous state;
wherein a level of binding of the probe in the test colon cell sample that is similar to the level of binding in a control sample is indicative of the cancerous state of the test colon cell.

2. The method of claim 1, wherein the probe is a polynucleotide probe and the gene product is nucleic acid.

3. The method of claim 1, wherein the gene product is a polypeptide.

4. The method of claim 1, wherein the gene product is immobilized on an array.

5. The method of claim 1, wherein the probe is immobilized on an array.

6. A method of identifying a cancerous colon cell, the method comprising the steps of :
detecting at least one differentially expressed gene product, where the gene product is encoded by a gene comprising the sequence of SEQ ID NO:1 in a test sample, where the test sample is derived from a test cell suspected of being a cancerous colon cell; and
comparing the expression level of the detected differentially expressed gene product with the expression level of the differentially expressed gene product in a control sample, where the control sample is derived from a cancerous colon cell;
wherein detection of the expression level of the differentially expressed gene product in the test sample that is similar to the expression level of the gene product in the control sample indicates that the test cell is a cancerous colon cell.

7. The method of claim 6, wherein said detecting is by hybridization of the test sample to a reference array, wherein the reference array comprises a polynucleotide comprising at least 12 contiguous nucleotides of the sequence of SEQ ID NO :1.

8. The method of claim 6, wherein the gene product detected is a polypeptide.

9. A method of identifying a cancerous colon cell, the method comprising the steps of :
detecting at least one differentially expressed gene product, where the gene product is encoded by a gene comprising the sequence of SEQ ID NO:1 in a test sample, where the test sample is derived from a test cell suspected of being a cancerous colon cell; and
comparing the expression level of the detected differentially expressed gene product with the expression level of the differentially expressed gene product in a control sample, where the control sample is derived from a normal colon cell;
wherein detection of the expression level of the differentially expressed gene product in the test sample that is increased relative to the expression level of the gene product in the control sample indicates that the test cell is a cancerous colon cell.

10. The method of claim 9, wherein detection of the expression level of the differentially expressed gene product in the test sample that is greater than the expression level of the gene product in the control sample indicates that the test cell is a colon tumor cell.

11. The method of claim 9, wherein detection of the expression level of the differentially expressed gene product in the test sample that is greater than the expression level of the gene product in the control sample indicates that the test cell is a metastatic colon tumor cell.

12. A method of identifying a cancerous colon cell, the method comprising the steps of :
detecting at least one differentially expressed gene product, wherein detection is by detecting hybridization of a polynucleotide comprising the sequence of SEQ ID NO: 1 in a test sample, where the test sample is derived from a test cell suspected of being a cancerous colon cell; and
comparing the hybridization level of the detected differentially expressed gene product with the hybridization level of the differentially expressed gene product in a control sample, where the control sample is derived from a cancerous colon cell;
wherein detection of the hybridization level of the differentially expressed gene product in the test sample that is increased relative to the hybridization level of the gene product in the control sample indicates that the test cell is a cancerous colon cell.

13. A method of identifying a cancerous colon cell, the method comprising the steps of:
detecting at least one differentially expressed gene product, wherein detection is by detecting hybridization of a polynucleotide comprising the sequence of SEQ ID NO: 1 in a test sample, where the test sample is derived from a test cell suspected of being a cancerous colon cell; and
comparing the hybridization level of the detected differentially expressed gene product with the hybridization level of the differentially expressed gene product in a control sample, where the control sample is derived from a normal colon cell;
wherein detection of the hybridization level of the differentially expressed gene product in the test sample that is increased relative to the hybridization level of the gene product in the control sample indicates that the test cell is a cancerous colon cell.

14. Use of an antisense polynucleotide for inhibition of expression of a gene comprising the sequence of SEQ ID NO:1 in the manufacture of a medicament for suppressing or inhibiting a cancerous phenotype of a cancerous colon cell.

15. The use of claim 14, wherein the cancerous phenotype is metastasis.

16. The use of claim 14, wherein the cancerous phenotype is aberrant cellular proliferation relative to a normal cell.

17. The use of claim 14, wherein the cancerous phenotype is loss of contact inhibition of cell growth.

18. Use of an antisense polynucleotide for inhibition of production of a gene product encoded by a polynucleotide comprising the sequence of SEQ ID NO:1 or of an antibodythat specifically binds to a polypeptide encoded by a polynucleotide comprising the sequence of SEQ ID NO:1 in the manufacture of a medicament for inhibiting colon tumor growth in a subject having a colon tumor expressing a gene comprising a sequence of SEQ ID NO:1.

19. A method for identifying a gene product as a target for a cancer therapeutic, the method comprising:
contacting a cancerous colon cell expressing a candidate gene product with an anti-cancer agent, wherein the candidate gene product corresponds to the sequence of SEQ ID NO: 1; and
analyzing the effect of the anti-cancer agent upon expression of the candidate gene product and upon a cancerous phenotype of the cancerous cell;
wherein modulation of the biological activity of the candidate gene product and modulation of the cancerous phenotype of the cancerous cell indicates the candidate gene product is a target for a cancer therapeutic.

20. The method of claim 19, wherein the anti-cancer-agent is an antisense oligonucleotide.

21. The method of claim 19, wherein the cancerous phenotype is aberrant cellular proliferation relative to a normal cell.

22. The method of claim 19, wherein the cancerous phenotype is colony formation due to loss of contact inhibition of growth.

23. A method for identifying agents that decrease biological activity of a gene product differentially expressed in a cancerous cell, the method comprising:
contacting a candidate agent with a differentially expressed gene product, wherein the differentially expressed gene product is an mRNA gene product encoded by the sequence of SEQ ID NO: 1, a cDNA gene product prepared from the mRNA gene product, or a polypeptide gene product expressed by the mRNA or cDNA gene product; and
detecting a decrease in a biological activity of the gene product relative to a level of biological activity of the gene product in the absence of the candidate agent.

24. The method of claim 23 wherein said detecting is by detection of a decrease in expression of the differentially expressed gene product.

25. An isolated polynucleotide comprising the nucleotide sequence of SEQ ID NO: 1.

26. An array comprising the polynucleotide of claim 25.

27. An array comprising at least two different polynucleotides, wherein the polynucleotides comprise a sequence having the sequence of SEQ ID NO: 1.

28. A recombinant host cell containing the polynucleotide of claim 25.

29. An isolated polypeptide encoded by the polynucleotide of claim 25.

30. An antibody that specifically binds a polypeptide of claim 29.

31. A polynucleotide comprising the nucleotide sequence of an insert contained in clone SK-1, deposited as ATCC Accession No. PTA-1360.

32. An isolated polynucleotide comprising the sequence encoding the polypeptide of SEQ ID NO: 2.

33. A pharmaceutical composition comprising an antisense polynucleotide for inhibition of production of a gene product encoded by a polynucleotide having a sequence of SEQ ID NO: 1.

34. The pharmaceutical composition of claim 33, wherein the antisense polynucleotide comprises a sequence selected from the group consisting of SEQ ID NOS: 68, 70, 72, and 74.

## Patentansprüche

1. Verfahren zur Erfassung einer karzinomatösen Colonzelle, das umfasst:
Inkontaktbringen einer von einer Test-Colonzelle erhaltenen Probe mit einer Sonde zur Erfassung eines Genprodukts eines Gens, das bei Colonkrebs differentiell exprimiert wird, wobei das Gen die Sequenz des Sequenzprotokolls SEQ ID NO:1 aufweist, und wobei das Inkontaktbringen während einer Zeitdauer erfolgt, die zur Bindung der Sonde an das Genprodukt ausreicht,
und
Vergleichen eines Niveaus der Sonde, die an die Probe bindet, mit einem Niveau der Sonde, die an eine von einer Kontroll-Colonzelle erhaltene Kontrollprobe bindet, wobei sich die Kontroll-Colonzelle in einem bekannten karzinomatösen Zustand befindet,
wobei ein Niveau der Bindung der Sonde in der Probe mit der Test-Colonzelle, das dem Niveau der Bindung in einer Kontrollprobe ähnlich ist, ein Hinweis auf den karzinomatösen Zustand der Test-Colonzelle ist.

2. Verfahren nach Anspruch 1, bei dem die Sonde eine Polynucleotidsonde ist und das Genprodukt eine Nucleinsäure ist.

3. Verfahren nach Anspruch 1, bei dem das Genprodukt ein Polypeptid ist.

4. Verfahren nach Anspruch 1, bei dem das Genprodukt auf einem Array immobilisiert ist.

5. Verfahren nach Anspruch 1, bei dem die Sonde auf einem Array immobilisiert ist.

6. Verfahren zur Identifizierung einer karzinomatösen Colonzelle, wobei das Verfahren folgende Schritte umfasst:
Erfassen mindestens eines differentiell exprimierten Genprodukts in einer Testprobe, wobei das Genprodukt durch ein Gen codiert wird, das die Sequenz des Sequenzprotokolls SEQ ID NO:1 aufweist, und wobei die Testprobe von einer Testzelle stammt, bei der es sich möglicherweise um eine karzinomatöse Colonzelle handelt,
und
Vergleichen des Expressionsniveaus des erfassten differentiell exprimierten Genprodukts mit dem Expressionsniveau des differentiell exprimierten Genprodukts in einer Kontrollprobe,
wobei die Kontrollprobe von einer karzinomatösen Colonzelle stammt,
wobei die Erfassung eines Expressionsniveaus des differentiell exprimierten Genprodukts in der Testprobe, das dem Expressionsniveau des Genprodukts in der Kontrollprobe ähnlich ist, anzeigt, dass die Testzelle eine karzinomatöse Colonzelle ist.

7. Verfahren nach Anspruch 6, bei dem die Erfassung durch Hybridisierung der Testprobe an einem Referenz-Array erfolgt,
wobei das Referenz-Array ein Polynucleotid aufweist, das mindestens 12 zusammenhängende Nucleotide der Sequenz des Sequenzprotokolls SEQ ID NO: aufweist.

8. Verfahren nach Anspruch 6, bei dem das erfasste Genprodukt ein Polypeptid ist.

9. Verfahren zur Identifizierung einer karzinomatösen Colonzelle, wobei das Verfahren folgende Schritte umfasst:
Erfassen mindestens eines differentiell exprimierten Genprodukts in einer Testprobe, wobei das Genprodukt durch ein Gen codiert wird, das die Sequenz des Sequenzprotokolls SEQ ID NO:1 aufweist, und wobei die Testprobe von einer Testzelle stammt, bei der es sich möglicherweise um eine karzinomatöse Colonzelle handelt,
und
Vergleichen des Expressionsniveaus des erfassten differentiell exprimierten Genprodukts mit dem Expressionsniveau des differentiell exprimierten Genprodukts in einer Kontrollprobe,
wobei die Kontrollprobe von einer karzinomatösen Colonzelle stammt,
wobei die Erfassung eines Expressionsniveaus des differentiell exprimierten Genprodukts in der Testprobe, das gegenüber dem Expressionsniveau des Genprodukts in der Kontrollprobe erhöht ist, anzeigt, dass die Testzelle eine karzinomatöse Colonzelle ist.

10. Verfahren nach Anspruch 9, bei dem die Erfassung eines Expressionsniveaus des differentiell exprimierten Genprodukts in der Testprobe, das größer ist als das Expressionsniveau des Genprodukts in der Kontrollprobe, anzeigt, dass die Testzelle eine Colontumorzelle ist.

11. Verfahren nach Anspruch 9, bei dem die Erfassung eines Expressionsniveaus des differentiell exprimierten Genprodukts in der Testprobe, das größer ist als das Expressionsniveau des Genprodukts in der Kontrollprobe, anzeigt, dass die Testzelle eine metastatische Colontumorzelle ist.

12. Verfahren zur Identifizierung einer karzinomatösen Colonzelle, wobei das Verfahren folgende Schritte aufweist:
Erfassen mindestens eines differentiell exprimierten Genprodukts in einer Testprobe, wobei die Erfassung durch Erfassen der Hybridisierung eines Polynucleotids erfolgt, das die Sequenz des Sequenzprotokolls SEQ ID NO:1 aufweist, wobei die Testprobe von einer Testzelle stammt, bei der es sich möglicherweise um eine karzinomatöse Colonzelle handelt, und
Vergleichen des Hybridisierungsniveaus des erfassten differentiell exprimierten Genprodukts mit dem Hybridisierungsniveau des differentiell exprimierten Genprodukts in einer Kontrollprobe, wobei die Kontrollprobe von einer karzinomatösen Colonzelle stammt,
wobei die Erfassung eines Hybridisierungsniveaus des differentiell exprimierten Genprodukts in der Testprobe, das gegenüber dem Hybridisierungsniveau des Genprodukts in der Kontrollprobe erhöht ist, anzeigt, dass die Testzelle eine karzinomatöse Colonzelle ist.

13. Verfahren zur Identifizierung einer karzinomatösen Colonzelle, wobei das Verfahren folgende Schritte aufweist:
Erfassen mindestens eines differentiell exprimierten Genprodukts in einer Testprobe, wobei die Erfassung durch Erfassen der Hybridisierung eines Polynucleotids erfolgt, das die Sequenz des Sequenzprotokolls SEQ ID NO:1 aufweist, wobei die Testprobe von einer Testzelle stammt, bei der es sich möglicherweise um eine karzinomatöse Colonzelle handelt, und
Vergleichen des Hybridisierungsniveaus des erfassten differentiell exprimierten Genprodukts mit dem Hybridisierungsniveau des differentiell exprimierten Genprodukts in einer Kontrollprobe, wobei die Kontrollprobe von einer normalen Colonzelle stammt,
wobei die. Erfassung eines Hybridisierungsniveaus des differentiell exprimierten Genprodukts in der Testprobe, das gegenüber dem Hybridisierungsniveau des Genprodukts in der Kontrollprobe erhöht ist, anzeigt, dass die Testzelle eine karzinomatöse Colonzelle ist.

14. Verwendung eines Antisense-Polynucleotids zur Inhibierung der Expression eines Gens, das die Sequenz des Sequenzprotokolls SEQ ID NO:1 aufweist, bei der Herstellung eines Arzneimittels zur Unterdrückung oder Inhibierung eines karzinomatösen Phänotyps einer karzinomatösen Colonzelle.

15. Verwendung nach Anspruch 14, wobei es sich bei dem karzinomatösen Phänotyp um eine Metastase handelt.

16. Verwendung nach Anspruch 14, wobei der karzinomatöse Phänotyp im Vergleich mit einer normalen Zelle eine anomale Zellproliferation aufweist.

17. Verwendung nach Anspruch 14, wobei es sich bei dem karzinomatösen Phänotyp um einen Verlust der Kontaktinhibierung des Zellwachstums handelt.

18. Verwendung eines Antisense-Polynucleotids zur Inhibierung der Erzeugung eines Genprodukts, das durch ein Polynucleotid codiert wird, das die Sequenz des Sequenzprotokolls SEQ ID NO:1 aufweist, oder eines Antikörpers, der spezifisch an ein Polypeptid bindet, das durch ein Polynucleotid codiert wird, das die Sequenz des Sequenzprotokolls SEQ ID NO:1 aufweist, bei der Herstellung eines Arzneimittels zur Inhibierung des Colontumorwachstums in einem Subjekt mit einem Colontumor, der ein Gen exprimiert, das eine Sequenz des Sequenzprotokolls SEQ ID NO:1 aufweist.

19. Verfahren zur Identifizierung eines Genprodukts als Target für ein Krebstherapeutikum,
wobei das Verfahren folgende Schritte umfasst:
Inkontaktbringen einer karzinomatösen Colonzelle, die ein Kandidaten-Genprodukt exprimiert, mit einem Antikrebsmittel, wobei das Kandidaten-Genprodukt der Sequenz des Sequenzprotokolls SEQ ID NO: entspricht,
und
Analysieren der Wirkung des Antikrebsmittels auf die Expression des Kandidaten-Genprodukts und auf einen karzinomatösen Phänotyp der karzinomatösen Zelle,
wobei die Modulation der biologischen Aktivität des Kandidaten-Genprodukts und die Modulation des karzinomatösen Phänotyps der karzinomatösen Zelle anzeigt, dass das Kandidaten-Genprodukt ein Target für ein Krebstherapeutikum ist.

20. Verfahren nach Anspruch 19, bei dem das Antikrebsmittel ein Antisense-Oligonucleotid ist.

21. Verfahren nach Anspruch 19, bei dem der karzinomatöse Phänotyp im Vergleich mit einer normalen Zelle eine anomale Zellproliferation aufweist.

22. Verfahren nach Anspruch 19, bei dem es sich um bei dem karzinomatösen Phänotyp um Koloniebildung aufgrund von Verlust der Kontaktinhibierung des Wachstums handelt.

23. Verfahren zur Identifizierung von Mitteln, welche die biologische Aktivität eines in einer karzinomatösen Zelle differentiell exprimierten Genprodukts verringern,
wobei das Verfahren folgende Schritte umfasst:
Inkontaktbringen eines Kandidaten-Mittels mit einem differentiell exprimierten Genprodukt, wobei das differentiell exprimierte Genprodukt ein mRNA-Genprodukt, das durch die Sequenz des Sequenzprotokolls SEQ ID NO:1 codiert wird, ein cDNA-Genprodukt, das aus dem mRNA-Genprodukt erzeugt ist, oder ein Polypeptid-Genprodukt ist, das durch das mRNA-Genprodukt oder das cDNA-Genprodukt exprimiert wird,
und
Erfassen einer Abnahme in einer biologischen Aktivität des Genprodukts in Bezug auf ein Niveau der biologischen Aktivität des Genprodukts bei Abwesenheit eines Kandidaten-Mittels.

24. Verfahren nach Anspruch 23, bei dem das Erfassen durch Erfassung einer Abnahme in der Expression des differentiell exprimierten Genprodukts erfolgt.

25. Isoliertes Polynucleotid, das die Nucleotidsequenz des Sequenzprotokolls SEQ ID NO:1 aufweist.

26. Array, welches das Polynucleotid von Anspruch 25 aufweist.

27. Array, das mindestens zwei verschiedene Polynucleotide aufweist, wobei die Polynucleotide eine Sequenz mit der Sequenz des Sequenzprotokolls SEQ ID NO:1 aufweisen.

28. Rekombinante Wirtszelle, die das Polynucleotid von Anspruch 25 enthält.

29. Isoliertes Polypeptid, das durch das Polynucleotid von Anspruch 25 codiert wird.

30. Antikörper, der ein Polypeptid von Anspruch 29 spezifisch bindet.

31. Polynucleotid, das die Nucleotidsequenz eines Inserts aufweist, das im Klon SK-1 enthalten ist, der bei ATCC unter der Hinterlegungsnummer PTA-1360 hinterlegt ist.

32. Isoliertes Polynucleotid, das die Sequenz aufweist, die das Polypeptid der Sequenz des Sequenzprotokolls SEQ ID NO:2 codiert.

33. Pharmazeutische Zusammensetzung, die ein Antisense-Polynucleotid zur Inhibierung der Erzeugung eines Genprodukts enthält, das durch ein Polynucleotid codiert wird, das eine Sequenz des Sequenzprotokolls SEQ ID NO:1 aufweist.

34. Pharmazeutische Zusammensetzung nach Anspruch 33, bei der das Antisense-Polynucleotid eine Sequenz aufweist, die aus der Gruppe ausgewählt ist, die besteht aus den Sequenzprotokollen SEQ ID NO:68, SEQ ID NO:70, SEQ ID NO:72 und SEQ ID NO:74.

## Revendications

1. Procédé de détection d'une cellule cancéreuse du côlon, qui comprend .
◆ la mise en contact d'un échantillon obtenu d'une cellule du côlon testée avec une sonde pour la détection d'un produit génique d'un gène exprimé de manière différentielle dans le cancer du côlon, dans lequel le gène comprend la séquence de SÉQ ID NO : 1, ladite mise en contact ayant lieu pendant une durée suffisante pour la liaison de la sonde au produit génique ; et
◆ la comparaison d'un niveau de la sonde se liant à l'échantillon à un niveau de la sonde se liant à un échantillon de contrôle obtenu d'une cellule du côlon de contrôle, dans lequel l'état cancéreux de la cellule du côlon de contrôle est connu ;
dans lequel un niveau de liaison de la sonde dans l'échantillon de cellule du côlon testé qui est similaire au niveau de liaison dans un échantillon de contrôle indique l'état cancéreux de la cellule du côlon test.

2. Procédé selon la revendication 1, dans lequel la sonde est une sonde polynucléotidique et le produit génique est un acide nucléique.

3. Procédé selon la revendication 1, dans lequel le produit génique est un polypeptide.

4. Procédé selon la revendication 1, dans lequel le produit génique est immobilisé sur une puce.

5. Procédé selon la revendication 1, dans lequel la sonde est immobilisée sur une puce.

6. Procédé d'identification d'une cellule cancéreuse du côlon, le procédé comprenant les étapes consistant à :
◆ détecter au moins un produit génique exprimé de manière différentielle, où le produit génique est codé par un gène comprenant une séquence de SÉQ ID NO : 1, dans un échantillon testé, où l'échantillon testé est dérivé d'une cellule testée que l'on soupçonne d'être une cellule cancéreuse du côlon ; et
◆ comparer le niveau d'expression du produit génique détecté exprimé de manière différentielle au niveau d'expression du produit génique exprimé de manière différentielle dans un échantillon de contrôle, où l'échantillon de contrôle est dérivé d'une cellule cancéreuse du côlon ;
dans lequel la détection du niveau d'expression du produit génique exprimé de manière différentielle dans l'échantillon testé qui est similaire au niveau d'expression du produit génique dans l'échantillon de contrôle indique que la cellule testée est une cellule cancéreuse du côlon.

7. Procédé selon la revendication 6, dans lequel ladite détection est réalisée par hybridation de l'échantillon testé à une puce de référence,
dans lequel la puce de référence comprend une séquence polynucléotidique comprenant au moins 12 nucléotides contigus de la séquence SÉQ ID NO : 1.

8. Procédé selon la revendication 6, dans lequel le produit génique détecté est un polypeptide.

9. Procédé d'identification d'une cellule cancéreuse du côlon, le procédé comprenant les étapes consistant à :
◆ détecter au moins un produit génique exprimé de manière différentielle, où le produit génique est codé par un gène comprenant la séquence de SÉQ ID NO : 1, dans un échantillon testé, où l'échantillon testé est dérivé d'une cellule testée que l'on soupçonne d'être une cellule cancéreuse du côlon ; et
◆ comparer le niveau d'expression du produit génique détecté exprimé de manière différentielle au niveau d'expression du produit génique exprimé de manière différentielle dans un échantillon de contrôle, où l'échantillon de contrôle est dérivé d'une cellule du côlon normale ;
dans lequel la détection du niveau d'expression du produit génique exprimé de manière différentielle dans l'échantillon testé qui plus élevé que le niveau d'expression du produit génique dans l'échantillon de contrôle indique que la cellule testée est une cellule cancéreuse du côlon.

10. Procédé selon la revendication 9, dans lequel la détection du niveau d'expression du produit génique exprimé de manière différentielle dans l'échantillon testé qui est supérieur au niveau d'expression du produit génique dans l'échantillon de contrôle indique que la cellule testée est une cellule de tumeur du côlon.

11. Procédé selon la revendication 9, dans lequel la détection du niveau d'expression du produit génique exprimé de manière différentielle dans l'échantillon testé qui est supérieur au niveau d'expression du produit génique dans l'échantillon de contrôle indique que la cellule testée est une cellule de tumeur métastatique du côlon.

12. Procédé d'identification d'une cellule cancéreuse du côlon, le procédé comprenant les étapes consistant à :
◆ détecter au moins un produit génique exprimé de manière différentielle, dans lequel la détection est réalisée par détection de l'hybridation d'un polynucléotide comprenant la séquence de SÉQ ID NO : 1, dans un échantillon testé, où l'échantillon testé est dérivé d'une cellule testée que l'on soupçonne d'être une cellule cancéreuse du côlon ; et
◆ comparer le niveau d'hybridation du produit génique détecté exprimé de manière différentielle au niveau d'hybridation du produit génique exprimé de manière différentielle dans un échantillon de contrôle, où l'échantillon de contrôle est dérivé d'une cellule cancéreuse du côlon ;
dans lequel la détection du niveau d'hybridation du produit génique exprimé de manière différentielle dans l'échantillon testé qui est plus élevé que le niveau d'hybridation du produit génique dans l'échantillon de contrôle indique que la cellule testée est une cellule cancéreuse du côlon.

13. Procédé d'identification d'une cellule cancéreuse du côlon, le procédé comprenant les étapes consistant à :
◆ détecter au moins un produit génique exprimé de manière différentielle, dans lequel la détection est réalisée par détection de l'hybridation d'un polynucléotide comprenant la séquence de SÉQ ID NO : 1, dans un échantillon testé, où l'échantillon testé est dérivé d'une cellule testée que l'on soupçonne d'être une cellule cancéreuse du côlon ; et
◆ comparer le niveau d'hybridation du produit génique détecté exprimé de manière différentielle au niveau d'hybridation du produit génique exprimé de manière différentielle dans un échantillon de contrôle, où l'échantillon de contrôle est dérivé d'une cellule du côlon normale ;
dans lequel la détection du niveau d'hybridation du produit génique exprimé de manière différentielle dans l'échantillon testé qui est plus élevé que le niveau d'hybridation du produit génique dans l'échantillon de contrôle indique que la cellule testée est une cellule cancéreuse du côlon.

14. Utilisation d'un polynucléotide antisens pour l'inhibition de l'expression d'un gène comprenant la séquence de SÉQ ID NO : 1, dans la fabrication d'un médicament destiné à supprimer ou à inhiber un phénotype cancéreux d'une cellule cancéreuse du côlon.

15. Utilisation selon la revendication 14, dans laquelle le phénotype cancéreux est la métastase.

16. Utilisation selon la revendication 14, dans laquelle le phénotype cancéreux est une prolifération cellulaire aberrante par rapport à une cellule normale.

17. Utilisation selon la revendication 14, dans laquelle le phénotype cancéreux est la perte de l'inhibition de contact de la croissance cellulaire.

18. Utilisation d'un polynucléotide antisens pour l'inhibition de la production d'un produit génique codé par un polynucléotide comprenant la séquence de SÉQ ID NO : 1, ou d'un anticorps qui se lie spécifiquement à un polypeptide codé par un polynucléotide comprenant la séquence de SÉQ ID NO : 1, dans la fabrication d'un médicament destiné à inhiber la croissance d'une tumeur du côlon chez un sujet ayant une tumeur du côlon exprimant un gène comprenant une séquence de SÉQ ID NO : 1.

19. Procédé d'identification d'un produit génique en tant qu'une cible d'un produit thérapeutique contre le cancer, le procédé comprenant :
la mise en contact d'une cellule cancéreuse du côlon exprimant un produit génique candidat avec un agent anticancéreux, dans lequel le produit génique candidat correspond à la séquence de SÉQ ID NO : 1 ; et
◆ l'analyse de l'effet de l'agent anticancéreux sur l'expression du produit génique candidat et sur un phénotype cancéreux de la cellule cancéreuse ;
dans lequel la modulation de l'activité biologique du produit génique candidat et la modulation du phénotype cancéreux de la cellule cancéreuse indiquent que le produit génique candidat est une cible pour un produit thérapeutique contre le cancer.

20. Procédé selon la revendication 19, dans lequel l'agent anticancéreux est un oligo-nucléotide antisens.

21. Procédé selon la revendication 19, dans lequel le phénotype cancéreux est une prolifération cellulaire aberrante par rapport à une cellule normale.

22. Procédé selon la revendication 19, dans lequel le phénotype cancéreux est la formation de colonies en raison de la perte d'inhibition de contact de la croissance.

23. Procédé d'identification d'agents qui font diminuer l'activité biologique d'un produit génique exprimé de manière différentielle dans une cellule cancéreuse, le procédé comprenant :
◆ la mise en contact d'un agent candidat avec un produit génique exprimé de manière différentielle, dans lequel le produit génique exprimé de manière différentielle est un produit génique d'ARNm codé par la séquence de SÉQ ID NO : 1, un produit génique d'ADNc préparé à partir du produit génique d'ARNm, ou un produit génique polypeptidique exprimé par le produit génique d'ARNm ou d'ADNc, et
◆ la détection d'une diminution d'une activité biologique du produit génique par rapport à un niveau d'activité biologique du produit génique en l'absence de l'agent candidat.

24. Procédé selon la revendication 23, dans lequel ladite détection est réalisée par détection d'une diminution de l'expression du produit génique exprimé de manière différentielle.

25. Polynucléotide isolé comprenant la séquence de nucléotides de SÉQ ID NO : 1.

26. Puce comprenant le polynucléotide selon la revendication 25.

27. Puce comprenant au moins deux polynucléotides différents, dans laquelle les polynucléotides comprennent une séquence ayant la séquence de SÉQ ID NO: 1.

28. Cellule hôte recombinante contenant le polynucléotide selon la revendication 25.

29. Polypeptide isolé codé par le polynucléotide selon la revendication 25.

30. Anticorps qui se lie spécifiquement à un polypeptide selon la revendication 29.

31. Polynucléotide comprenant la séquence de nucléotides d'un insert contenu dans un clone SK-1, déposé sous le n° d'accès ATCC PTA-1360.

32. Polynucléotide isolé comprenant la séquence codant pour le polypeptide de SÉQ ID NO : 2.

33. Composition pharmaceutique comprenant un polynucléotide antisens pour l'inhibition de la production d'un produit génique codé par un polynucléotide ayant une séquence de SÉQ ID NO : 1.

34. Composition pharmaceutique selon la revendication 33, dans laquelle le polynucléotide antisens comprend la séquence choisie dans le groupe constitué par SÉQ ID NO : 68, 70, 72, et 74.


### SEQUENCE LISTING

<110> Kennedy, Giulia C.
   Kang, Sanmao
   Reinhard, Christoph
   Jefferson, Anne Bennett
<120> POLYNUCLEOTIDES RELATED TO COLON CANCER
<130> PP-1663.003
<140> Unassigned
   <141> 2001-06-15
<150> 60/211,835
   <151> 2000-06-15
<160> 127
<170> FastSEQ for Windows Version 4.0
<210> 1
   <211> 564
   <212> DNA
   <213> Homo sapiens
<220>
   <221> CDS
   <222> (21)...(396)
<400> 1
   ggcgagattt gtgcggcgac atg aaa ctg ctt acc cac aat ctg ctg agc tcg      53
                         Met Lys Leu Leu Thr His Asn Leu Leu Ser Ser
                          1               5                  10
   cat gtg cgg ggg gtg ggg tcc cgt ggc ttc ccc ctg cgc ctc cag gcc       101
   His Val Arg Gly Val Gly Ser Arg Gly Phe Pro Leu Arg Leu Gln Ala
                15                  20                  25
   acc gag gtc cgt atc tgc cct gtg gaa ttc aac ccc aac ttc gtg gcg       149
   Thr Glu Val Arg Ile Cys Pro Val Glu Phe Asn Pro Asn Phe Val Ala
            30                  35                  40
   cgt atg ata cct aaa gtg gag tgg tcg gcg ttc ctg gag gcg gcc gat       197
   Arg Met Ile Pro Lys Val Glu Trp Ser Ala Phe Leu Glu Ala Ala Asp
        45                  50                  55
   aac ttg cgt ctg atc cag gtg ccg aaa ggg ccg gtt gag gga tat gag       245
   Asn Leu Arg Leu Ile Gln Val Pro Lys Gly Pro Val Glu Gly Tyr Glu
    60                  65                  70                  75
   gag aat gag gag ttt ctg agg acc atg cac cac ctg ctg ctg gag gtg       293
   Glu Asn Glu Glu Phe Leu Arg Thr Met His His Leu Leu Leu Glu Val
                    80                  85                  90
   gaa gtg ata gag ggc acc ctg cag tgc ccg gaa tct gga cgt atg ttc       341
   Glu Val Ile Glu Gly Thr Leu Gln Cys Pro Glu Ser Gly Arg Met Phe
                95                 100                 105
   ccc atc agc cgc ggg atc ccc aac atg ctg ctg agt gaa gag gaa act       389
   Pro Ile Ser Arg Gly Ile Pro Asn Met Leu Leu Ser Glu Glu Glu Thr
           110                 115                 120
   gag agt t gattgtgcca ggcgccagtt tttcttgtta tgactgtgta tttttgttga     446
   Glu Ser
       125
   tctataccct gtttccgaat tctgccgtgt gtatccccaa cccttgaccc aatgacacca     506
   aacacagtgt ttttgagctc ggtattatat atttttttct cattaaaggt ttaaaacc       564
<210> 2
   <211> 125
   <212> PRT
   <213> Homo sapiens
<400> 2
   Met Lys Leu Leu Thr His Asn Leu Leu Ser Ser His Val Arg Gly Val
    1               5                  10                  15
   Gly Ser Arg Gly Phe Pro Leu Arg Leu Gln Ala Thr Glu Val Arg Ile
               20                  25                  30
   Cys Pro Val Glu Phe Asn Pro Asn Phe Val Ala Arg Met Ile Pro Lys
           35                  40                  45
   Val Glu Trp Ser Ala Phe Leu Glu Ala Ala Asp Asn Leu Arg Leu Ile
       50                  55                  60
   Gln Val Pro Lys Gly Pro Val Glu Gly Tyr Glu Glu Asn Glu Glu Phe
   65                  70                  75                  80
   Leu Arg Thr Met His His Leu Leu Leu Glu Val Glu Val Ile Glu Gly
                   85                  90                  95
   Thr Leu Gln Cys Pro Glu Ser Gly Arg Met Phe Pro Ile Ser Arg Gly
               100                 105                 110
   Ile Pro Asn Met Leu Leu Ser Glu Glu Glu Thr Glu Ser
           115                 120                 125
<210> 3
   <211> 919
   <212> DNA
   <213> Homo sapiens
<220>
   <221> CDS
   <222> (219)...(693)
<400> 3
   tggcacgagg tggcacgagg gtccgggtcg ttgaggatta ggctgctcgg gcgtaaccgg    60
   agctggggcg cggctgccaa gggccggccc ggaagtccca gcggtcttta aattctcccg   120
   tgctagggcc agcctgcgca ttcttacctg tcggggtgcg gcgagtgtct cacctctctg   180
   cacttccaag gactcttgtc atctgcctta ggcgggaa atg ctg ttg ctg gat tgc   236
                                             Met Leu Leu Leu Asp Cys
                                             1               5
   aac ccc gag gtg gat ggt ctg aag cat ttg ctg gag aca ggg gcc tcg     284
   Asn Pro Glu Val Asp Gly Leu Lys His Leu Leu Glu Thr Gly Ala Ser
               10                  15                  20
   gtc aac gca ccc ccg gat ccc tgc aag cag tcg cct gtc cac tta gcc     332
   Val Asn Ala Pro Pro Asp Pro Cys Lys Gln Ser Pro Val His Leu Ala
           25                  30                  35
   gca gga agc ggc ctt gct tgc ttt ctt ctc tgg cag ctg caa acg ggc     380
   Ala Gly Ser Gly Leu Ala Cys Phe Leu Leu Trp Gln Leu Gln Thr Gly
       40                  45                  50
   gct gac ctc aac cag cag gat gtt tta gga gaa gct cca cta cac aag     428
   Ala Asp Leu Asn Gln Gln Asp Val Leu Gly Glu Ala Pro Leu His Lys
   55                  60                  65                  70
   gca gca aaa gtt gga agc ctg gag tgc cta agc ctg ctt gta gcc agt     476
   Ala Ala Lys Val Gly Ser Leu Glu Cys Leu Ser Leu Leu Val Ala Ser
                   75                  80                  85
   gat gcc caa att gat tta tgt aat aag aac ggg caa aca gct gaa gat     524
   Asp Ala Gln Ile Asp Leu Cys Asn Lys Asn Gly Gln Thr Ala Glu Asp
               90                  95                  100
   ctc gct tgg tca tgt gga ttt cca gac tgt gcc aag ttt ctt aca aca     572
   Leu Ala Trp Ser Cys Gly Phe Pro Asp Cys Ala Lys Phe Leu Thr Thr
           105                 110                 115
   att aaa tgt atg cag aca ata aaa gca agt gaa cac cct gac agg aat     620
   Ile Lys Cys Met Gln Thr Ile Lys Ala Ser Glu His Pro Asp Arg Asn
       120                 125                 130
   gat tgt gtt gcc gtg ctc aga cag aaa cgg agt ctc gga agt gta gaa     668
   Asp Cys Val Ala Val Leu Arg Gln Lys Arg Ser Leu Gly Ser Val Glu
   135                 140                 145                 150
   aat acc agt ggg aaa agg aag tgc t gatgtcacgt gggttatgaa             713
   Asn Thr Ser Gly Lys Arg Lys Cys
                   155
   gaagtctgaa gaacgccttc atttcatgca aatctataag ctcctgcttt tggctttacc   773
   atatgttgtg tctaatctcc ttctgagaag gacgaaaaac tttcttccaa gtgaagatcc   833
   atttaagaac acatgtattt acatgcctat aatatgctgg ttgtgtatgc tttgtctttt   893
   aagttattaa aggaacgtct aaaaaa                                        919
<210> 4
   <211> 158
   <212> PRT
   <213> Homo sapiens
<400> 4
   Met Leu Leu Leu Asp Cys Asn Pro Glu Val Asp Gly Leu Lys His Leu
    1               5                  10                  15
   Leu Glu Thr Gly Ala Ser Val Asn Ala Pro Pro Asp Pro Cys Lys Gln
               20                  25                  30
   Ser Pro Val His Leu Ala Ala Gly Ser Gly Leu Ala Cys Phe Leu Leu
           35                  40                  45
   Trp Gln Leu Gln Thr Gly Ala Asp Leu Asn Gln Gln Asp Val Leu Gly
       50                  55                  60
   Glu Ala Pro Leu His Lys Ala Ala Lys Val Gly Ser Leu Glu Cys Leu
   65                  70                  75                  80
   Ser Leu Leu Val Ala Ser Asp Ala Gln Ile Asp Leu Cys Asn Lys Asn
                   85                  90                  95
   Gly Gln Thr Ala Glu Asp Leu Ala Trp Ser Cys Gly Phe Pro Asp Cys
               100                 105                 110
   Ala Lys Phe Leu Thr Thr Ile Lys Cys Met Gln Thr Ile Lys Ala Ser
           115                 120                 125
   Glu His Pro Asp Arg Asn Asp Cys Val Ala Val Leu Arg Gln Lys Arg
       130                 135                 140
   Ser Leu Gly Ser Val Glu Asn Thr Ser Gly Lys Arg Lys Cys
   145                 150                 155
<210> 5
   <211> 1949
   <212> DNA
   <213> Homo sapiens
<220>
   <221> CDS
   <222> (5)...(1760)
<400> 5
   caac atg gcg ccg tcc acg ccg ctc ttg aca gtc cga gga tca gaa gga 49
   Met Ala Pro Ser Thr Pro Leu Leu Thr Val Arg Gly Ser Glu Gly
   1 5 10 15
   ctg tac atg gtg aat gga cca cca cat ttt aca gaa agc aca gtg ttt 97
   Leu Tyr Met Val Asn Gly Pro Pro His Phe Thr Glu Ser Thr Val Phe
   20 25 30
   cca agg gaa tct ggg aag aat tgc aaa gtc tgt atc ttt agt aag gat 145
   Pro Arg Glu Ser Gly Lys Asn Cys Lys Val Cys Ile Phe Ser Lys Asp
   35 40 45
   ggg acc ttg ttt gcc tgg ggc aat gga gaa aaa gta aat att atc agt 193
   Gly Thr Leu Phe Ala Trp Gly Asn Gly Glu Lys Val Asn Ile Ile Ser
   50 55 60
   gtc act aac aag gga cta ctg cac tcc ttc gac ctc ctg aag gca gtt 241
   Val Thr Asn Lys Gly Leu Leu His Ser Phe Asp Leu Leu Lys Ala Val
   65 70 75
   tgc ctt gaa ttc tca ccc aaa aat act gtc ctg gca acg tgg cag cct 289
   Cys Leu Glu Phe Ser Pro Lys Asn Thr Val Leu Ala Thr Trp Gln Pro
   80 85 90 95
   tac act act tct aaa gat ggc aca gct ggg ata ccc aac cta caa ctt 337
   Tyr Thr Thr Ser Lys Asp Gly Thr Ala Gly Ile Pro Asn Leu Gln Leu
   100 105 110
   tat gat gtg aaa act ggg aca tgt ttg aaa tct ttc atc cag aaa aaa 385
   Tyr Asp Val Lys Thr Gly Thr Cys Leu Lys Ser Phe Ile Gln Lys Lys
   115 120 125
   atg caa aat tgg tgt cca tcc tgg tca gaa gat gaa act ctt tgt gcc 433
   Met Gln Asn Trp Cys Pro Ser Trp Ser Glu Asp Glu Thr Leu Cys Ala
   130 135 140
   cgc aat gtt aac aat gaa gtt cac ttc ttt gaa aac aac aat ttt aac 481
   Arg Asn Val Asn Asn Glu Val His Phe Phe Glu Asn Asn Asn Phe Asn
   145 150 155
   aca att gca aat aaa ttg cat ttg caa aaa att aat gac ttt gta tta 529
   Thr Ile Ala Asn Lys Leu His Leu Gln Lys Ile Asn Asp Phe Val Leu
   160 165 170 175
   tca cct gga ccc caa cca tac aag gtg gct gtc tat gtt cca gga agt 577
   Ser Pro Gly Pro Gln Pro Tyr Lys Val Ala Val Tyr Val Pro Gly Ser
   180 185 190
   aaa ggt gca cct tca ttt gtt aga tta tat cag tac ccc aac ttt gct 625
   Lys Gly Ala Pro Ser Phe Val Arg Leu Tyr Gln Tyr Pro Asn Phe Ala
   195 200 205
   gga cct cat gca gct tta gct aat aaa agt ttc ttt aag gca gat aaa 673
   Gly Pro His Ala Ala Leu Ala Asn Lys Ser Phe Phe Lys Ala Asp Lys
   210 215 220
   gtt aca atg ctg tgg aat aaa aaa gct act gct gtg ttg gta ata gct 721
   Val Thr Met Leu Trp Asn Lys Lys Ala Thr Ala Val Leu Val Ile Ala
   225 230 235
   agc aca gat gtt gac aag aca gga gct tcc tac tat gga gaa caa act      769
   Ser Thr Asp Val Asp Lys Thr Gly Ala Ser Tyr Tyr Gly Glu Gln Thr
   240                 245                 250                 255
   cta cac tac att gca aca aat gga gaa agt gct gta gtg caa tta cca      817
   Leu His Tyr Ile Ala Thr Asn Gly Glu Ser Ala Val Val Gln Leu Pro
                   260                 265                 270
   aaa aat ggc ccc att tat gat gta gtt tgg aat tct agt tct act gag      865
   Lys Asn Gly Pro Ile Tyr Asp Val Val Trp Asn Ser Ser Ser Thr Glu
               275                 280                 285
   ttt tgt gct gta tat ggt ttt atg cct gcc aaa gcg aca att ttc aac      913
   Phe Cys Ala Val Tyr Gly Phe Met Pro Ala Lys Ala Thr Ile Phe Asn
           290                 295                 300
   ttg aaa tgt gat cct gta ttt gac ttt gga act ggt cct cgt aat gca      961
   Leu Lys Cys Asp Pro Val Phe Asp Phe Gly Thr Gly Pro Arg Asn Ala
       305                 310                 315
   gcc tac tat agc cct cat gga cat ata tta gta tta gct gga ttt gga     1009
   Ala Tyr Tyr Ser Pro His Gly His Ile Leu Val Leu Ala Gly Phe Gly
   320                 325                 330                 335
   aat ctg agg gga caa atg gaa gtg tgg gat gtg aaa aac tac aaa ctt     1057
   Asn Leu Arg Gly Gln Met Glu Val Trp Asp Val Lys Asn Tyr Lys Leu
                   340                 345                 350
   att tct aaa ccg gtg gct tct gat tct aca tat ttt gct tgg tgc ccg     1105
   Ile Ser Lys Pro Val Ala Ser Asp Ser Thr Tyr Phe Ala Trp Cys Pro
               355                 360                 365
   gat ggt gag cat att tta aca gct aca tgt gct ccc agg tta cgg gtt     1153
   Asp Gly Glu His Ile Leu Thr Ala Thr Cys Ala Pro Arg Leu Arg Val
           370                 375                 380
   aat aat gga tac aaa att tgg cat tat act ggc tct atc ttg cac aag     1201
   Asn Asn Gly Tyr Lys Ile Trp His Tyr Thr Gly Ser Ile Leu His Lys
       385                 390                 395
   tat gat gtg cca tca aat gca gaa tta tgg cag gtt tct tgg cag cca     1249
   Tyr Asp Val Pro Ser Asn Ala Glu Leu Trp Gln Val Ser Trp Gln Pro
   400                 405                 410                 415
   ttt ttg gat gga ata ttt cca gca aaa aca ata act tac caa gca gtt     1297
   Phe Leu Asp Gly Ile Phe Pro Ala Lys Thr Ile Thr Tyr Gln Ala Val
                   420                 425                 430
   cca agt gaa gta ccc aat gag gaa cct aaa gtt gca aca gct tat aga     1345
   Pro Ser Glu Val Pro Asn Glu Glu Pro Lys Val Ala Thr Ala Tyr Arg
               435                 440                 445
   ccc cca gct tta aga aat aaa cca atc acc aat tcc aaa ttg cat gaa     1393
   Pro Pro Ala Leu Arg Asn Lys Pro Ile Thr Asn Ser Lys Leu His Glu
           450                 455                 460
   gag gaa cca cct cag aat atg aaa cca caa tca gga aac gat aag cca     1441
   Glu Glu Pro Pro Gln Asn Met Lys Pro Gln Ser Gly Asn Asp Lys Pro
       465                 470                 475
   tta tca aaa aca gct ctt aaa aat caa agg aag cat gaa gct aag aaa     1489
   Leu Ser Lys Thr Ala Leu Lys Asn Gln Arg Lys His Glu Ala Lys Lys
   480                 485                 490                 495
   gct gca aag cag gaa gca aga agt gac aag agt cca gat ttg gca cct      1537
   Ala Ala Lys Gln Glu Ala Arg Ser Asp Lys Ser Pro Asp Leu Ala Pro
                   500                 505                 510
   act cct gcc cca cag agc aca cca cga aac act gtc tct cag tca att      1585
   Thr Pro Ala Pro Gln Ser Thr Pro Arg Asn Thr Val Ser Gln Ser Ile
               515                 520                 525
   tct ggg gac cct gag ata gac aaa aaa atc aag aac cta aag aag aaa      1633
   Ser Gly Asp Pro Glu Ile Asp Lys Lys Ile Lys Asn Leu Lys Lys Lys
           530                 535                 540
   ctg aaa gca atc gaa caa ctg aaa gaa caa gca gca act gga aaa cag      1681
   Leu Lys Ala Ile Glu Gln Leu Lys Glu Gln Ala Ala Thr Gly Lys Gln
       545                 550                 555
   cta gaa aaa aat cag ttg gag aaa att cag aaa gaa aca gcc ctt ctc      1729
   Leu Glu Lys Asn Gln Leu Glu Lys Ile Gln Lys Glu Thr Ala Leu Leu
   560                 565                 570                 575
   cag gag ctg gaa gat ttg gaa ttg ggt att t aaagattcac ggaaagcaag      1780
   Gln Glu Leu Glu Asp Leu Glu Leu Gly Ile
                   580                 585
   ttgatgacca gaaatcagtg caaacacatc ttctgttaaa cccattggta tacacagaat    1840
   attcctgtgc ccacacttaa tgtcaatcta taattttaac catttatcca agattctact    1900
   aagtgtaaaa ttatttaata atgtctatta aattgatatt tatatcttg                1949
<210> 6
   <211> 585
   <212> PRT
   <213> Homo sapiens
<400> 6
   Met Ala Pro Ser Thr Pro Leu Leu Thr Val Arg Gly Ser Glu Gly Leu
    1               5                  10                  15
   Tyr Met Val Asn Gly Pro Pro His Phe Thr Glu Ser Thr Val Phe Pro
               20                  25                  30
   Arg Glu Ser Gly Lys Asn Cys Lys Val Cys Ile Phe Ser Lys Asp Gly
           35                  40                  45
   Thr Leu Phe Ala Trp Gly Asn Gly Glu Lys Val Asn Ile Ile Ser Val
       50                  55                  60
   Thr Asn Lys Gly Leu Leu His Ser Phe Asp Leu Leu Lys Ala Val Cys
   65                  70                  75                  80
   Leu Glu Phe Ser Pro Lys Asn Thr Val Leu Ala Thr Trp Gln Pro Tyr
                   85                  90                  95
   Thr Thr Ser Lys Asp Gly Thr Ala Gly Ile Pro Asn Leu Gln Leu Tyr
               100                 105                 110
   Asp Val Lys Thr Gly Thr Cys Leu Lys Ser Phe Ile Gln Lys Lys Met
           115                 120                 125
   Gln Asn Trp Cys Pro Ser Trp Ser Glu Asp Glu Thr Leu Cys Ala Arg
       130                 135                 140
   Asn Val Asn Asn Glu Val His Phe Phe Glu Asn Asn Asn Phe Asn Thr
   145                 150                 155                 160
   Ile Ala Asn Lys Leu His Leu Gln Lys Ile Asn Asp Phe Val Leu Ser
                   165                 170                 175
   Pro Gly Pro Gln Pro Tyr Lys Val Ala Val Tyr Val Pro Gly Ser Lys
               180                 185                 190
   Gly Ala Pro Ser Phe Val Arg Leu Tyr Gln Tyr Pro Asn Phe Ala Gly
           195                 200                 205
   Pro His Ala Ala Leu Ala Asn Lys Ser Phe Phe Lys Ala Asp Lys Val
       210                 215                 220
   Thr Met Leu Trp Asn Lys Lys Ala Thr Ala Val Leu Val Ile Ala Ser
   225                 230                 235                 240
   Thr Asp Val Asp Lys Thr Gly Ala Ser Tyr Tyr Gly Glu Gln Thr Leu
                   245                 250                 255
   His Tyr Ile Ala Thr Asn Gly Glu Ser Ala Val Val Gln Leu Pro Lys
               260                 265                 270
   Asn Gly Pro Ile Tyr Asp Val Val Trp Asn Ser Ser Ser Thr Glu Phe
           275                 280                 285
   Cys Ala Val Tyr Gly Phe Met Pro Ala Lys Ala Thr Ile Phe Asn Leu
       290                 295                 300
   Lys Cys Asp Pro Val Phe Asp Phe Gly Thr Gly Pro Arg Asn Ala Ala
   305                 310                 315                 320
   Tyr Tyr Ser Pro His Gly His Ile Leu Val Leu Ala Gly Phe Gly Asn
                   325                 330                 335
   Leu Arg Gly Gln Met Glu Val Trp Asp Val Lys Asn Tyr Lys Leu Ile
               340                 345                 350
   Ser Lys Pro Val Ala Ser Asp Ser Thr Tyr Phe Ala Trp Cys Pro Asp
           355                 360                 365
   Gly Glu His Ile Leu Thr Ala Thr Cys Ala Pro Arg Leu Arg Val Asn
       370                 375                 380
   Asn Gly Tyr Lys Ile Trp His Tyr Thr Gly Ser Ile Leu His Lys Tyr
   385                 390                 395                 400
   Asp Val Pro Ser Asn Ala Glu Leu Trp Gln Val Ser Trp Gln Pro Phe
                   405                 410                 415
   Leu Asp Gly Ile Phe Pro Ala Lys Thr Ile Thr Tyr Gln Ala Val Pro
               420                 425                 430
   Ser Glu Val Pro Asn Glu Glu Pro Lys Val Ala Thr Ala Tyr Arg Pro
           435                 440                 445
   Pro Ala Leu Arg Asn Lys Pro Ile Thr Asn Ser Lys Leu His Glu Glu
       450                 455                 460
   Glu Pro Pro Gln Asn Met Lys Pro Gln Ser Gly Asn Asp Lys Pro Leu
   465                 470                 475                 480
   Ser Lys Thr Ala Leu Lys Asn Gln Arg Lys His Glu Ala Lys Lys Ala
                   485                 490                 495
   Ala Lys Gln Glu Ala Arg Ser Asp Lys Ser Pro Asp Leu Ala Pro Thr
               500                 505                 510
   Pro Ala Pro Gln Ser Thr Pro Arg Asn Thr Val Ser Gln Ser Ile Ser
           515                 520                 525
   Gly Asp Pro Glu Ile Asp Lys Lys Ile Lys Asn Leu Lys Lys Lys Leu
       530                 535                 540
   Lys Ala Ile Glu Gln Leu Lys Glu Gln Ala Ala Thr Gly Lys Gln Leu
   545                 550                 555                 560
   Glu Lys Asn Gln Leu Glu Lys Ile Gln Lys Glu Thr Ala Leu Leu Gln
                   565                 570                 575
   Glu Leu Glu Asp Leu Glu Leu Gly Ile
               580                 585
<210> 7
   <211> 1110
   <212> DNA
   <213> Homo sapiens
<220>
   <221> CDS
   <222> (78)...(642)
<400> 7
   tagtgggccc cagtgttgcg ctctctggcc gttccttaca ctttgcttca ggctccagtg      60
   caggggcgta gtgggat atg gcc aac tcg ggc tgc aag gac gtc acg ggt       110
                      Met Ala Asn Ser Gly Cys Lys Asp Val Thr Gly
                      1               5                   10
   cca gat gag gag agt ttt ctg tac ttt gcc tac ggc agc aac ctg ctg      158
   Pro Asp Glu Glu Ser Phe Leu Tyr Phe Ala Tyr Gly Ser Asn Leu Leu
               15                  20                  25
   aca gag agg atc cac ctc cga aac ccc tcg gcg gcg ttc ttc tgt gtg      206
   Thr Glu Arg Ile His Leu Arg Asn Pro Ser Ala Ala Phe Phe Cys Val
           30                  35                  40
   gcc cgc ctg cag gat ttt aag ctt gac ttt ggc aat tcc caa ggc aaa      254
   Ala Arg Leu Gln Asp Phe Lys Leu Asp Phe Gly Asn Ser Gln Gly Lys
       45                  50                  55
   aca agt caa act tgg cat gga ggg ata gcc acc att ttt cag agt cct      302
   Thr Ser Gln Thr Trp His Gly Gly Ile Ala Thr Ile Phe Gln Ser Pro
   60                  65                  70                  75
   ggc gat gaa gtg tgg gga gta gta tgg aaa atg aac aaa agc aat tta      350
   Gly Asp Glu Val Trp Gly Val Val Trp Lys Met Asn Lys Ser Asn Leu
                   80                  85                  90
   aat tct ctg gat gag caa gaa ggg gtt aaa agt gga atg tat gtt gta      398
   Asn Ser Leu Asp Glu Gln Glu Gly Val Lys Ser Gly Met Tyr Val Val
               95                  100                 105
   ata gaa gtt aaa gtt gca act caa gaa gga aaa gaa ata acc tgt cga      446
   Ile Glu Val Lys Val Ala Thr Gln Glu Gly Lys Glu Ile Thr Cys Arg
           110                 115                 120
   agt tat ctg atg aca aat tac gaa agt gct ccc cca tcc cca cag tat      494
   Ser Tyr Leu Met Thr Asn Tyr Glu Ser Ala Pro Pro Ser Pro Gln Tyr
       125                 130                 135
   aaa aag att att tgc atg ggt gca aaa gaa aat ggt ttg ccg ctg gag      542
   Lys Lys Ile Ile Cys Met Gly Ala Lys Glu Asn Gly Leu Pro Leu Glu
   140                 145                 150                 155
   tat caa gag aag tta aaa gca ata gaa cca aat gac tat aca gga aag      590
   Tyr Gln Glu Lys Leu Lys Ala Ile Glu Pro Asn Asp Tyr Thr Gly Lys
                   160                 165                 170
   gtc tca gaa gaa att gaa gac atc atc aaa aag ggg gaa aca caa act      638
   Val Ser Glu Glu Ile Glu Asp Ile Ile Lys Lys Gly Glu Thr Gln Thr
               175                 180                 185
   ctt t agaacataac agaatatatc taagggtatt ctatgtgcta atataaaata         692
   Leu
   tttttaacac ttgagaacag ggatctgggg gatctccacg tttgatccat tttcagcagt    752
   gctctgaagg agtatcttac ttgggtgatt ccttgttttt agactataaa aagaaactgg    812
   gataggagtt agacaattta aaaggggtgt atgagggcct gaaatatgtg acaaatgaat     872
   gtgagtaccc cttctgtgaa cactgaaagc tattctcttg aattgatctt aagtgtctcc    932
   ttgctctggt aaaagataga tttgtagctc acttgatgat ggtgctggtg aattgctctg    992
   ctctgtctga gatttttaaa aatcagctta atgagagtaa tctgcagaca attgataata   1052
   acattttgaa aattggaaag atggtatact gtttttagag gaataaacgt atttgtgg     1110
<210> 8
   <211> 188
   <212> PRT
   <213> Homo sapiens
<400> 8
   Met Ala Asn Ser Gly Cys Lys Asp Val Thr Gly Pro Asp Glu Glu Ser
    1               5                  10                  15
   Phe Leu Tyr Phe Ala Tyr Gly Ser Asn Leu Leu Thr Glu Arg Ile His
               20                  25                  30
   Leu Arg Asn Pro Ser Ala Ala Phe Phe Cys Val Ala Arg Leu Gln Asp
           35                  40                  45
   Phe Lys Leu Asp Phe Gly Asn Ser Gln Gly Lys Thr Ser Gln Thr Trp
       50                  55                  60
   His Gly Gly Ile Ala Thr Ile Phe Gln Ser Pro Gly Asp Glu Val Trp
   65                  70                  75                  80
   Gly Val Val Trp Lys Met Asn Lys Ser Asn Leu Asn Ser Leu Asp Glu
                   85                  90                  95
   Gln Glu Gly Val Lys Ser Gly Met Tyr Val Val Ile Glu Val Lys Val
               100                 105                 110
   Ala Thr Gln Glu Gly Lys Glu Ile Thr Cys Arg Ser Tyr Leu Met Thr
           115                 120                 125
   Asn Tyr Glu Ser Ala Pro Pro Ser Pro Gln Tyr Lys Lys Ile Ile Cys
       130                 135                 140
   Met Gly Ala Lys Glu Asn Gly Leu Pro Leu Glu Tyr Gln Glu Lys Leu
   145                 150                 155                 160
   Lys Ala Ile Glu Pro Asn Asp Tyr Thr Gly Lys Val Ser Glu Glu Ile
                   165                 170                 175
   Glu Asp Ile Ile Lys Lys Gly Glu Thr Gln Thr Leu
               180                 185
<210> 9
   <211> 965
   <212> DNA
   <213> Homo sapiens
<220>
   <221> CDS
   <222> (79)...(232)
<400> 9
   gtagtgggcc ccagtgttgc gctctctggc cgttccttac actttgcttc aggctccagt     60
   gcaggggcgt agtgggat atg gcc aac tcg ggc tgc aag gac gtc acg ggt      111
                       Met Ala Asn Ser Gly Cys Lys Asp Val Thr Gly
                        1               5                   10
   cca gat gag gag agt ttt ctg tac ttt gcc tac ggc agc aac ctg ctg      159
   Pro Asp Glu Glu Ser Phe Leu Tyr Phe Ala Tyr Gly Ser Asn Leu Leu
               15                  20                  25
   aca gag agg atc cac ctc cga aac ccc tcg gcg gcg ttc ttc tgt gtg      207
   Thr Glu Arg Ile His Leu Arg Asn Pro Ser Ala Ala Phe Phe Cys Val
           30                  35                  40
   gcc cgc ctg cag gca aga agg ggt t aaaagtggaa tgtatgttgt              252
   Ala Arg Leu Gln Ala Arg Arg Gly
       45                  50
   aatagaagtt aaagttgcaa ctcaagaagg aaaagaaata acctgtcgaa gttatctgat    312
   gacaaattac gaaagtgctc ccccatcccc acagtataaa aagattattt gcatgggtgc    372
   aaaagaaaat ggtttgccgc tggagtatca agagaagtta aaagcaatag aaccaaatga    432
   ctatacagga aaggtctcag aagaaattga agacatcatc aaaaaggggg aaacacaaac    492
   tctttagaac ataacagaat atatctaagg gtattctatg tgctaatata aaatattttt    552
   aacacttgag aacagggatc tgggggatct ccacgtttga tccattttca gcagtgctct    612
   gaaggagtat cttacttggg tgattccttg tttttagact ataaaaagaa actgggatag    672
   gagttagaca atttaaaagg ggtgtatgag ggcctgaaat atgtgacaaa tgaatgtgag    732
   taccccttct gtgaacactg aaagctattc tcttgaattg atcttaagtg tctccttgct    792
   ctggtaaaag atagatttgt agctcacttg atgatggtgc tggtgaattg ctctgctctg    852
   tctgagattt ttaaaaatca gcttaatgag agtaatctgc agacaattga taataacatt    912
   ttgaaaattg gaaagatggt atactgtttt tagaggaata aacgtatttg tgg            965
<210> 10
   <211> 51
   <212> PRT
   <213> Homo sapiens
<400> 10
   Met Ala Asn Ser Gly Cys Lys Asp Val Thr Gly Pro Asp Glu Glu Ser
   1               5                   10                  15
   Phe Leu Tyr Phe Ala Tyr Gly Ser Asn Leu Leu Thr Glu Arg Ile His
               20                  25                  30
   Leu Arg Asn Pro Ser Ala Ala Phe Phe Cys Val Ala Arg Leu Gln Ala
           35                  40                  45
   Arg Arg Gly
       50
<210> 11
   <211> 658
   <212> DNA
   <213> Homo sapiens
<400> 11
   tcgagaggag ctgacagcct tcctgcaccc cgaggagtga ggtgggccac tgggtgctgc      60
   cccctgccca ggaccagccc ctggtggaag ccaaccacct gctgcacgag agcgacacgg     120
   acaaggacgg gcggctgagc aaagcggaaa tcctgggtaa ttggaacatg tttgtgggca     180
   gtcaggccac caactatggt gaggacctga cccggcacca cgatgagctg tgagccccgc     240
   gcacctgcca cagcctcaga ggcccgcaca atgaccggag gaggggccgc tgtggtctgg     300
   ccccctccct gtccaggccc cgcaggaggc agatgcagtc ccaggcatcc tcctgcccct     360
   gggctctcag ggaccccctg ggtcggcttc tgtccctgtc acacccccaa ccccagggag     420
   gggctgtcat agtcccagag gataagcaat acctatttct gactgagtct cccagcccag     480
   acccagggac cctggcccca agctcagctc taagaaccgc caccaacccc tccagctcca     540
   aatctgagcc tccaccacat agactgaaac tcccctggcc ccagccctct cctgcctggc     600
   ctggcctggg acacctcctc tctgccagga ggcaataaaa gccagcgccg ggaccttg       658
<210> 12
   <211> 1507
   <212> DNA
   <213> Homo sapiens
<220>
   <221> misc feature
   <222> 1047, 1301
   <223> n = A,T,C or G
<400> 12
   ggaacgcaga gcggagcgtg gagagcggag cgaagctgga taacagggga ccgatgatgt      60
   ggcgaccatc agttctgctg cttctgttgc tactgaggca cggggcccag gggaagccat     120
   ccccagacgc aggccctcat ggccagggga gggtgcacca ggcggccccc ctgagcgacg     180
   ctccccatga tgacgcccac gggaacttcc agtacgacca tgaggctttc ctgggacggg     240
   aagtggccaa ggaattcgac caactcaccc cagaggaaag ccaggcccgt ctggggcgga     300
   tcgtggaccg catggaccgc gcgggggacg gcgacggctg ggtgtcgctg gccgagcttc     360
   gcgcgtggat cgcgcacacg cagcagcggc acatacggga ctcggtgagc gcggcctggg     420
   acacgtacga cacggaccgc gacgggcgtg tgggttggga ggagctgcgc aacgccacct     480
   atggccacta cgcgcccggt gaagaatttc atgacgtgga ggatgcagag acctacaaaa     540
   agatgctggc tcgggacgag cggcgtttcc gggtggccga ccaggatggg gactcgatgg     600
   ccactcgaga ggagctgaca gccttcctgc accccgagga gttccctcac atgcgggaca     660
   tcgtgattgc tgaaaccctg gaggacctgg acagaaacaa agatggctat gtccaggtgg     720
   aggagtacat cgcggatctg tactcagccg agcctgggga ggaggagccg gcgtgggtgc     780
   agacggagag gcagcagttc cgggacttcc gggatctgaa caaggatggg cacctggatg     840
   ggagtgaggt gggccactgg gtgctgcccc ctgcccagga ccagcccctg gtggaagcca     900
   accacctgct gcacgaragc gacacggaca aggaygggcg gctgagcaaa gcgsaaatcc     960
   tgggtaattg gaacatgttt gtgggcagtc aggccaccaa ctatggygag gacctgaccc    1020
   ggcaccacga tgagctgtga gcmccgngca cctgccacag cctcagaggc ccgcacaatg    1080
   accggaggag gggccgctgt ggtctggccc cctccctgtc caggccccgc aggaggcaga    1140
   tgcagtccca ggcatcctcc tkcccctggg ctctcaggga ccccctgggt cggcttctgt    1200
   ccctgtcaca cccccaaccc cagggagggg ctgtcatagt cccagaggat aagcaatacc    1260
   tatttctgac tgagtctccc agcccagacc cagggaccct nggccccaag ctcagctcta    1320
   agaaccgccc caacccctcc agctccaaat ctgagcctcc accacataga ctgaaactcc    1380
   cctggcccca gccctctcct gcctggcctg gcctgggaca cctcctctct gccaggaggc    1440
   aataaaagcc agcgccggga aaaaaaaaaa aaaaaaaaaa aaaaaaaaaa aaaaaaaaaa    1500
   aaaaaan                                                              1507
<210> 13
   <211> 661
   <212> DNA
   <213> Homo sapiens
<220>
   <221> CDS
   <222> (79)...(376)
<400> 13
   ggcgcggtgc agggctctta agaacgaacg gcttgggcgc ggactggtat ccggggactg      60
   tgacttgcag ggtccgcc atg gag cca gag cag atg ctg gag gga caa acg       111
                       Met Glu Pro Glu Gln Met Leu Glu Gly Gln Thr
                        1               5                  10
   cag gtt gca gaa aat cct cac tct gag tac ggt ctc aca gac aac gtt       159
   Gln Val Ala Glu Asn Pro His Ser Glu Tyr Gly Leu Thr Asp Asn Val
               15                  20                  25
   gag aga ata gta gaa aat gag aag att aat gca gaa aag tca tca aag       207
   Glu Arg Ile Val Glu Asn Glu Lys Ile Asn Ala Glu Lys Ser Ser Lys
           30                  35                  40
   cag aag gta gat ctc cag tct ttg cca act cgt gcc tac ctg gat cag       255
   Gln Lys Val Asp Leu Gln Ser Leu Pro Thr Arg Ala Tyr Leu Asp Gln
       45                  50                  55
   aca gtt gtg cct atc tta tta cag gga ctt gct gtg ctt gca aag gaa       303
   Thr Val Val Pro Ile Leu Leu Gln Gly Leu Ala Val Leu Ala Lys Glu
    60                  65                  70                  75
   aga cca cca aat ccc att gaa ttt cta gca tct tat ctt tta aaa aac       351
   Arg Pro Pro Asn Pro Ile Glu Phe Leu Ala Ser Tyr Leu Leu Lys Asn
                   80                  85                  90
   aag gca cag ttt gaa gat cga aac t gacttaatgg gaagaacaga               396
   Lys Ala Gln Phe Glu Asp Arg Asn
               95
   aaaatttagt tgctactgta gatttacatg attaagaggc agctttaatt gccatgatca      456
   ttccctcttt ttggatgtat aagaaccttc cggacaacag aacctatttc tggaattgca      516
   gaagataaca tatttccctt attttgattt aatcaccata aaccatacct atttaatgag      576
   tgtattctgt gcaatttttt tctcagattg tctttaactt tgtttttaaa atgaccttca      636
   aaataaactg tcaaaacacc attat                                            661
<210> 14
   <211> 99
   <212> PRT
   <213> Homo sapiens
<400> 14
   Met Glu Pro Glu Gln Met Leu Glu Gly Gln Thr Gln Val Ala Glu Asn
    1               5                  10                  15
   Pro His Ser Glu Tyr Gly Leu Thr Asp Asn Val Glu Arg Ile Val Glu
               20                  25                  30
   Asn Glu Lys Ile Asn Ala Glu Lys Ser Ser Lys Gln Lys Val Asp Leu
           35                  40                  45
   Gln Ser Leu Pro Thr Arg Ala Tyr Leu Asp Gln Thr Val Val Pro Ile
       50                  55                  60
   Leu Leu Gln Gly Leu Ala Val Leu Ala Lys Glu Arg Pro Pro Asn Pro
   65                  70                  75                  80
   Ile Glu Phe Leu Ala Ser Tyr Leu Leu Lys Asn Lys Ala Gln Phe Glu
                   85                  90                  95
   Asp Arg Asn
<210> 15
   <211> 1507
   <212> DNA
   <213> Homo sapiens
<220>
   <221> misc_feature
   <222> 1047, 1301
   <223> n = A,T,C or G
<400> 15
   ggaacgcaga gcggagcgtg gagagcggag cgaagctgga taacagggga ccgatgatgt      60
   ggcgaccatc agttctgctg cttctgttgc tactgaggca cggggcccag gggaagccat     120
   ccccagacgc aggccctcat ggccagggga gggtgcacca ggcggccccc ctgagcgacg     180
   ctccccatga tgacgcccac gggaacttcc agtacgacca tgaggctttc ctgggacggg     240
   aagtggccaa ggaattcgac caactcaccc cagaggaaag ccaggcccgt ctggggcgga     300
   tcgtggaccg catggaccgc gcgggggacg gcgacggctg ggtgtcgctg gccgagcttc     360
   gcgcgtggat cgcgcacacg cagcagcggc acatacggga ctcggtgagc gcggcctggg     420
   acacgtacga cacggaccgc gacgggcgtg tgggttggga ggagctgcgc aacgccacct     480
   atggccacta cgcgcccggt gaagaatttc atgacgtgga ggatgcagag acctacaaaa     540
   agatgctggc tcgggacgag cggcgtttcc gggtggccga ccaggatggg gactcgatgg     600
   ccactcgaga ggagctgaca gccttcctgc accccgagga gttccctcac atgcgggaca     660
   tcgtgattgc tgaaaccctg gaggacctgg acagaaacaa agatggctat gtccaggtgg     720
   aggagtacat cgcggatctg tactcagccg agcctgggga ggaggagccg gcgtgggtgc     780
   agacggagag gcagcagttc cgggacttcc gggatctgaa caaggatggg cacctggatg     840
   ggagtgaggt gggccactgg gtgctgcccc ctgcccagga ccagcccctg gtggaagcca     900
   accacctgct gcacgaragc gacacggaca aggaygggcg gctgagcaaa gcgsaaatcc     960
   tgggtaattg gaacatgttt gtgggcagtc aggccaccaa ctatggygag gacctgaccc    1020
   ggcaccacga tgagctgtga gcmccgngca cctgccacag cctcagaggc ccgcacaatg    1080
   accggaggag gggccgctgt ggtctggccc cctccctgtc caggccccgc aggaggcaga    1140
   tgcagtccca ggcatcctcc tkcccctggg ctctcaggga ccccctgggt cggcttctgt    1200
   ccctgtcaca cccccaaccc cagggagggg ctgtcatagt cccagaggat aagcaatacc    1260
   tatttctgac tgagtctccc agcccagacc cagggaccct nggccccaag ctcagctcta    1320
   agaaccgccc caacccctcc agctccaaat ctgagcctcc accacataga ctgaaactcc    1380
   cctggcccca gccctctcct gcctggcctg gcctgggaca cctcctctct gccaggaggc    1440
   aataaaagcc agcgccggga aaaaaaaaaa aaaaaaaaaa aaaaaaaaaa aaaaaaaaaa    1500
   aaaaaan                                                              1507
<210> 16
   <211> 716
   <212> DNA
   <213> Homo sapiens
<220>
   <221> CDS
   <222> (16)...(538)
<400> 16
   ctcgatcgaa gcgag atg gcg gac gtg cta gat ctt cac gag gct ggg ggc      51
                    Met Ala Asp Val Leu Asp Leu His Glu Ala Gly Gly
                     1               5                  10
   gaa gat ttc gcc atg gat gag gat ggg gac gag agc att cac aaa ctg       99
   Glu Asp Phe Ala Met Asp Glu Asp Gly Asp Glu Ser Ile His Lys Leu
            15                  20                  25
   aaa gaa aaa gcg aag aaa cgg aag ggt cgc ggc ttt ggc tcc gaa gag      147
   Lys Glu Lys Ala Lys Lys Arg Lys Gly Arg Gly Phe Gly Ser Glu Glu
        30                  35                  40
   ggg tcc cga gcg cgg atg cgt gag gat tat gac agc gtg gag cag gat      195
   Gly Ser Arg Ala Arg Met Arg Glu Asp Tyr Asp Ser Val Glu Gln Asp
    45                  50                  55                  60
   ggc gat gaa ccc gga cca caa cgc tct gtt gaa ggc tgg att ctc ttt      243
   Gly Asp Glu Pro Gly Pro Gln Arg Ser Val Glu Gly Trp Ile Leu Phe
                    65                  70                  75
   gta act gga gtc cat gag gaa gcc acc gaa gaa gac ata cac gac aaa      291
   Val Thr Gly Val His Glu Glu Ala Thr Glu Glu Asp Ile His Asp Lys
                80                  85                  90
   ttc gca gaa tat ggg gaa att aaa aac att cat ctc aac ctc gac agg      339
   Phe Ala Glu Tyr Gly Glu Ile Lys Asn Ile His Leu Asn Leu Asp Arg
            95                 100                 105
   cga aca gga tat ctg aag ggg tat act cta gtt gaa tat gaa aca tac      387
   Arg Thr Gly Tyr Leu Lys Gly Tyr Thr Leu Val Glu Tyr Glu Thr Tyr
       110                 115                 120
   aag gaa gcc cag gct gct atg gag gga ctc aat ggc cag gat ttg atg      435
   Lys Glu Ala Gln Ala Ala Met Glu Gly Leu Asn Gly Gln Asp Leu Met
   125                 130                 135                 140
   gga cag ccc atc agc gtt gac tgg tgt ttt gtt cgg ggt cca cca aaa      483
   Gly Gln Pro Ile Ser Val Asp Trp Cys Phe Val Arg Gly Pro Pro Lys
                   145                 150                 155
   ggc aag agg aga ggt ggc cga aga cgc agc aga agt cca gac cgg aga      531
   Gly Lys Arg Arg Gly Gly Arg Arg Arg Ser Arg Ser Pro Asp Arg Arg
               160                 165                 170
   cgt cgc t gacaggtcct ctgttgtcca ggtgttctct tcaagattcc atttgaccat      588
   Arg Arg
   gcagccttgg acaaatagga ctggggtgga acttgctgtg tttatattta atctcttacc      648
   gtatatgcgt agtatttgag ttgcgaataa atgttccatt ttgttttcta caaaaaaaaa      708
   aaaaaaaa                                                             716
<210> 17
   <211> 174
   <212> PRT
   <213> Homo sapiens
<400> 17
   Met Ala Asp Val Leu Asp Leu His Glu Ala Gly Gly Glu Asp Phe Ala
    1               5                  10                  15
   Met Asp Glu Asp Gly Asp Glu Ser Ile His Lys Leu Lys Glu Lys Ala
               20                  25                  30
   Lys Lys Arg Lys Gly Arg Gly Phe Gly Ser Glu Glu Gly Ser Arg Ala
           35                  40                  45
   Arg Met Arg Glu Asp Tyr Asp Ser Val Glu Gln Asp Gly Asp Glu Pro
       50                  55                  60
   Gly Pro Gln Arg Ser Val Glu Gly Trp Ile Leu Phe Val Thr Gly Val
   65                  70                  75                  80
   His Glu Glu Ala Thr Glu Glu Asp Ile His Asp Lys Phe Ala Glu Tyr
                   85                  90                  95
   Gly Glu Ile Lys Asn Ile His Leu Asn Leu Asp Arg Arg Thr Gly Tyr
               100                 105                 110
   Leu Lys Gly Tyr Thr Leu Val Glu Tyr Glu Thr Tyr Lys Glu Ala Gln
           115                 120                 125
   Ala Ala Met Glu Gly Leu Asn Gly Gln Asp Leu Met Gly Gln Pro Ile
       130                 135                 140
   Ser Val Asp Trp Cys Phe Val Arg Gly Pro Pro Lys Gly Lys Arg Arg
   145                 150                 155                 160
   Gly Gly Arg Arg Arg Ser Arg Ser Pro Asp Arg Arg Arg Arg
                   165                 170
<210> 18
   <211> 763
   <212> DNA
   <213> Homo sapiens
<220>
   <221> CDS
   <222> (2)...(551)
<400> 18
   c atg gcc aag ccg tgt ggg gtg cgc ctg agc ggg gaa gcc cgc aaa cag      49
     Met Ala Lys Pro Cys Gly Val Arg Leu Ser Gly Glu Ala Arg Lys Gln
      1               5                  10                  15
   gtg gag gtc ttc aga cag aat ctt ttc cag gag gct gag gaa ttc ctc        97
   Val Glu Val Phe Arg Gln Asn Leu Phe Gln Glu Ala Glu Glu Phe Leu
                   20                  25                  30
   tac aga ttc ttg cca cag aaa atc ata tac ctg aat cag ctc ttg caa       145
   Tyr Arg Phe Leu Pro Gln Lys Ile Ile Tyr Leu Asn Gln Leu Leu Gln
               35                  40                  45
   gag gac tcc ctc aat gtg gct gac ttg act tcc ctc cgg gcc cca ctg       193
   Glu Asp Ser Leu Asn Val Ala Asp Leu Thr Ser Leu Arg Ala Pro Leu
       50                  55                  60
   gac atc ccc atc cca gac cct cca ccc aag gat gat gag atg gaa aca       241
   Asp Ile Pro Ile Pro Asp Pro Pro Pro Lys Asp Asp Glu Met Glu Thr
   65                  70                  75                  80
   gat aag cag gag aag aaa gaa gtc cct aag tgt gga ttt ctc cct ggg       289
   Asp Lys Gln Glu Lys Lys Glu Val Pro Lys Cys Gly Phe Leu Pro Gly
                   85                  90                  95
   aat gag aaa gtc ctg tcc ctg ctt gcc ctg gtt aag cca gaa gtc tgg       337
   Asn Glu Lys Val Leu Ser Leu Leu Ala Leu Val Lys Pro Glu Val Trp
               100                 105                 110
   act ctc aaa gag aaa tgc att ctg gtg att aca tgg atc caa cac ctg       385
   Thr Leu Lys Glu Lys Cys Ile Leu Val Ile Thr Trp Ile Gln His Leu
           115                 120                 125
   atc ccc aag att gaa gat gga aat gat ttt ggg gta gca atc cag gag       433
   Ile Pro Lys Ile Glu Asp Gly Asn Asp Phe Gly Val Ala Ile Gln Glu
       130                 135                 140
   aag gtg ctg gag agg gtg aat gcc gtc aag acc aaa gtg aag ctt tcc       481
   Lys Val Leu Glu Arg Val Asn Ala Val Lys Thr Lys Val Lys Leu Ser
   145                 150                 155                 160
   aga caa cca ttt cca agt act tct cag aac gtg ggg atg ctg tgg cca       529
   Arg Gln Pro Phe Pro Ser Thr Ser Gln Asn Val Gly Met Leu Trp Pro
                   165                 170                 175
   agg cct cca agg aga ctc atg t aatggattac cgggccttgg tgcatgagcg        581
   Arg Pro Pro Arg Arg Leu Met
               180
   agatgaggca gcctatgggg agctcagggc catggtgctg gacctgaggg ccttctatgc     641
   tgagctttat catatcatca gcagcaacct ggagaaaatt gtcaacccaa agggtgaaga     701
   aaagccatct atgtactgaa cccgggacta gaaggaaaat aaatgatcta tatgttgtgt     761
   gg                                                                    763
<210> 19
   <211> 183
   <212> PRT
   <213> Homo sapiens
<400> 19
   Met Ala Lys Pro Cys Gly Val Arg Leu Ser Gly Glu Ala Arg Lys Gln
    1               5                  10                  15
   Val Glu Val Phe Arg Gln Asn Leu Phe Gln Glu Ala Glu Glu Phe Leu
               20                  25                  30
   Tyr Arg Phe Leu Pro Gln Lys Ile Ile Tyr Leu Asn Gln Leu Leu Gln
           35                  40                  45
   Glu Asp Ser Leu Asn Val Ala Asp Leu Thr Ser Leu Arg Ala Pro Leu
       50                  55                  60
   Asp Ile Pro Ile Pro Asp Pro Pro Pro Lys Asp Asp Glu Met Glu Thr
   65                  70                  75                  80
   Asp Lys Gln Glu Lys Lys Glu Val Pro Lys Cys Gly Phe Leu Pro Gly
                   85                  90                  95
   Asn Glu Lys Val Leu Ser Leu Leu Ala Leu Val Lys Pro Glu Val Trp
               100                 105                 110
   Thr Leu Lys Glu Lys Cys Ile Leu Val Ile Thr Trp Ile Gln His Leu
           115                 120                 125
   Ile Pro Lys Ile Glu Asp Gly Asn Asp Phe Gly Val Ala Ile Gln Glu
       130                 135                 140
   Lys Val Leu Glu Arg Val Asn Ala Val Lys Thr Lys Val Lys Leu Ser
   145                 150                 155                 160
   Arg Gln Pro Phe Pro Ser Thr Ser Gln Asn Val Gly Met Leu Trp Pro
                   165                 170                 175
   Arg Pro Pro Arg Arg Leu Met
               180
<210> 20
   <211> 790
   <212> DNA
   <213> Homo sapiens
<220>
   <221> CDS
   <222> (240)...(585)
<400> 20
   acgttttaca gtctttaatt aagcacataa aactgtacta tttaatatat ttctccatga     60
   actttttgtg aaattcagat cgcagtgtgt catttacaaa tcttttgtct ttcttctggt    120
   catctacacc ttttgcacag ttcttgaaga caacgtcatc atcccacctt cttttaactt    180
   tgaagttggc ctgaggctgg gatgggccag tgagattaag gagagggttt ccgctcaga     239
   atg ttt tcc ata cga atc ctc tct tct tca gct ttt tgt tct tgt tcc      287
   Met Phe Ser Ile Arg Ile Leu Ser Ser Ser Ala Phe Cys Ser Cys Ser
    1               5                  10                  15
   ttc ctg gcc tgc tct tca gct ctt tct ttt tta att ttt tcc agt tct       335
   Phe Leu Ala Cys Ser Ser Ala Leu Ser Phe Leu Ile Phe Ser Ser Ser
               20                  25                  30
   gca aga aga gct gca gta tca tca tca tca ctt tct tct tca aaa tct       383
   Ala Arg Arg Ala Ala Val Ser Ser Ser Ser Leu Ser Ser Ser Lys Ser
           35                  40                  45
   tca tct tcc tca tct gtt aga ggg tca tct gca tca agg ttg gcg gca       431
   Ser Ser Ser Ser Ser Val Arg Gly Ser Ser Ala Ser Arg Leu Ala Ala
       50                  55                  60
   gga atc tgg tct aac cgt ggc ttt ttt gac act gaa gag gag gtt gta       479
   Gly Ile Trp Ser Asn Arg Gly Phe Phe Asp Thr Glu Glu Glu Val Val
   65                  70                  75                  80
   tgt tct cgg gtt gga cga tcc cta ttt ttc tct ctt gca gca gct ctc       527
   Cys Ser Arg Val Gly Arg Ser Leu Phe Phe Ser Leu Ala Ala Ala Leu
                   85                  90                  95
   tct ctt tct tcc aac tct ctc ctg aag tca cgg tta cga acc tct tca       575
   Ser Leu Ser Ser Asn Ser Leu Leu Lys Ser Arg Leu Arg Thr Ser Ser
               100                 105                 110
   ggg gca tcc t gagtagtctg tctgtatttt atctttgtat gagagggtag             625
   Gly Ala Ser
           115
   gtctctgctt gaatactgct ttgaaagttg gctcaaatca ccttctcctt ttccccttcc     685
   acctctggca ggttcaaagg ttggcctggc tgctgttgtc atcttttatg actggccgag     745
   gtccgatgca gcaggctccg aagatcatac agacgccatt accac                     790
<210> 21
   <211> 115
   <212> PRT
   <213> Homo sapiens
<400> 21
   Met Phe Ser Ile Arg Ile Leu Ser Ser Ser Ala Phe Cys Ser Cys Ser
   1               5                   10                  15
   Phe Leu Ala Cys Ser Ser Ala Leu Ser Phe Leu Ile Phe Ser Ser Ser
               20                  25                  30
   Ala Arg Arg Ala Ala Val Ser Ser Ser Ser Leu Ser Ser Ser Lys Ser
           35                  40                  45
   Ser Ser Ser Ser Ser Val Arg Gly Ser Ser Ala Ser Arg Leu Ala Ala
       50                  55                  60
   Gly Ile Trp Ser Asn Arg Gly Phe Phe Asp Thr Glu Glu Glu Val Val
   65                  70                  75                  80
   Cys Ser Arg Val Gly Arg Ser Leu Phe Phe Ser Leu Ala Ala Ala Leu
                   85                  90                  95
   Ser Leu Ser Ser Asn Ser Leu Leu Lys Ser Arg Leu Arg Thr Ser Ser
               100                 105                 110
   Gly Ala Ser
           115
<210> 22
   <211> 1939
   <212> DNA
   <213> Homo sapiens
<220>
   <221> CDS
   <222> (53)...(1700)
<400> 22
   gtggcgccag cggaggcagg ttgctgtgtt tgtgcttcct tctacagcca at atg aaa      58
                                                             Met Lys
                                                              1
   agg cct aag tta aag aaa gca agt aaa cgc atg acc tgc cat aag cgg       106
   Arg Pro Lys Leu Lys Lys Ala Ser Lys Arg Met Thr Cys His Lys Arg
           5                   10                  15
   tat aaa atc caa aaa aag gtt cga gaa cat cat cga aaa tta aga aag       154
   Tyr Lys Ile Gln Lys Lys Val Arg Glu His His Arg Lys Leu Arg Lys
       20                  25                  30
   gag gct aaa aag cag ggt cac aag aag cct agg aaa gac cca gga gtt       202
   Glu Ala Lys Lys Gln Gly His Lys Lys Pro Arg Lys Asp Pro Gly Val
   35                  40                  45                  50
   cca aac agt gct ccc ttt aag gag gct ctt ctt agg gaa gct gag cta       250
   Pro Asn Ser Ala Pro Phe Lys Glu Ala Leu Leu Arg Glu Ala Glu Leu
                   55                  60                  65
   agg aaa cag agg ctt gaa gaa cta aaa cag cag cag aaa ctt gac agg       298
   Arg Lys Gln Arg Leu Glu Glu Leu Lys Gln Gln Gln Lys Leu Asp Arg
               70                  75                  80
   cag aag gaa cta gaa aag aaa aga aaa ctt gaa act aat cct gat att       346
   Gln Lys Glu Leu Glu Lys Lys Arg Lys Leu Glu Thr Asn Pro Asp Ile
           85                  90                  95
   aag cca tca aat gtg gaa cct atg gaa aag gag ttt ggg ctt tgc aaa       394
   Lys Pro Ser Asn Val Glu Pro Met Glu Lys Glu Phe Gly Leu Cys Lys
       100                 105                 110
   act gag aac aaa gcc aag tcg ggc aaa cag aat tca aag aag ctg tac       442
   Thr Glu Asn Lys Ala Lys Ser Gly Lys Gln Asn Ser Lys Lys Leu Tyr
   115                 120                 125                 130
   tgc caa gaa ctt aaa aag gtg att gaa gcc tcc gat gtt gtc cta gag       490
   Cys Gln Glu Leu Lys Lys Val Ile Glu Ala Ser Asp Val Val Leu Glu
                   135                 140                 145
   gtg ttg gat gcc aga gat cct ctt ggt tgc aga tgt cct cag gta gaa       538
   Val Leu Asp Ala Arg Asp Pro Leu Gly Cys Arg Cys Pro Gln Val Glu
               150                 155                 160
   gag gcc att gtc cag agt gga cag aaa aag ctg gta ctt ata tta aat       586
   Glu Ala Ile Val Gln Ser Gly Gln Lys Lys Leu Val Leu Ile Leu Asn
           165                 170                 175
   aaa tca gat ctg gta cca aag gag aat ttg gag agc tgg cta aat tat       634
   Lys Ser Asp Leu Val Pro Lys Glu Asn Leu Glu Ser Trp Leu Asn Tyr
       180                 185                 190
   ttg aag aaa gaa ttg cca aca gtg gtg ttc aga gcc tca aca aaa cca       682
   Leu Lys Lys Glu Leu Pro Thr Val Val Phe Arg Ala Ser Thr Lys Pro
   195                 200                 205                 210
   aag gat aaa ggg aag ata acc aag cgt gtg aag gca aag aag aat gct       730
   Lys Asp Lys Gly Lys Ile Thr Lys Arg Val Lys Ala Lys Lys Asn Ala
                   215                 220                 225
   gct cca ttc aga agt gaa gtc tgc ttt ggg aaa gag ggc ctt tgg aaa 778
   Ala Pro Phe Arg Ser Glu Val Cys Phe Gly Lys Glu Gly Leu Trp Lys
   230 235 240
   ctt ctt gga ggt ttt cag gaa act tgc agc aaa gcc att cgg gtt gga 826
   Leu Leu Gly Gly Phe Gln Glu Thr Cys Ser Lys Ala Ile Arg Val Gly
   245 250 255
   gta att ggt ttc cca aat gtg ggg aaa agc agc att atc aat agc tta 874
   Val Ile Gly Phe Pro Asn Val Gly Lys Ser Ser Ile Ile Asn Ser Leu
   260 265 270
   aaa caa gaa cag atg tgt aat gtt ggt gta tcc atg ggg ctt aca agg 922
   Lys Gln Glu Gln Met Cys Asn Val Gly Val Ser Met Gly Leu Thr Arg
   275 280 285 290
   agc atg caa gtt gtc ccc ttg gac aaa cag atc aca atc ata gat agt 970
   Ser Met Gln Val Val Pro Leu Asp Lys Gln Ile Thr Ile Ile Asp Ser
   295 300 305
   ccg agc ttc atc gta tct cca ctt aat tcc tcc tct gcg ctt gct ctg 1018
   Pro Ser Phe Ile Val Ser Pro Leu Asn Ser Ser Ser Ala Leu Ala Leu
   310 315 320
   cga agt cca gca agt att gaa gta gta aaa ccg atg gag gct gcc agt 1066
   Arg Ser Pro Ala Ser Ile Glu Val Val Lys Pro Met Glu Ala Ala Ser
   325 330 335
   gcc atc ctt tcc cag gct gat gct cga cag gta gta ctg aaa tat act 1114
   Ala Ile Leu Ser Gln Ala Asp Ala Arg Gln Val Val Leu Lys Tyr Thr
   340 345 350
   gtc cca ggc tac agg aat tct ctg gaa ttt ttt act atg ctt gct cag 1162
   Val Pro Gly Tyr Arg Asn Ser Leu Glu Phe Phe Thr Met Leu Ala Gln
   355 360 365 370
   aga aga ggt atg cac caa aaa ggt gga atc cca aat gtt gaa ggt gct 1210
   Arg Arg Gly Met His Gln Lys Gly Gly Ile Pro Asn Val Glu Gly Ala
   375 380 385
   gcc aaa ctg ctg tgg tct gag tgg aca ggt gcc tca tta gct tac tat 1258
   Ala Lys Leu Leu Trp Ser Glu Trp Thr Gly Ala Ser Leu Ala Tyr Tyr
   390 395 400
   tgc cat ccc cct aca tct tgg act cct cct cca tat ttt aat gag agt 1306
   Cys His Pro Pro Thr Ser Trp Thr Pro Pro Pro Tyr Phe Asn Glu Ser
   405 410 415
   att gtg gta gac atg aaa agc ggc ttc aat ctg gaa gaa ctg gaa aag 1354
   Ile Val Val Asp Met Lys Ser Gly Phe Asn Leu Glu Glu Leu Glu Lys
   420 425 430
   aac aat gca cag agc ata aga gcc atc aag ggc cct cat ttg gcc aat 1402
   Asn Asn Ala Gln Ser Ile Arg Ala Ile Lys Gly Pro His Leu Ala Asn
   435 440 445 450
   agc atc ctt ttc cag tct tcc ggt ctg aca aat gga ata ata gaa gaa 1450
   Ser Ile Leu Phe Gln Ser Ser Gly Leu Thr Asn Gly Ile Ile Glu Glu
   455 460 465
   aag gac ata cat gaa gaa ttg cca aaa cgg aaa gaa agg aag cag gag 1498
   Lys Asp Ile His Glu Glu Leu Pro Lys Arg Lys Glu Arg Lys Gln Glu
   470 475 480
   gag agg gag gat gac aaa gac agt gac cag gaa act gtt gat gaa gaa      1546
   Glu Arg Glu Asp Asp Lys Asp Ser Asp Gln Glu Thr Val Asp Glu Glu
           485                 490                 495
   gtt gat gaa aac agc tca ggc atg ttt gct gca gaa gag aca ggg gag      1594
   Val Asp Glu Asn Ser Ser Gly Met Phe Ala Ala Glu Glu Thr Gly Glu
       500                 505                 510
   gca ctg tct gag gag act aca gca ggt gaa cag tct aca agg tct ttt      1642
   Ala Leu Ser Glu Glu Thr Thr Ala Gly Glu Gln Ser Thr Arg Ser Phe
   515                 520                 525                 530
   atc ttg gat aaa atc att gaa gag gat gat gct tat gac ttc agt aca      1690
   Ile Leu Asp Lys Ile Ile Glu Glu Asp Asp Ala Tyr Asp Phe Ser Thr
                   535                 540                 545
   gat tat gtg t aacagaacaa tggcttttta tgattttttt ttttaacatt            1740
   Asp Tyr Val
   ttaagcagac tgctaaactg ttctctgtat aagttatggt atgcatgagc tgtgtaaatt    1800
   ttgtgaatat gtattatatt aaaaccaggc aacttggaat ccctaaattc tgtaaaaaga    1860
   caattcatct cattgtgagt ggaagtagtt atctggaata aaaaaagaag atacctattg    1920
   aaaaaaaaaa aaaaaaaaa                                                  1939
<210> 23
   <211> 549
   <212> PRT
   <213> Homo sapiens
<400> 23
   Met Lys Arg Pro Lys Leu Lys Lys Ala Ser Lys Arg Met Thr Cys His
    1               5                  10                  15
   Lys Arg Tyr Lys Ile Gln Lys Lys Val Arg Glu His His Arg Lys Leu
               20                  25                  30
   Arg Lys Glu Ala Lys Lys Gln Gly His Lys Lys Pro Arg Lys Asp Pro
           35                  40                  45
   Gly Val Pro Asn Ser Ala Pro Phe Lys Glu Ala Leu Leu Arg Glu Ala
       50                  55                  60
   Glu Leu Arg Lys Gln Arg Leu Glu Glu Leu Lys Gln Gln Gln Lys Leu
   65                  70                  75                  80
   Asp Arg Gln Lys Glu Leu Glu Lys Lys Arg Lys Leu Glu Thr Asn Pro
                   85                  90                  95
   Asp Ile Lys Pro Ser Asn Val Glu Pro Met Glu Lys Glu Phe Gly Leu
               100                 105                 110
   Cys Lys Thr Glu Asn Lys Ala Lys Ser Gly Lys Gln Asn Ser Lys Lys
           115                 120                 125
   Leu Tyr Cys Gln Glu Leu Lys Lys Val Ile Glu Ala Ser Asp Val Val
       130                 135                 140
   Leu Glu Val Leu Asp Ala Arg Asp Pro Leu Gly Cys Arg Cys Pro Gln
   145                 150                 155                 160
   Val Glu Glu Ala Ile Val Gln Ser Gly Gln Lys Lys Leu Val Leu Ile
                   165                 170                 175
   Leu Asn Lys Ser Asp Leu Val Pro Lys Glu Asn Leu Glu Ser Trp Leu
               180                 185                 190
   Asn Tyr Leu Lys Lys Glu Leu Pro Thr Val Val Phe Arg Ala Ser Thr
           195                 200                 205
   Lys Pro Lys Asp Lys Gly Lys Ile Thr Lys Arg Val Lys Ala Lys Lys
       210                 215                 220
   Asn Ala Ala Pro Phe Arg Ser Glu Val Cys Phe Gly Lys Glu Gly Leu
   225                 230                 235                 240
   Trp Lys Leu Leu Gly Gly Phe Gln Glu Thr Cys Ser Lys Ala Ile Arg
                   245                 250                 255
   Val Gly Val Ile Gly Phe Pro Asn Val Gly Lys Ser Ser Ile Ile Asn
               260                 265                 270
   Ser Leu Lys Gln Glu Gln Met Cys Asn Val Gly Val Ser Met Gly Leu
           275                 280                 285
   Thr Arg Ser Met Gln Val Val Pro Leu Asp Lys Gln Ile Thr Ile Ile
       290                 295                 300
   Asp Ser Pro Ser Phe Ile Val Ser Pro Leu Asn Ser Ser Ser Ala Leu
   305                 310                 315                 320
   Ala Leu Arg Ser Pro Ala Ser Ile Glu Val Val Lys Pro Met Glu Ala
                   325                 330                 335
   Ala Ser Ala Ile Leu Ser Gln Ala Asp Ala Arg Gln Val Val Leu Lys
               340                 345                 350
   Tyr Thr Val Pro Gly Tyr Arg Asn Ser Leu Glu Phe Phe Thr Met Leu
           355                 360                 365
   Ala Gln Arg Arg Gly Met His Gln Lys Gly Gly Ile Pro Asn Val Glu
       370                 375                 380
   Gly Ala Ala Lys Leu Leu Trp Ser Glu Trp Thr Gly Ala Ser Leu Ala
   385                 390                 395                 400
   Tyr Tyr Cys His Pro Pro Thr Ser Trp Thr Pro Pro Pro Tyr Phe Asn
                   405                 410                 415
   Glu Ser Ile Val Val Asp Met Lys Ser Gly Phe Asn Leu Glu Glu Leu
               420                 425                 430
   Glu Lys Asn Asn Ala Gln Ser Ile Arg Ala Ile Lys Gly Pro His Leu
           435                 440                 445
   Ala Asn Ser Ile Leu Phe Gln Ser Ser Gly Leu Thr Asn Gly Ile Ile
       450                 455                 460
   Glu Glu Lys Asp Ile His Glu Glu Leu Pro Lys Arg Lys Glu Arg Lys
   465                 470                 475                 480
   Gln Glu Glu Arg Glu Asp Asp Lys Asp Ser Asp Gln Glu Thr Val Asp
                   485                 490                 495
   Glu Glu Val Asp Glu Asn Ser Ser Gly Met Phe Ala Ala Glu Glu Thr
               500                 505                 510
   Gly Glu Ala Leu Ser Glu Glu Thr Thr Ala Gly Glu Gln Ser Thr Arg
           515                 520                 525
   Ser Phe Ile Leu Asp Lys Ile Ile Glu Glu Asp Asp Ala Tyr Asp Phe
       530                 535                 540
   Ser Thr Asp Tyr Val
   545
<210> 24
   <211> 503
   <212> DNA
   <213> Homo sapiens
<220>
   <221> CDS
   <222> (10)...(400)
<400> 24
   ggccacgta atg tcc gta gtt cgc tca tcc gtc cat gcc aga tgg att gtg      51
             Met Ser Val Val Arg Ser Ser Val His Ala Arg Trp Ile Val
              1               5                   10
   ggg aag gtg att ggg aca aaa atg caa aag act gct aaa gtg aga gtg        99
   Gly Lys Val Ile Gly Thr Lys Met Gln Lys Thr Ala Lys Val Arg Val
    15                  20                  25                  30
   acc agg ctt gtt ctg gat ccc tat tta tta aag tat ttt aat aag cgg       147
   Thr Arg Leu Val Leu Asp Pro Tyr Leu Leu Lys Tyr Phe Asn Lys Arg
                    35                  40                  45
   aaa acc tac ttt gct cac gat gcc ctt cag cag tgc aca gtt ggg gat       195
   Lys Thr Tyr Phe Ala His Asp Ala Leu Gln Gln Cys Thr Val Gly Asp
                50                  55                  60
   att gtg ctt ctc aga gct tta cct gtt cca cga gca aag cat gtg aaa       243
   Ile Val Leu Leu Arg Ala Leu Pro Val Pro Arg Ala Lys His Val Lys
           65                  70                  75
   cat gaa ctg gct gag atc gtt ttc aaa gtt gga aaa gtc ata gat cca       291
   His Glu Leu Ala Glu Ile Val Phe Lys Val Gly Lys Val Ile Asp Pro
       80                  85                  90
   gtg aca gga aag ccc tgt gct gga act acc tac ctg gag agt ccg ttg       339
   Val Thr Gly Lys Pro Cys Ala Gly Thr Thr Tyr Leu Glu Ser Pro Leu
    95                 100                 105                 110
   agt tcg gaa acc acc cag cta agc aaa aat ctg gaa gaa ctc aat atc       387
   Ser Ser Glu Thr Thr Gln Leu Ser Lys Asn Leu Glu Glu Leu Asn Ile
                   115                 120                 125
   tct tca gca cag t gaagcgggag tggaagaagg atctgaaggg aaaaactgac         440
   Ser Ser Ala Gln
             130
   atgtttatgt tatggaaaaa gaaatttttc taagtttcat cacaaaaaaa aaaaaaaaaa      500
   aaa                                                                    503
<210> 25
   <211> 130
   <212> PRT
   <213> Homo sapiens
<400> 25
   Met Ser Val Val Arg Ser Ser Val His Ala Arg Trp Ile Val Gly Lys
    1               5                  10                  15
   Val Ile Gly Thr Lys Met Gln Lys Thr Ala Lys Val Arg Val Thr Arg
               20                  25                  30
   Leu Val Leu Asp Pro Tyr Leu Leu Lys Tyr Phe Asn Lys Arg Lys Thr
           35                  40                  45
   Tyr Phe Ala His Asp Ala Leu Gln Gln Cys Thr Val Gly Asp Ile Val
       50                  55                  60
   Leu Leu Arg Ala Leu Pro Val Pro Arg Ala Lys His Val Lys His Glu
   65                  70                  75                  80
   Leu Ala Glu Ile Val Phe Lys Val Gly Lys Val Ile Asp Pro Val Thr
                   85                  90                  95
   Gly Lys Pro Cys Ala Gly Thr Thr Tyr Leu Glu Ser Pro Leu Ser Ser
               100                 105                 110
   Glu Thr Thr Gln Leu Ser Lys Asn Leu Glu Glu Leu Asn Ile Ser Ser
           115                 120                 125
   Ala Gln
       130
<210> 26
   <211> 651
   <212> DNA
   <213> Homo sapiens
<400> 26
   ggaattcggc acgaggtcga ctcctgtgag gtatggtgct gggtgcagat gcagtgtggc      60
   tctggatagc accttatgga cagttgtgtc cccaaggaag gatgagaata gctactgaag     120
   tcctaaagag caagcctaac tcaagccatt ggcacacagg cattagacag aaagctggaa     180
   gttgaaatgg tggagtccaa cttgcctgga ccagcttaat ggttctgctc ctggtaacgt     240
   ttttatccat ggatgacttg cttgggtaag gacatgaaga cagttcctgt catacctttt     300
   aaaggtatgg agagtcggct tgactacact gtgtggagca agttttaaag aagcaaagga     360
   ctcagaattc atgattgaag aaatgcaggc agacctgtta tcctaaacta gggtttttaa     420
   tgaccacaac aagcaagcat gcagcttact gcttgaaagg gtcttgcctc acccaagcta     480
   gagtgcagtg gcctttgaag cttactacag cctcaaactt ctgggctcaa gtgatcctca     540
   gcctcccagt ggtctttgta gactgcctga tggagtctca tggcacaaga agattaaaac     600
   agtgtctcca attttaataa atttttgcaa tccaaaaaaa aaaaaaaaaa a              651
<210> 27
   <211> 559
   <212> DNA
   <213> Homo sapiens
<220>
   <221> CDS
   <222> (324)...(519)
<400> 27
   ggctctggat agcaccttat ggacagttgt gtccccaagg aaggatgaga atagctactg      60
   aagtcctaaa gagcaagcct aactcaagcc attggcacac aggcattaga cagaaagctg     120
   gaagttgaaa tggtggagtc caacttgcct ggaccagctt aatggttctg ctcctggtaa     180
   cgtttttatc catggatgac ttgcttgggt atggagagtc ggcttgacta cactgtgtgg     240
   agcaagtttt aaagaagcaa aggactcaga attcatgatt gaagaaatgc aggcagacct     300
   gttatcctaa actagggttt tta atg acc aca aca agc aag cat gca gct tac     353
                             Met Thr Thr Thr Ser Lys His Ala Ala Tyr
                              1               5                  10
   tgc ttg aaa ggg tct tgc ctc acc caa gct aga gtg cag tgg cct ttg       401
   Cys Leu Lys Gly Ser Cys Leu Thr Gln Ala Arg Val Gln Trp Pro Leu
                   15                  20                  25
   aag ctt act aca gcc tca aac ttc tgg gct caa gtg atc ctc agc ctc       449
   Lys Leu Thr Thr Ala Ser Asn Phe Trp Ala Gln Val Ile Leu Ser Leu
               30                  35                  40
   cca gtg gtc ttt gta gac tgc ctg atg gag tct cat ggc aca aga aga       497
   Pro Val Val Phe Val Asp Cys Leu Met Glu Ser His Gly Thr Arg Arg
           45                  50                  55
   tta aaa cag tgt ctc caa ttt t aataaatttt tgcaatccaa aaaaaaaaaa        549
   Leu Lys Gln Cys Leu Gln Phe
       60                  65
   aaaaaaaaaa                                                            559
<210> 28
   <211> 65
   <212> PRT
   <213> Homo sapiens
<400> 28
   Met Thr Thr Thr Ser Lys His Ala Ala Tyr Cys Leu Lys Gly Ser Cys
    1               5                  10                  15
   Leu Thr Gln Ala Arg Val Gln Trp Pro Leu Lys Leu Thr Thr Ala Ser
               20                  25                  30
   Asn Phe Trp Ala Gln Val Ile Leu Ser Leu Pro Val Val Phe Val Asp
           35                  40                  45
   Cys Leu Met Glu Ser His Gly Thr Arg Arg Leu Lys Gln Cys Leu Gln
       50                  55                  60
   Phe
   65
<210> 29
   <211> 623
   <212> DNA
   <213> Homo sapiens
<220>
   <223> primer sequence
<400> 29
   tcgagcggcc gcccgggcag gtgtcgactc ctgtgaggta tggtgctggg tgcagatgca      60
   gtgtggctct ggatagcacc ttatggacag ttgtgtcccc aaggaaggat gagaatagct     120
   actgaagtcc taaagagcaa gcctaactca agccattggc acacaggcat tagacagaaa     180
   gctggaagtt gaaatggtgg agtccaactt gcctggacca gcttaatggt tctgctcctg     240
   gtaacgtttt tatccatgga tgacttgctt gggtatggag agtcggcttg actacactgt     300
   gtggagcaag ttttaaagaa gcaaaggact cagaattcat gattgaagaa atgcaggcag     360
   acctgttatc ctaaactagg gtttttaatg accacaacaa gcaagcatgc agcttactgc     420
   ttgaaagggt cttgcctcac ccaagctaga gtgcagtggc ctttgaagct tactacagcc     480
   tcaaacttct gggctcaagt gatcctcagc ctcccagtgg tctttgtaga ctgcctgatg     540
   gagtctcatg gcacaagaag attaaaacag tgtctccaat tttaataaat ttttgcaatc     600
   caaaaaaaaa aaaaaaaaaa aaa                                             623
<210> 30
   <211> 23
   <212> DNA
   <213> Artificial Sequence
<220>
   <223> Primer
<400> 30
   aggagtttct gaggaccatg cac 23
<210> 31
   <211> 22
   <212> DNA
   <213> Artificial Sequence
<220>
   <223> Primer
<400> 31
   tcaagggttg gggatacaca cg 22
<210> 32
   <211> 22
   <212> DNA
   <213> Artificial Sequence
<220>
   <223> primer sequence
<400> 32
   cttgcttgct ttcttctctg gc 22
<210> 33
   <211> 24
   <212> DNA
   <213> Artificial Sequence
<220>
   <223> primer sequence
<400> 33
   agtctggaaa tccacatgac caag 24
<210> 34
   <211> 23
   <212> DNA
   <213> Artificial Sequence
<220>
   <223> primer sequence
<400> 34
   cccaatgagg aacctaaagt tgc 23
<210> 35
   <211> 23
   <212> DNA
   <213> Artificial Sequence
<220>
   <223> primer sequence
<400> 35
   ggtgccaaat ctggactctt gtc 23
<210> 36
   <211> 23
   <212> DNA
   <213> Artificial Sequence
<220>
   <223> Primer
<400> 36
   gatccatttt cagcagtgct ctg 23
<210> 37
   <211> 25
   <212> DNA
   <213> Artificial Sequence
<220>
   <223> Primer
<400> 37
   cagtgttcac agaaggggta ctcac 25
<210> 38
   <211> 20
   <212> DNA
   <213> Artificial Sequence
<220>
   <223> Primer
<400> 38
   acgagagcga cacggacaag 20
<210> 39
   <211> 20
   <212> DNA
   <213> Artificial Sequence
<220>
   <223> Primer
<400> 39
   tctgaggctg tggcaggtgc 20
<210> 40
   <211> 21
   <212> DNA
   <213> Artificial Sequence
<220>
   <223> Primer
<400> 40
   ccagtctttg ccaactcgtg c 21
<210> 41
   <211> 23
   <212> DNA
   <213> Artificial Sequence
<220>
   <223> primer sequence
<400> 41
   ttcgatcttc aaactgtgcc ttg 23
<210> 42
   <211> 20
   <212> DNA
   <213> Artificial Sequence
<220>
   <223> Primer
<400> 42
   ttggcaacca gaccagcatc 20
<210> 43
   <211> 22
   <212> DNA
   <213> Artificial Sequence
<220>
   <223> Primer
<400> 43
   tttcccatag gtgtgagtgg cg 22
<210> 44
   <211> 22
   <212> DNA
   <213> Artificial Sequence
<220>
   <223> Primer
<400> 44
   gactggtgtt ttgttcgggg tc 22
<210> 45
   <211> 21
   <212> DNA
   <213> Artificial Sequence
<220>
   <223> Primer
<400> 45
   tttgtccaag gctgcatggt c 21
<210> 46
   <211> 22
   <212> DNA
   <213> Artificial Sequence
<220>
   <223> Primer
<400> 46
   tgccctggtt aagccagaag tc 22
<210> 47
   <211> 22
   <212> DNA
   <213> Artificial Sequence
<220>
   <223> Primer
<400> 47
   agcttcactt tggtcttgac gg 22
<210> 48
   <211> 22
   <212> DNA
   <213> Artificial Sequence
<220>
   <223> Primer
<400> 48
   ggtcatctgc atcaaggttg gc 22
<210> 49
   <211> 22
   <212> DNA
   <213> Artificial Sequence
<220>
   <223> Primer
<400> 49
   ggttcgtaac cgtgacttca gg 22
<210> 50
   <211> 21
   <212> DNA
   <213> Artificial Sequence
<220>
   <223> Primer
<400> 50
   gcatcctttt ccagtcttcc g 21
<210> 51
   <211> 20
   <212> DNA
   <213> Artificial Sequence
<220>
   <223> Primer
<400> 51
   tgcagcaaac atgcctgagc 20
<210> 52
   <211> 22
   <212> DNA
   <213> Artificial Sequence
<220>
   <223> Primer
<400> 52
   tgttccacga gcaaagcatg tg 22
<210> 53
   <211> 22
   <212> DNA
   <213> Artificial Sequence
<220>
   <223> Primer
<400> 53
   atccttcttc cactcccgct tc 22
<210> 54
   <211> 22
   <212> DNA
   <213> Artificial Sequence
<220>
   <223> Primer
<400> 54
   tcggcttgac tacactgtgt gg 22
<210> 55
   <211> 22
   <212> DNA
   <213> Artificial Sequence
<220>
   <223> Primer
<400> 55
   tacaaagacc actgggaggc tg 22
<210> 56
   <211> 24
   <212> DNA
   <213> Artificial Sequence
<220>
   <223> Primer
<400> 56
   cgggaaatcg tgcgtgacat taag 24
<210> 57
   <211> 24
   <212> DNA
   <213> Artificial Sequence
<220>
   <223> Primer
<400> 57
   tgatctcctt ctgcatcctg tcgg 24
<210> 58
   <211> 21
   <212> DNA
   <213> Artificial Sequence
<220>
   <223> Primer
<400> 58
   tttggctaca gcaacagggt g 21
<210> 59
   <211> 22
   <212> DNA
   <213> Artificial Sequence
<220>
   <223> Primer
<400> 59
   tgtgaggagg ggagattcag tg 22
<210> 60
   <211> 16
   <212> DNA
   <213> Artificial Sequence
<220>
   <223> Primer
<400> 60
   cgctgacctc aaccag 16
<210> 61
   <211> 21
   <212> DNA
   <213> Artificial Sequence
<220>
   <223> Primer
<400> 61
   ctgtttgccc gttcttatta c 21
<210> 62
   <211> 24
   <212> DNA
   <213> Artificial Sequence
<220>
   <223> Primer
<400> 62
   cgggaaatcg tgcgtgacat taag 24
<210> 63
   <211> 24
   <212> DNA
   <213> Artificial Sequence
<220>
   <223> Primer
<400> 63
   tgatctcctt ctgcatcctg tcgg 24
<210> 64
   <211> 25
   <212> DNA
   <213> Artificial Sequence
<220>
   <223> Antisense oligonucleotide
<400> 64
   atttgggcat cactggctac aagca 25
<210> 65
   <211> 25
   <212> DNA
   <213> Artificial Sequence
<220>
   <223> Reverse control oligonucleotide
<400> 65
   acgaacatcg gtcactacgg gttta 25
<210> 66
   <211> 24
   <212> DNA
   <213> Artificial Sequence
<220>
   <223> Antisense oligonucleotide
<400> 66
   cagagaggtg agacactcgc cgca 24
<210> 67
   <211> 24
   <212> DNA
   <213> Artificial Sequence
<220>
   <223> Reverse control oligonucleotide
<400> 67
   acgccgctca cagagtggag agac 24
<210> 68
   <211> 25
   <212> DNA
   <213> Artificial Sequence
<220>
   <223> Synthetic oligonucleotide
<400> 68
   ttggtgtcat tgggtcaagg gttgg 25
<210> 69
   <211> 25
   <212> DNA
   <213> Artificial Sequence
<220>
   <223> Synthetic oligonucleotide
<400> 69
   ggttgggaac tgggttactg tggtt 25
<210> 70
   <211> 24
   <212> DNA
   <213> Artificial Sequence
<220>
   <223> Synthetic oligonucleotide
<400> 70
   acagggcaga tacggacctc ggtg 24
<210> 71
   <211> 24
   <212> DNA
   <213> Artificial Sequence
<220>
   <223> Synthetic oligonucleotide
<400> 71
   gtggctccag gcatagacgg gaca 24
<210> 72
   <211> 25
   <212> DNA
   <213> Artificial Sequence
<220>
   <223> Synthetic oligonucleotide
<400> 72
   ttgtgggtaa gcagtttcat gtcgc 25
<210> 73
   <211> 25
   <212> DNA
   <213> Artificial Sequence
<220>
   <223> Synthetic oligonucleotide
<400> 73
   cgctgtactt tgacgaatgg gtgtt 25
<210> 74
   <211> 25
   <212> DNA
   <213> Artificial Sequence
<220>
   <223> Synthetic oligonucleotide
<400> 74
   cctggatcag acgcaagtta tcggc 25
<210> 75
   <211> 25
   <212> DNA
   <213> Artificial Sequence
<220>
   <223> Synthetic oligonucleotide
<400> 75
   cggctattga acgcagacta ggtcc 25
<210> 76
   <211> 25
   <212> DNA
   <213> Artificial Sequence
<220>
   <223> Synthetic oligonucleotide
<400> 76
   ctactcccca cacttcatcg ccagg 25
<210> 77
   <211> 25
   <212> DNA
   <213> Artificial Sequence
<220>
   <223> Synthetic oligonucleotide
<400> 77
   ggaccgctac ttcacacccc tcatc 25
<210> 78
   <211> 25
   <212> DNA
   <213> Artificial Sequence
<220>
   <223> Synthetic oligonucleotide
<400> 78
   ctcttgatac tccagcggca aacca 25
<210> 79
   <211> 25
   <212> DNA
   <213> Artificial Sequence
<220>
   <223> Synthetic oligonucleotide
<400> 79
   accaaacggc gacctcatag ttctc 25
<210> 80
   <211> 24
   <212> DNA
   <213> Artificial Sequence
<220>
   <223> Synthetic oligonucleotide
<400> 80
   gcgcccaagc cgttcgttct taag 24
<210> 81
   <211> 24
   <212> DNA
   <213> Artificial Sequence
<220>
   <223> Synthetic oligonucleotide
<400> 81
   gaattcttgc ttgccgaacc cgcg 24
<210> 82
   <211> 25
   <212> DNA
   <213> Artificial Sequence
<220>
   <223> Synthetic oligonucleotide
<400> 82
   ccaggtaggc acgagttggc aaaga 25
<210> 83
   <211> 25
   <212> DNA
   <213> Artificial Sequence
<220>
   <223> Synthetic oligonucleotide
<400> 83
   agaaacggtt gagcacggat ggacc 25
<210> 84
   <211> 25
   <212> DNA
   <213> Artificial Sequence
<220>
   <223> Synthetic oligonucleotide
<400> 84
   gccattgaag atgcccagat cccac 25
<210> 85
   <211> 25
   <212> DNA
   <213> Artificial Sequence
<220>
   <223> Synthetic oligonucleotide
<400> 85
   caccctagac ccgtagaagt taccg 25
<210> 86
   <211> 24
   <212> DNA
   <213> Artificial Sequence
<220>
   <223> Synthetic oligonucleotide
<400> 86
   cctgcgtttg tccctccagc atct 24
<210> 87
   <211> 24
   <212> DNA
   <213> Artificial Sequence
<220>
   <223> Synthetic oligonucleotide
<400> 87
   tctacgacct ccctgtttgc gtcc 24
<210> 88
   <211> 25
   <212> DNA
   <213> Artificial Sequence
<220>
   <223> Synthetic oligonucleotide
<400> 88
   aagtcacagt ccccggatac cagtc 25
<210> 89
   <211> 25
   <212> DNA
   <213> Artificial Sequence
<220>
   <223> Synthetic oligonucleotide
<400> 89
   ctgaccatag gcccctgaca ctgaa 25
<210> 90
   <211> 25
   <212> DNA
   <213> Artificial Sequence
<220>
   <223> Synthetic oligonucleotide
<400> 90
   ttgtcgcttt ggcaggcata aaacc 25
<210> 91
   <211> 25
   <212> DNA
   <213> Artificial Sequence
<220>
   <223> Synthetic oligonucleotide
<400> 91
   tctggtcatc aacttgcttt ccgtg 25
<210> 92
   <211> 25
   <212> DNA
   <213> Artificial Sequence
<220>
   <223> Synthetic oligonucleotide
<400> 92
   cagtgtttcg tggtgtgctc tgtgg 25
<210> 93
   <211> 23
   <212> DNA
   <213> Artificial Sequence
<220>
   <223> Synthetic oligonucleotide
<400> 93
   gctcaccatc cgggcaccaa gca 23
<210> 94
   <211> 25
   <212> DNA
   <213> Artificial Sequence
<220>
   <223> Synthetic oligonucleotide
<400> 94
   tgagagacag tgtttcgtgg tgtgc 25
<210> 95
   <211> 25
   <212> DNA
   <213> Artificial Sequence
<220>
   <223> Synthetic oligonucleotide
<400> 95
   tgccttcaca cgcttggtta tcttc 25
<210> 96
   <211> 25
   <212> DNA
   <213> Artificial Sequence
<220>
   <223> Synthetic oligonucleotide
<400> 96
   gacaacatcg gaggcttcaa tcacc 25
<210> 97
   <211> 25
   <212> DNA
   <213> Artificial Sequence
<220>
   <223> Synthetic oligonucleotide
<400> 97
   gttgaggctc tgaacaccac tgttg 25
<210> 98
   <211> 25
   <212> DNA
   <213> Artificial Sequence
<220>
   <223> Synthetic oligonucleotide
<400> 98
   gtttggcagc accttcaaca tttgg 25
<210> 99
   <211> 24
   <212> DNA
   <213> Artificial Sequence
<220>
   <223> Synthetic oligonucleotide
<400> 99
   agcagtttgg cagcaccttc aaca 24
<210> 100
   <211> 25
   <212> DNA
   <213> Artificial Sequence
<220>
   <223> Synthetic oligonucleotide
<400> 100
   cttctattgg ttcgcacact tccgt 25
<210> 101
   <211> 25
   <212> DNA
   <213> Artificial Sequence
<220>
   <223> Synthetic oligonucleotide
<400> 101
   ccactaactt cggaggctac aacag 25
<210> 102
   <211> 25
   <212> DNA
   <213> Artificial Sequence
<220>
   <223> Synthetic oligonucleotide
<400> 102
   gttgtcacca caagtctcgg agttg 25
<210> 103
   <211> 25
   <212> DNA
   <213> Artificial Sequence
<220>
   <223> Synthetic oligonucleotide
<400> 103
   ggtttacaac ttccacgacg gtttg 25
<210> 104
   <211> 24
   <212> DNA
   <213> Artificial Sequence
<220>
   <223> Synthetic oligonucleotide
<400> 104
   acaacttcca cgacggtttg acga 24
<210> 105
   <211> 24
   <212> DNA
   <213> Artificial Sequence
<220>
   <223> Synthetic oligonucleotide
<400> 105
   atctggcatg gacggatgag cgaa 24
<210> 106
   <211> 24
   <212> DNA
   <213> Artificial Sequence
<220>
   <223> Synthetic oligonucleotide
<400> 106
   gctgggtggt ttccgaactc aacg 24
<210> 107
   <211> 25
   <212> DNA
   <213> Artificial Sequence
<220>
   <223> Synthetic oligonucleotide
<400> 107
   gtcccaatca ccttccccac aatcc 25
<210> 108
   <211> 25
   <212> DNA
   <213> Artificial Sequence
<220>
   <223> Synthetic oligonucleotide
<400> 108
   tcagatcctt cttccactcc cgctt 25
<210> 109
   <211> 25
   <212> DNA
   <213> Artificial Sequence
<220>
   <223> Synthetic oligonucleotide
<400> 109
   tgctcgtgga acaggtaaag ctctg 25
<210> 110
   <211> 24
   <212> DNA
   <213> Artificial Sequence
<220>
   <223> Synthetic oligonucleotide
<400> 110
   aagcgagtag gcaggtacgg tcta 24
<210> 111
   <211> 24
   <212> DNA
   <213> Artificial Sequence
<220>
   <223> Synthetic oligonucleotide
<400> 111
   gcaactcaag cctttggtgg gtcg 24
<210> 112
   <211> 25
   <212> DNA
   <213> Artificial Sequence
<220>
   <223> Synthetic oligonucleotide
<400> 112
   cctaacaccc cttccactaa ccctg 25
<210> 113
   <211> 25
   <212> DNA
   <213> Artificial Sequence
<220>
   <223> Synthetic oligonucleotide
<400> 113
   ttcgccctca ccttcttcct agact 25
<210> 114
   <211> 25
   <212> DNA
   <213> Artificial Sequence
<220>
   <223> Synthetic oligonucleotide
<400> 114
   gtctcgaaat ggacaaggtg ctcgt 25
<210> 115
   <211> 25
   <212> DNA
   <213> Artificial Sequence
<220>
   <223> Synthetic oligonucleotide
<400> 115
   agcttcactt tggtcttgac ggcat 25
<210> 116
   <211> 24
   <212> DNA
   <213> Artificial Sequence
<220>
   <223> Synthetic oligonucleotide
<400> 116
   cggagggaag tcaagtcagc caca 24
<210> 117
   <211> 24
   <212> DNA
   <213> Artificial Sequence
<220>
   <223> Synthetic oligonucleotide
<400> 117
   cggcattcac cctctccagc acct 24
<210> 118
   <211> 23
   <212> DNA
   <213> Artificial Sequence
<220>
   <223> Synthetic oligonucleotide
<400> 118
   cctccacctg tttgcgggct tcc 23
<210> 119
   <211> 25
   <212> DNA
   <213> Artificial Sequence
<220>
   <223> Synthetic oligonucleotide
<400> 119
   ccacattgag ggagtcctct tgcaa 25
<210> 120
   <211> 25
   <212> DNA
   <213> Artificial Sequence
<220>
   <223> Synthetic oligonucleotide
<400> 120
   tacggcagtt ctggtttcac ttcga 25
<210> 121
   <211> 24
   <212> DNA
   <213> Artificial Sequence
<220>
   <223> Synthetic oligonucleotide
<400> 121
   acaccgactg aactgaaggg aggc 24
<210> 122
   <211> 24
   <212> DNA
   <213> Artificial Sequence
<220>
   <223> Synthetic oligonucleotide
<400> 122
   tccacgacct ctcccactta cggc 24
<210> 123
   <211> 23
   <212> DNA
   <213> Artificial Sequence
<220>
   <223> Synthetic oligonucleotide
<400> 123
   ccttcgggcg tttgtccacc tcc 23
<210> 124
   <211> 24
   <212> DNA
   <213> Artificial Sequence
<220>
   <223> synthetic oligonucleotide
<400> 124
   ccccgaacaa aacaccagtc aacg 24
<210> 125
   <211> 24
   <212 > DNA
   <213> Artificial Sequence
<220>
   <223> synthetic oligonucleotide
<400> 125
   gcaactgacc acaaaacaag cccc 24
<210> 126
   <211> 25
   <212> DNA
   <213> Artificial Sequence
<220>
   <223> synthetic oligonucleotide
<400> 126
   ggccattgag tccctccata gcagc 25
<210> 127
   <211> 25
   <212> DNA
   <213> Artificial Sequence
<220>
   <223> synthetic oligonucleotide
<400> 127
   cgacgatacc tccctgagtt accgg 25